# EUROPEAN PATENT APPLICATION

(11) **EP 2 033 645 A1**
(43) Date of publication of application: **11.03.2009**
(21) Application number: 08015611.0
(22) Date of filing: 25.02.2005
(51) Int. Cl.: A61K 31/473, C07D 221/18

(54) **Isoquinoline derivatives and methods of use thereof**

(30) Priority: 26.02.2004 US 547899 P
(62) Divisional of application: 05723909.7
(71) Applicant: Inotek Pharmaceuticals Corporation, Beverly, MA 01915 (US)
(72) Inventor: Jagtap, Prakash, North Andover, Massachusetts 01845 (US); Baloglu, Erkan, Stoneham MA 02180 (US); Van Duzer, John, H., Georgetown MA 01833 (US); Szabo, Csaba, Washington 98199 (US); Salzman, Andrew, L., Belmont MA 02478 (US); Roy, Aloka, Acton MA 01720 (US); Wiilliams, William, Ipswich MA 01938 (US); Nivorozhkin, Alexander, West Roxbury MA 02132 (US)
(74) Representative: Maiwald Patentanwalts GmbH

(57) **Abstract**

The present invention relates to Isoquinoline Derivatives, compositions comprising an effective amount of a Isoquinoline Derivative and methods for treating or preventing an inflammatory disease, a reperfusion injury, an ischemic condition, renal failure, diabetes, a diabetic complication, a vascular disease other than a cardiovascular disease, cardiovascular disease, reoxygenation injury resulting from organ transplantation, Parkinson's disease, or cancer, comprising administering to an animal in need thereof an effective amount of a Isoquinoline Derivative.

## Description

This application claims the benefit of U.S. Provisional Application No. 60/547,899, filed February 26, 2004, which is incorporated by reference herein in its entirety.

### 1. FIELD OF THE INVENTION

The present invention relates to Isoquinoline Derivatives, compositions comprising an effective amount of a Isoquinoline Derivative and methods for treating or preventing an inflammatory disease, a reperfusion injury, an ischemic condition, renal failure, diabetes, a diabetic complication, a vascular disease other than a cardiovascular disease, cardiovascular disease, reoxygenation injury resulting from organ transplantation, Parkinson's disease, or cancer, comprising administering to an animal in need thereof an effective amount of an Isoquinoline Derivative.

### 2. BACKGROUND OF THE INVENTION

Inflammatory diseases, such as arthritis, colitis, and autoimmune diabetes, typically manifest themselves as disorders distinct from those associated with reperfusion injuries, e.g., stroke and heart attack, and can clinically manifest themselves as different entities. However, there can be common underlying mechanisms between these two types of disorders. In particular, inflammatory disease and reperfusion injury can induce proinflammatory cytokine and chemokine synthesis which can, in turn, result in production of cytotoxic free radicals such as nitric oxide and superoxide. NO and superoxide can react to form peroxynitrite (ONOO⁻) (Szabó et al., Shock 6:79-88, 1996).

The ONOO⁻-induced cell necrosis observed in inflammatory disease and in reperfusion injury involves the activation of the nuclear enzyme poly (ADP-ribose) synthetase (PARS). Activation of PARS is thought to be an important step in the cell-mediated death observed in inflammation and reperfusion injury (Szabó et al., Trends Pharmacol. Sci. 19:287-98, 1998).

A number of PARS inhibitors have been described in the art. See, *e*.*g*., Banasik et al., J. Biol. Chem., 267:1569-75, 1992, and Banasik et al., Mol. Cell. Biochem., 138:185-97, 1994; WO 00/39104; WO 00/39070; WO 99/59975; WO 99/59973; WO 99/11649; WO 99/11645; WO 99/11644; WO 99/11628; WO 99/11623; WO 99/11311; WO 00/42040; Zhang et al., Biochem. Biophys. Res. Commun., 278:590-98,2000; White et al., J. Med. Chem., 43:4084-4097, 2000; Griffin et al., J. Med. Chem., 41:5247-5256, 1998; Shinkwin et al., Bioorg. Med. Chem., 7:297-308, 1999; and Soriano et at., Nature Medicine, 7:108-113, 2001. Adverse effects associated with administration of PARS inhibitors have been discussed in Milan et al., Science, 223:589-591, 1984.

Isoquinoline compounds have been previously discussed in the art. For example, cytotoxic non-camptothecin topoisomerase I inhibitors are reported in Cushman et al., J. Med. Chem., 43:3688-3698, 2300 and Cushman et al., J. Med. Chem. 42:446-57, 1999; indeno[1,2-c]isoquinolines are reported as antineoplastic agents in Cushman et al., WO 00/21537; and as neoplasm inhibitors in Hrbata et al., WO 93/05023.

Syntheses of isoquinoline compounds have been reported. For example, see Wawzonek et al., Org. Prep. Proc. Int., 14:163-8, 1982; Wawzonek et al., Can. J. Chem., 59:2833, 1981; Andoi et al., Bull. Chem. Soc. Japan, 47:1014-17, 1974; Dusemund et al., Arch. Pharm (Weinheim, Ger.), 3 17:381-2, 1984; and Lal et al., Indian J. Chem., Sect. B, 38B:33-39, 1999.

There remains, however, a need in the art for compounds useful for treating or preventing an inflammatory disease, a reperfusion injury, an ischemic condition, renal failure, diabetes, a diabetic complication, a vascular disease other than a cardiovascular disease, or cancer.

Citation of any reference in Section 2 of this application is not an admission that the reference is prior art.

### 3. SUMMARY OF THE INVENTION

Accordingly, in one aspect the invention includes a compound of Formula **I,** Formula Formula **II,** Formula **III,** Formula **IV** or a pharmaceutically acceptable salt thereof (an "Isoquinoline Derivative") as set forth below. Isoquinoline Derivatives are useful for treating or preventing an inflammatory disease, a reperfusion injury, an ischemic condition, renal failure, diabetes, a diabetic complication, a vascular disease other than a cardiovascular disease, cardiovascular disease, reoxygenation injury resulting from organ transplantation, Parkinson's disease, or cancer (each being a "Condition").

Also provided are compositions comprising an effective amount of an Isoquinoline Derivative and a physiologically acceptable carrier or vehicle.

Also provided by the invention are methods for treating or preventing a Condition, comprising administering to an animal in need of such treatment or prevention an effective amount of an Isoquinoline Derivative.

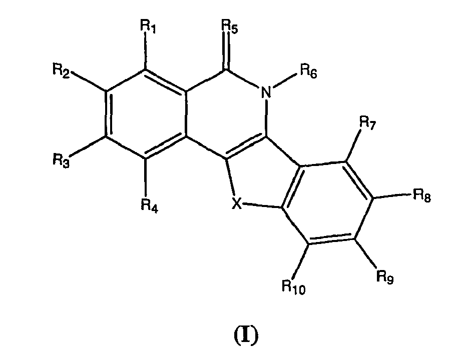

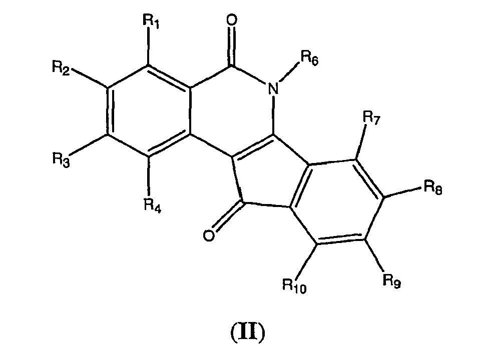

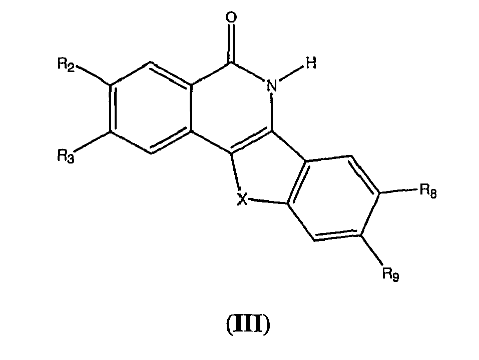

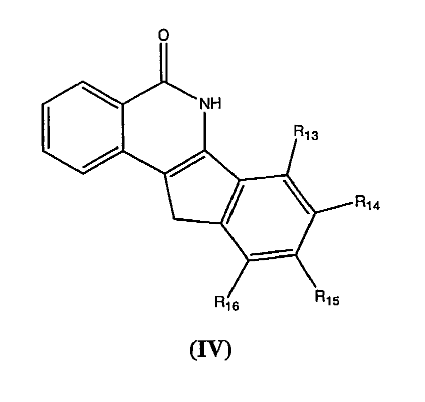

The details of the invention are set forth in the accompanying description below.

Although any methods and materials similar or equivalent to those described herein can be used in the practice or testing of the present invention, illustrative methods and materials are now described. Other features, objects, and advantages of the invention will be apparent from the description and from the claims. All references cited in this specification are incorporated by reference.

### 4. BRIEF DESCRIPTION OF THE FIGURES

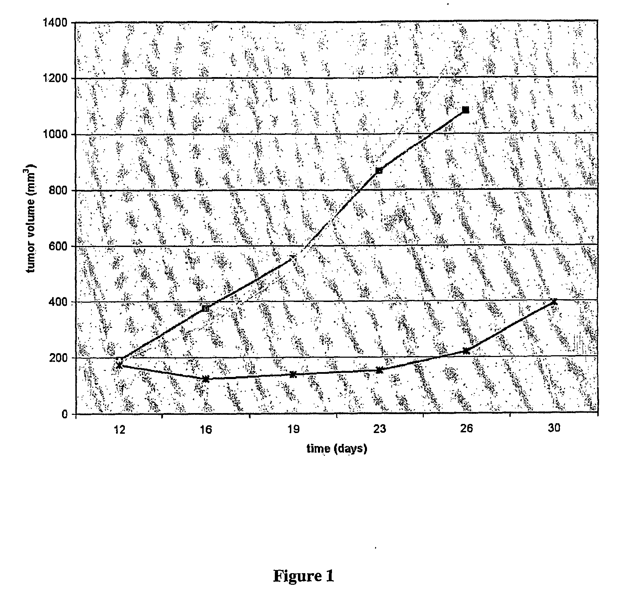

Figure 1 is a graph showing the effect of compound 81 (mesylate salt) and temozolomide on tumor volume. In Figure 1, -■- = control; -▲- = compound 81 (mesylate salt); -x- = temozolomide; and -*- = compound 81 (mesylate salt) and temozolomide.

### 5. DETAILED DESCRIPTION OF THE INVENTION

The present invention provides Isoquinoline Derivatives according to Formula **I,** Formula **II,** Formula **III,** and Formula **IV** below: 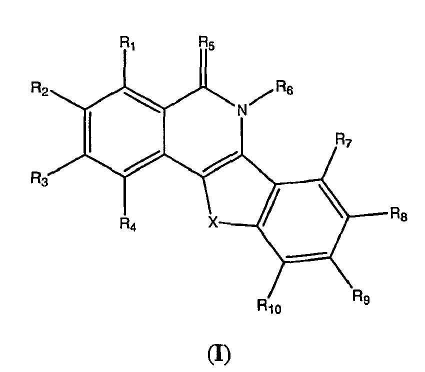 and pharmaceutically acceptable salts thereof,
wherein:
R₅ is O, NH or S;
R₆ is -H or -C₁-C₅ alkyl;
X is -C(O)-, -CH₂-, -CH(halo)-, -CH(OH)-(CH₂)ₙ-, -CH(OH), -CH(-aryl)-, -O-, -NH-, -S-, -CH(NR₁₁R₁₂)- or -N(SO₂Y)-, wherein Y is -OH, -NH₂ or -(C₁-C₅ alkyl)-(-3-to 7-membered monocyclic heterocycle);
R₁₁ and R₁₂ are independently -hydrogen or -C₁-C₁₀ alkyl, or N, R₁₁ and R₁₂ are taken together to form a nitrogen-containing-3- to 7-membered monocyclic heterocycle;
R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A-B;
A is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -NZ₁Z₂, -(C₁-C₅ alkylene)-NZ₁,Z₂, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -C(NH)NH₂, each of which, other than -NZ₁Z₂, -C(O)OH, or -C(NH)NH₂, is unsubstituted or substituted with one or more of -O-(C₁-C₅ alkyl), -halo, -hydroxy, -NO₂, -CN, -NZ₁Z₂, -nitrogen-containing-3- to 7-membered monocyclic heterocycle, -C₁-C₁₀ alkyl, -C₂-C₁₀ alkenyl, -C₂-C₁₀ alkynyl, -aryl, -benzyl, -C(O)OH, -C₁-C₅ alkylene-C(O)O-(C₁-C₅ alkyl) or -C₁-C₅ alkylene-OC(O)-(C₁-C₅ alkyl);
R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀, alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
n is an integer ranging from 0-5.

In one embodiment, X is -C(O)-, -CH₂-, -CH(halo)-, -CH(OH)-(CH₂)ₙ-, -CH(OH), -CH(-aryl)-, -O-, -NH-, -S- or -CH(NR₁₁R₁₂)-, wherein n is an integer ranging from 0-5.

In one embodiment, R⁵ is O.

In another embodiment, R⁵ is S.

In a further embodiment, R⁵ is NH.

In another embodiment, X is -N(SO₂Y)-.

In one embodiment, A is -SO₂- or -SO₂NH-.

In another embodiment, B is -C₁-C₁₀ alkyl, -C₂-C₁₀ alkenyl, -3- to 7-membered monocyclic heterocycle, -7- to 10-membered bicyclic heterocycle, -C₃-C₈ monocyclic cycloalkyl, -aryl, -NZ₁Z₂, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl) or -C(O)O-phenyl, each of which, other than -NZ₁Z₂, -C(O)OH, or -C(NH)NH₂, is unsubstituted or substituted with one or more of -O-(C₁-C₅ alkyl), -halo, -hydroxy, -NO₂, -NZ₁Z₂, -nitrogen-containing-3- to 7-membered monocyclic heterocycle, -C₁-C₁₀ alkyl, -C₂-C₁₀ alkenyl, -C₂-C₁₀ alkynyl, -aryl, -benzyl, -C₁-C₅ alkylene-C(O)O-C₁-C₅ alkyl or -C₁-C₅ alkylene-OC(O)-C₁-C₅ alkyl.

In another embodiment, R₁-R₄ are hydrogen.

In a further embodiment, at least one of R₁, R₂, R₃, R₄, R₇, R₈, R₉ and R₁₀ is other than hydrogen.

In one embodiment, one of R₇-R₁₀ is -NHC(O)-(CH₂)ₙ-OAc or -NHC(O)-(CH₂)ₙ-OH.

In other illustrative embodiments R⁵ and X in a compound of formula **(I)** are as set forth below:

| **R⁵** | **X** |
|---|---|
| NH | -C(O)- |
| NH | -CH₂- |
| NH | -CH(halo)- |
| NH | -CH(OH)CH₂)ₙ- |
| NH | -CH(OH)- |
| NH | -CH(-aryl)- |
| NH | -O- |
| NH | -NH- |
| NH | -S- |
| NH | -CH(NR¹¹R¹²)- |
| NH | -N(SO₂Y)- |
| S | -C(O)- |
| S | -CH₂- |
| S | -CH(halo)- |
| S | -CH(OH)(CH₂)ₙ- |
| S | -CH(OH)- |
| S | -CH(-aryl)- |
| S | -O- |
| S | -NH- |
| S | -S- |
| S | -CH(NR¹¹R¹²)- |
| O | -N(SO₂Y)- |
| O | -C(O)- |
| O | -CH₂- |
| O | -CH(halo)- |
| O | -CH(OH)(CH₂)ₙ- |
| O | -CH(OH)- |
| O | -CH(-aryl)- |
| O | -O- |
| O | -NH- |
| O | -S- |
| O | -CH(NR¹¹R¹²)- |
| O | -N(SO₂Y)- |

In another embodiment, the compounds of Formula (I) have the Formula (Ia): 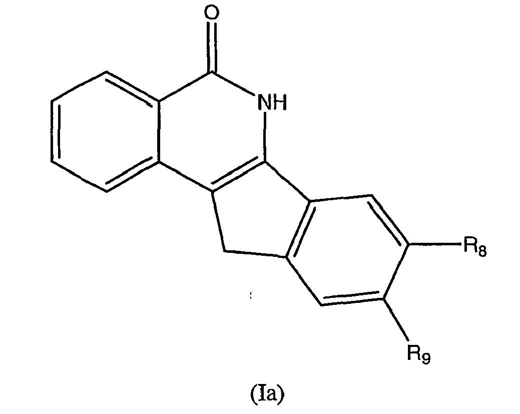 where R⁸ and R⁹ are as defined above for Formula (I).

In one embodiment, the compounds of Formula (Ia) are those wherein R₈ is -H, R₉ is -A-B, A is -SO₂- and B is -NZ₁Z₂ or -(C₁-C₅ alkylene)NZ₁Z₂.

Illustrative examples of compounds of Formula (Ia) are set forth below:

| **Compound** | **R₈** | **R₉** |
|---|---|---|
| 43 | -H | -NHC(O)CH₂N(CH₃)₂ |
| 44 | -H | -SO₂NH(CH₂)₃-(morpholin-4-yl) |
| 45 | -NHC(O)CH₂N(CH₃)₂ | -H |
| 46 | -SO₂NH(CH₂)₃-(morpholin-4-yl) | -H |
| 97 | -NO₂ | -H |
| 98 | -H | -NO₂ |
| 99 | -F | -H |
| 100 | -H | -F |
| 101 | -NH₂ | -H |
| 102 | -H | -NH₂ |
| 103 | -H | -NHCOCH₂OAc |
| 104 | -H | -NHCOCH₂OH |
| 105 | -H | -NHCONH-n-propyl |
| 106 | -H | -SO₂NH(CH₂)₃-phenyl |
| 107 | -F | -SO₂NH(CH₂)₃-morpholine |
| 108 | -F | -SO₂NH-morpholine |
| 109 | -F | -SO₂-imidazole |
| 110 | -H | -SO₃Na |
| 111 | -SO₃Na | -H |

and pharmaceutically acceptable salts thereof.

In another embodiment, the compounds of Formula (I) have the Formula (Ib): 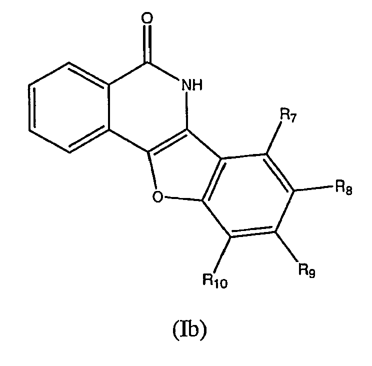 where R₇, R₈, R₉ and R₁₀ are as defined above for Formula (I).

Illustrative examples of compounds of Formula (Ib) are set forth below:

| **Compound** | **R₇** | **R₈** | **R₉** | **R₁₀** |
|---|---|---|---|---|
| 22a | -H | -H | -H | -H |
| 22b | -H | -OMe | -H | -H |
| 22c | -H | -H | -OMe | -H |
| 22d | -H | -H | -H | -OMe |
| 22e | -H | -Me | -H | -H |
| 22f | -H | -COOH | -H | -H |
| 22g | -H | -H | -COOH | -H |
| 23a | -H | -OH | -H | -H |
| 23b | -H | -H | -OH | -H |
| 23c | -H | -H | -H | -OH |
| 25a | -H | -H | -(CH₂)₄OH | -H |
| 25b | -H | -H | -(CH₂)₅OH | -H |
| 25c | -H | -H | -(CH₂)₆OH | -H |
| 25d | -H | -H | -(CH₂)₄COOH | -H |
| 25e | -H | -H | -(CH₂)₅COOH | -H |
| 26a | -H | -C(O)NH(CH₂)₃-(morpholin-1-yl) | -H | -H |
| 26b | -H | -C(O)NH(CH₂)₂-COOH | -H | -H |
| 26c | -H | -C(O)NH(CH₂)₃-*N*-(1,3-imidazole) | -H | -H |
| 26d | -H | -C(O)NH(CH₂)₂-NMe₂ | -H | -H |
| 26e | -H | -H | -SO₃Na | -H |
| 26f | -H | -SO₃Na | -H | -H |

and pharmaceutically acceptable salts thereof.

In another embodiment, the compounds of Formula (I) have the Formula (Ic): 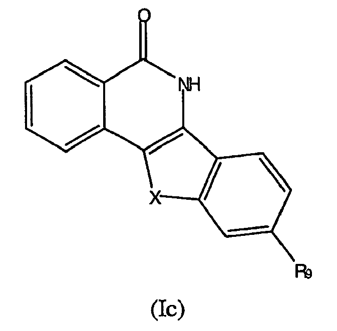 where X and R⁹ are as defined above for Formula (I).

Illustrative examples of compounds of Formula (Ic) are set forth below:

| **Compound** | **X** | **R₉** |
|---|---|---|
| 34 | -N(SO₃H)- | -SO₃H |
| 35a | -N(SO₂NH₂)- | -SO₂NH₂ |
| 35b | -N[SO₂NH(CH₂)₃-(morpholin-4-yl)]- | -SO₂NH(CH₂)₃-(morpholin-4-yl) |
| 40a | -S- | -H |

and pharmaceutically acceptable salts thereof.

In another embodiment, the compounds of Formula (I) have the Formula (Id): 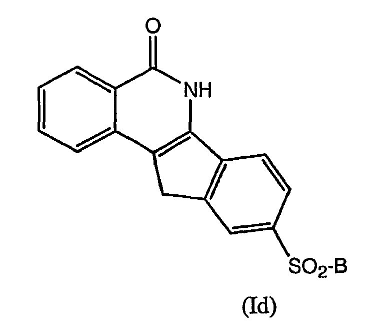 where B is as defined above for the compounds of Formula (I).

In one embodiment, B is -NZ₁Z₂ or -(C₁-C₅ alkylene)-NZ₁Z₂, or -(C₁-C₅ alkylene) substituted with -NH₂ or -OH.

The invention also relates to compounds of Formula **II:** 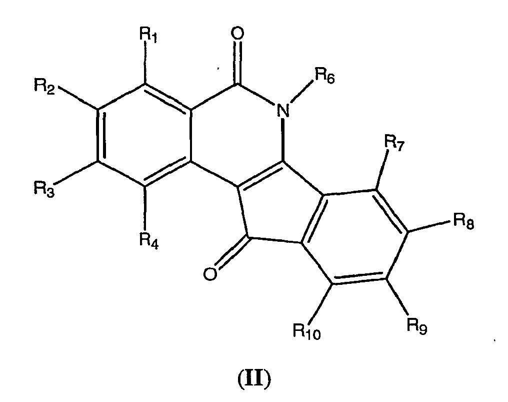 and pharmaceutically acceptable salts thereof,
wherein:
R₆ is -H or C₁-C₅ alkyl;
R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A'-B';
A' is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
B' is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂;
R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
A is -SO₂-, -SO₂NH-, -NHCO-, -NHCONH-, -O-, -CO-, -OC(O)-, -C(O)O-, -CONH-, -CON(C₁-C₅ alkyl)-, -NH-, -CH₂-, -S- or -C(S)-;
B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂; and
Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle.

In one embodiment, B is a nitrogen-containing-3- to 7-membered monocyclic heterocycle.

In still another embodiment, R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, - amino-substituted C₁-C₅ alkyl, -C(O)OH, -C(O)O(C₁-C₅ alkyl), -NO₂ or -A'-B';
A' is -SO₂-, -SO₂NH-, -NHCO-, -NHCONH-, -CO-, -C(O)O-, -CONH-, -CON(C₁-C₅ alkyl)-, -NH-, -CH₂, -S- or -C(S)-; and
B' is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -amino-substituted C₁-C₅ alkyl, -C₁-C₅ alkyl-(3- to 7-membered monocyclic heterocycle), - H₂NC(O)-substituted aryl, -C(O)OH, -C(O)O-(C₁-C₅ alkyl), -C(O)O-phenyl or -NZ₁Z₂.

In a further embodiment, at least one of R₁, R₂, R₃, R₄, R₇, R₈, R₉ and R₁₀ is not hydrogen.

In another embodiment, at least one of R₂, R₄ and R₁₀ is other than hydrogen.

In one embodiment, A is other than -CONH-.

The invention also relates to compounds of Formula **III:** 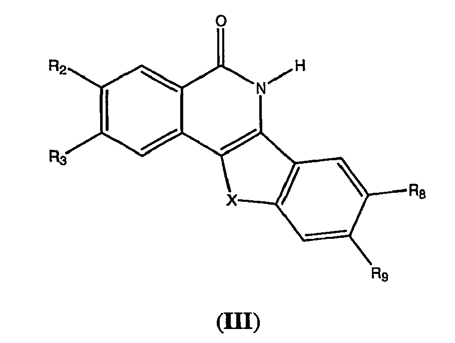 and pharmaceutically acceptable salts thereof,
wherein:
X is -CH₂- or -O-;
R₂ and R₃ are independently -hydrogen, -halo, -halo-substituted C₁-C₅ alkyl, -hydroxy, -O-(C₁-C₅ alkyl), -C₁-C₅ alkyl, -NO₂, -NH₂, -CONH₂, -C(O)OH, -OC(O)-C₁-C₅ alkyl or -C(O)O-C₁-C₅ alkyl;
R₈ and R₉ are independently -hydrogen or -A-B;
A is -SO₂-, -SO₂NH- or -NHCO-;
B is -C₁-C₅ alkyl, -NZ₁Z₂, -3- to 7-membered monocyclic heterocycle, or -7- to 10-membered bicyclic heterocycle, each of which is unsubstituted or substituted with one or more of -hydroxy-substituted C₁-C₅ alkyl, -amino-substituted C₁-C₅ alkyl, -3- to 7-membered monocyclic heterocycle, or -7- to 10-membered bicyclic heterocycle, each unsubstituted or substituted with -C₁-C₁₀ alkyl or -hydroxy-substituted C₁-C₅ alkyl; and
Z₁ and Z₂ are independently -hydrogen or -C₁-C₈ alkyl, which is unsubstituted or substituted with one or more of -hydroxy or -N(Z₃)(Z₄), where Z₃ and Z₄ are independently, -H or -C₁-C₅ alkyl, which is unsubstituted or substituted with one or more of -hydroxy or -NH₂; or N, Z₃ and Z₄ are taken together to form a nitrogen-containing-3-to 7-membered monocyclic heterocycle; or N, Z₁ and Z₂ are taken together to form a nitrogen-containing-3- to 7-membered monocyclic heterocycle.

In one embodiment, -X- is -CH₂-.

In another embodiment, -X- is -O-.

In one embodiment, R₈ is hydrogen and R₉ is -A-B.

In another embodiment, R₈ is -A-B and R₉ is hydrogen.

In one embodiment, either R₈ is hydrogen and R₉ is -A-B, or R₈ is -A-B and R₉ is hydrogen.

In still another embodiment, R₂, R₃ and R₈ are hydrogen and R₉ is -A-B, wherein A is
-SO₂- or -SO₂NH-.

In a further embodiment, at least one of R₂, R₃, R₈ and R₉ is not hydrogen.

The invention further relates to compounds of Formula **13:** 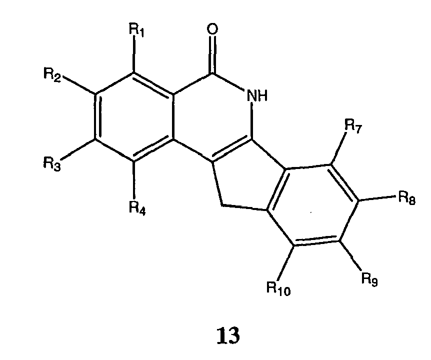 and pharmaceutically acceptable salts thereof,
wherein:
R₁, R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
A is -SO₂-, -SO₂NH-, -NHCO-, -NHCONH-, -O-, -CO-, -OC(O)-, -C(O)O-, -CONH-, -CON(C₁-C₅ alkyl)-, -NH-, -CH₂-, -S- or -C(S)-;
B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -NZ₁Z₂, -(C₁-C₅ alkylene)-NZ₁Z₂, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -C(NH)NH₂, each of which, other than -NZ₁Z₂, -C(O)OH, or -C(NH)NH₂, is unsubstituted or substituted with one or more of -O-(C₁-C₅ alkyl), -halo, -hydroxy, -NO₂, -CN, -NZ₁Z₂, -nitrogen-containing-3- to 7-membered monocyclic heterocycle, -C₁-C₁₀ alkyl, -C₂-C₁₀ alkenyl, -C₂-C₁₀ alkynyl, -aryl, -benzyl, -C(O)OH, -C₁-C₅ alkylene-C(O)O-(C₁-C₅ alkyl) or -C₁-C₅ alkylene-OC(O)-(C₁-C₅ alkyl); and
Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle.

In one embodiment, R₉ is -A-B, wherein -A- is -SO₂- or -SO₂NH-.

In another embodiment, R₁-R₄ are each hydrogen.

In another embodiment, R₁-R₄ are each hydrogen.

In a further embodiment, at least one of R₁, R₂, R₃, R₄, R₇, R₈, R₉ and R₁₀ is other than hydrogen.

In one embodiment, A is other than -CONH-.

The invention further still relates to compounds of Formula **22:** 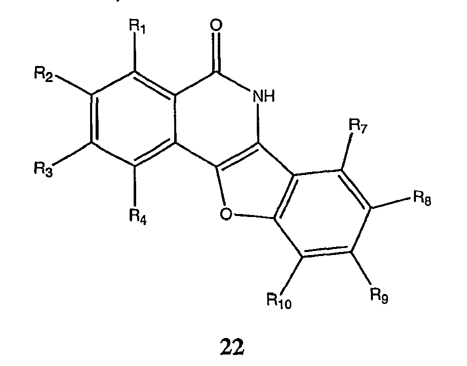 and pharmaceutically acceptable salts thereof,
wherein:
R₁-R₄ and R₇-R₁₀ are as defined above for Formula **13.**

In one embodiment, R₉ is -A-B, wherein -A- is -SO₂- or -SO₂NH-.

In another embodiment, R₁-R₄ are each hydrogen.

In a further embodiment, at least one of R₁, R₂, R₃, R₄, R₇, R₈, R₉ and R₁₀ is other than hydrogen.

The invention further still relates to compounds of Formula **37:** 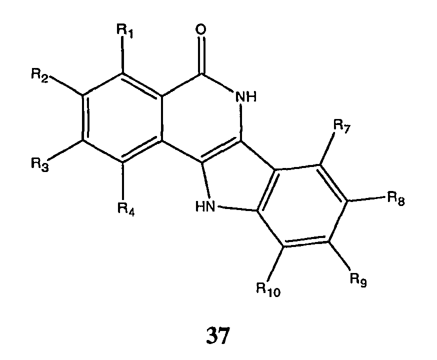 and pharmaceutically acceptable salts thereof,
wherein:
R₁-R₄ and R₇-R₁₀ are as defined above for Formula **13.**

In one embodiment, R₁-R₄ are each hydrogen.

In another embodiment, R₉ is -A-B, wherein -A- is -SO₂- or -SO₂NH-.

In a further embodiment, at least one of R₁, R₂, R₃, R₄, R₇, R₈, R₉ and R₁₀ is other than hydrogen.

The invention also relates to compounds of Formula **40:** 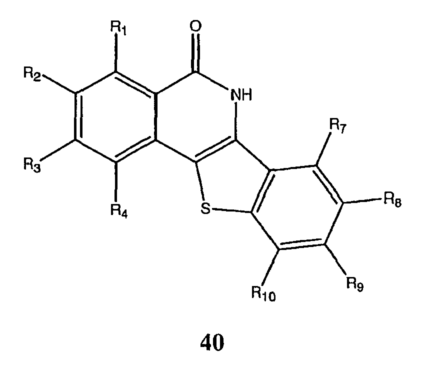 and pharmaceutically acceptable salts thereof,
wherein:
R₁-R₄ and R₇-R₁₀ are as defined above for Formula **13**.

In one embodiment, R₁-R₄ are each hydrogen.

In one embodiment, R₉ is -A-B, wherein -A- is -SO₂- or -SO₂NH-.

In a further embodiment, at least one of R₁, R₂, R₃, R₄, R₇, R₈, R₉ and R₁₀ is other than hydrogen.

The invention further relates to compounds of Formula **(IV):** 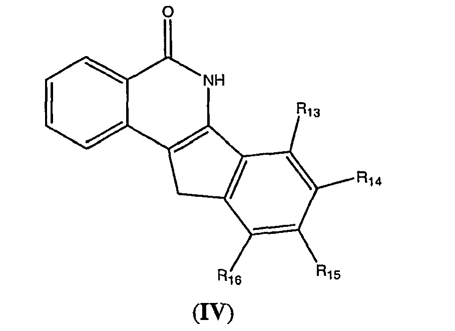 and pharmaceutically acceptable salts thereof,
wherein:
R₁₃ and R₁₆ are hydrogen;
one of the R₁₄ and R₁₅ groups is -NHC(O)-(CH₂)ₙ-NZ₁Z₂, and the other group is - hydrogen;
Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
n is an integer ranging from 0-5.

In one embodiment, R₁₄ is -NHC(O)-(CH₂)ₙ-NZ₁Z₂ and R₁₃, R₁₅, and R₁₆ are each hydrogen.

In one embodiment, R₁₅ is -NHC(O)-(CH₂)ₙ-NZ₁Z₂ and R₁₃, R₁₄, and R₁₆ are each hydrogen.

In one embodiment, n is 1.

In another embodiment, n is 2.

In another embodiment, n is 3.

In one embodiment, n is 4.

In another embodiment, n is 5.

Illustrative examples of the compounds of Formula (**IV**) include the compounds of Formula (**IVa**): 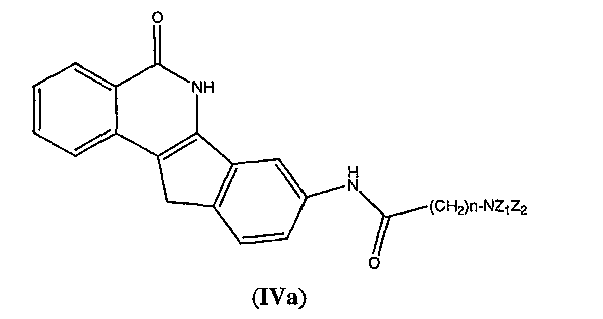 and pharmaceutically acceptable salts thereof.

In one embodiment, the compound of Formula (**IVa**) is:

| **Compound** | **n** | **-NZ₁Z₂** |
|---|---|---|
| 57 | 2 | -N(CH₃)₂ |
| 58 | 3 | -N(CH₃)₂ |
| 59 | 4 | -N(CH₃)₂ |
| 60 | 5 | -N(CH₃)₂ |
| 61 | 1 | 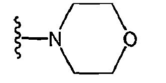 |
| 62 | 2 | 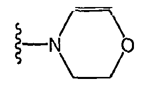 |
| 63 | 3 | 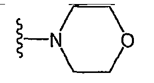 |
| 64 | 4 | 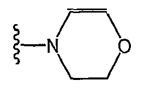 |
| 65 | 5 | 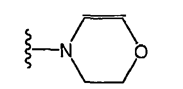 |

or a pharmaceutically acceptable salt thereof.

Illustrative examples of the compounds of Formula (**IV**) also include the compounds of Formula **(IVb):** 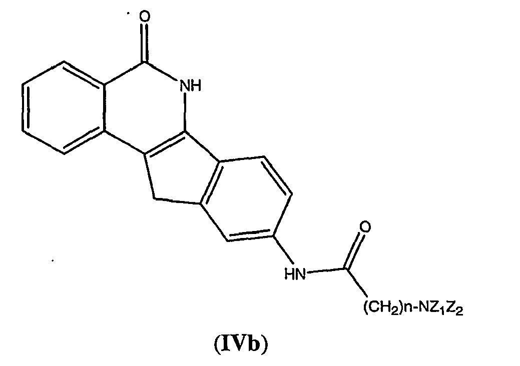 and pharmaceutically acceptable salts thereof.

In one embodiment, the compound of Formula (**IVb**) is:

| **Compound** | **n** | **-NZ₁Z₂** |
|---|---|---|
| 67 | 2 | -N(CH₃)₂ |
| 68 | 3 | -N(CH₃)₂ |
| 69 | 4 | -N(CH₃)₂ |
| 70 | 5 | -N(CH₃)₂ |
| 71 | 1 | 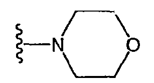 |
| 72 | 2 | 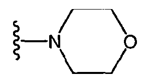 |
| 73 | 3 | 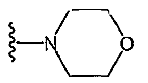 |
| 74 | 4 | 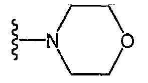 |
| 75 | 5 | 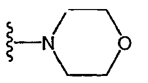 |

or a pharmaceutically acceptable salt thereof.

Additional illustrative compounds of Formula **(IV)** include compounds 43, 45, and 97-109, above, and pharmaceutically acceptable salts thereof.

### 5.1 DEFINITIONS

The following definitions are used in connection with the Isoquinoline Derivatives:

"C₁-C₅ alkyl" refers to a straight or branched chain saturated hydrocarbon containing 1-4 carbon atoms. Examples of a C₁-C₅ alkyl group include, but are not limited to, methyl, ethyl, propyl, butyl, pentyl, isopropyl, isobutyl, *sec*-butyl and tert-butyl, isopentyl and neopentyl.

"C₁-C₈ alkyl" refers to a straight or branched chain saturated hydrocarbon containing 1-8 carbon atoms. Examples of a C₁-C₈ alkyl group include, but are not limited to, methyl, ethyl, propyl, butyl, pentyl, hexyl, heptyl, octyl, isopropyl, isobutyl, *sec*-butyl and *tert*-butyl, isopentyl, neopentyl, isohexyl, isoheptyl and isooctyl.

"C₁-C₁₀ alkyl" refers to a straight or branched chain saturated hydrocarbon containing 1-10 carbon atoms. Examples of a C₁-C₁₀ alkyl group include, but are not limited to, methyl, ethyl, propyl, butyl, pentyl, hexyl, heptyl, octyl, -nonyl, decyl, isopropyl, isobutyl, *sec*-butyl and tert-butyl, isopentyl, neopentyl, isohexyl, isoheptyl, isooctyl, isononyl and isodecyl.

"C₂-C₁₀ alkenyl" refers to a straight or branched chain unsaturated hydrocarbon containing 2-10 carbon atoms and at least one double bond. Examples of a C₂-C₁₀ alkenyl group include, but are not limited to, ethylene, propylene, 1-butylene, 2-butylene, isobutylene, *sec*-butylene, 1-pentene, 2-pentene, isopentene, 1-hexene, 2-hexene, 3-hexene, isohexene, 1-heptene, 2-heptene, 3-heptene, 1-octene, 2-octene, 3-octene, 4-octene, 1-nonene, 2-nonene, 3-nonene, 4-nonene, 1-decene, 2-decene, 3-decene, 4-decene and 5-decene.

"C₂-C₁₀ alkynyl" refers to a straight or branched chain unsaturated hydrocarbon containing 2-10 carbon atoms and at least one triple bond. Examples of a C₂-C₁₀ alkynyl group include, but are not limited to, acetylene, propyne, 1-butyne, 2-butyne, isobutyne, sec-butyne, 1-pentyne, 2-pentyne, isopentyne, 1-hexyne, 2-hexyne, 3-hexyne, isohexyne, 1-heptyne, 2-heptyne, 3-heptyne, 1-octyne, 2-octyne, 3-octyne, 4-octyne, 1-nonyne, 2-nonyne, 3-nonyne, 4-nonyne, 1-decyne, 2-decyne, 3-decyne, 4-decyne and 5-decyne.

"C₁-C₅ alkylene" refers to a C₁-C₅ alkyl group in which one of the C₁-C₅ alkyl group's hydrogen atoms has been replaced with a bond. Examples of a C₁-C₅ alkylene include -CH₂-, -CH₂CH₂-, -CH₂CH₂CH₂-, -CH₂CH₂CH₂CH₂- and -CH₂CH₂CH₂CH₂CH₂-.

"Halo-substituted C₁-C₅ alkyl" refers to a C₁-C₅ alkyl group, as defined above, wherein one or more of the C₁-C₅ alkyl group's hydrogen atoms has been replaced with - F, -Cl, -Br or -I. Representative examples of an alkylhalo group include, but are not limited to, -CH₂F, -CCl₃, -CF₃, -CH₂Cl, -CH₂CH₂Br, -CH₂CH₂I, -CH₂CH₂CH₂F, -CH₂CH₂CH₂Cl, -CH₂CH₂CH₂CH₂Br, -CH₂CH₂CH₂CH₂I, -CH₂CH₂CH₂CH₂CH₂Br, -CH₂CH₂CH₂CH₂CH₂I, -CH₂CH(Br)CH_{3,} -CH₂CH(Cl)CH₂CH₃, -CH(F)CH₂CH₃ and -C(CH₃)₂(CH₂Cl).

"Amino-substituted C₁-C₅ alkyl" refers to a C₁-C₅ alkyl group, as defined above, wherein one or more of the C₁-C5 alkyl group's hydrogen atoms has been replaced with -NH₂. Representative examples of an amino-substituted C₁-C₅ alkyl group include, but are not limited to, -CH₂NH₂, -CH₂CH₂NH₂, -CH₂CH₂CH₂NH₂, -CH₂CH₂CH₂CH₂NH₂, -CH₂CH₂CH₂CH₂CH₂NH₂, -CH₂CH(NH₂)CH₃, -CH₂CH(NH₂)CH₂CH₃, -CH(NH₂)CH₂CH₃ and -C(CH₃)₂(CH₂NH₂).

"Aryl" refers to a phenyl or pyridyl group. Examples of an aryl group include, but are not limited to, phenyl, N-pyridyl, 2-pyridyl, 3-pyridyl and 4-pyridyl. An aryl group can be unsubstituted or substituted with one or more of the following groups: -C₁-C₅ alkyl, halo, -halo-substituted C₁-C₅ alkyl, hydroxy, -O-C₁-C₅ alkyl, -N(R^{a})₂, -COOH, -C(O)O-(C₁-C₅ alkyl), -OC(O)-(C₁-C₅ alkyl), -C(O)NH₂, or -NO₂, wherein each occurrence of R^{a} is independently -H or C₁-C₁₀ alkyl,

"NH₂C(O)-substituted aryl" refers to an aryl group, as defined above, wherein one of the aryl group's hydrogen atoms has been replaced with one or more -C(O)NH₂ groups. Representative examples of a -NH₂C(O)-substituted aryl group include 2-C(O)NH₂-phenyl, 3-C(O)NH₂-phenyl, 4-C(O)NH₂-phenyl, 2-C(O)NH₂-pyridyl, 3-C(O)NH₂-pyridyl and 4-C(O)NH₂-pyridyl.

"-(C₁-C₅ alkyl)-(3- to 7-membered monocyclic heterocycle)" refers to a C₁-C₅ alkyl group, as defined above, wherein one of the C₁-C₅ alkyl group's hydrogen atoms has been replaced with a -3- to 7-membered monocyclic heterocycle. Representative examples of a -(C₁-C₅ alkyl)-(3- to 7-membered monocyclic heterocycle) group include, but are not limited to, -CH₂CH₂-morpholine, -CH₂CH₂-piperidine, -CH₂CH₂CH₂-morpholine and -CH₂CH₂CH₂-imidazole.

"Hydroxy-substituted C₁-C₅ alkyl" refers to a C₁-C₅ alkyl group, as defined above, wherein one of the C₁-C₅ alkyl group's hydrogen atoms has been replaced with a hydroxyl group. Representative examples of an alkanol group include, but are not limited to, -CH₂OH, -CH₂CH₂OH, -CH₂CH₂CH₂OH, -CH₂CH₂CH₂CH₂OH, -CH₂CH₂CH₂CH₂CH₂OH, -CH₂CH(OH)CH₃, -CH₂CH(OH)CH₂CH₃, -CH(OH)CH₂CH₃ and -C(CH₃)₂CH₂OH.

An "Arylene" group is a phenyl group in which one of the phenyl group's hydrogen atoms has been replaced with a bond. An arylene group can bein an ortho, meta, or para configuration and can be unsubstituted or independently substituted with one or more of the following groups: -C₁-C₅ alkyl, halo, hydroxy, -O-C₁-C₅ alkyl, -N(R^{a})₂, -COOH, -halo-substituted C₁-C₅ alkyl, -C(O)O-(C₁-C₅ alkyl), -OC(O)-(C₁-C₅ alkyl), -C(O)NH₂ or -NO₂, wherein each occurrence of R^{a} is independently -H or C₁-C₁₀ alkyl.

A "C₃-C₈ monocyclic cycloalkyl" is a non-aromatic, saturated hydrocarbon ring containing 3-8 carbon atoms. Representative examples of a C₃-C₈ monocyclic cycloalkyl include, but are not limited to, cyclopropyl, cyclobutyl, cyclopentyl, cyclohexyl, cycloheptyl and cyclooctyl. A C₃-C₈ monocyclic cycloalkyl can be unsubstituted or independently substituted with one or more of the following groups: -C₁-C₅ alkyl, halo, - halo-substituted C₁-C₅ alkyl, hydroxy, -O-C₁-C₅ alkyl, -N(R^{a})₂, -COOH, -C(O)O-(C₁-C₅ alkyl), -OC(O)-(C₁-C₅ alkyl), -C(O)NH₂, or -NO₂, wherein each occurrence of R^{a} is independently -H or C₁-C₁₀ alkyl.

A "3- to 7-membered monocyclic heterocycle" refers to a monocyclic 3- to 7-membered aromatic or non-aromatic monocyclic cycloalkyl in which 1-4 of the ring carbon atoms have been independently replaced with a N, O or S atom. The 3- to 7-membered monocyclic heterocycles can be attached via a nitrogen, sulfur, or carbon atom. Representative examples of a 3- to 7-membered monocyclic heterocycle group include, but are not limited to, piperidinyl, piperazinyl, morpholinyl, pyrrolyl, oxazinyl, thiazinyl, diazinyl, triazinyl, tetrazinyl, imidazolyl, tetrazolyl, pyrrolidinyl, isoxazolyl, furanyl, furazanyl, pyridinyl, oxazolyl, thiazolyl, thiophenyl, pyrazolyl, triazolyl, and pyrimidinyl.

A "7- to 10-membered bicyclic heterocycle" refers to a bicyclic 7- to 10-membered aromatic or non-aromatic bicyclic cycloalkyl in which 1-4 of the ring carbon atoms have been independently replaced with a N, O or S atom. The 7- to 10-membered bicyclic heterocycles can be attached via a nitrogen, sulfur, or carbon atom. Representative examples of a 7- to 10-membered bicyclic heterocycle group include, but are not limited to, benzimidazolyl, indolyl, isoquinolinyl, indazolyl, quinolinyl, quinazolinyl, purinyl, benzisoxazolyl, benzoxazolyl, benzthiazolyl, benzodiazolyl, benzotriazolyl, isoindolyl and indazolyl.

A "nitrogen-containing 3- to 7-membered monocyclic heterocycle" refers to a 3-to 7-membered monocyclic heterocycle, defined above, which contains at least one ring nitrogen atom. The nitrogen-containing 3- to 7-membered monocyclic heterocycles can be attached via a nitrogen, sulfur, or carbon atom. Representative examples of nitrogen-containing-3- to 7-membered monocyclic heterocycles include, but are not limited to, piperidinyl, piperazinyl, pyrrolyl, oxazinyl, thiazinyl, diazinyl, triazinyl, tetrazinyl, imidazolyl, tetrazolyl, pyrrolidinyl, isoxazolyl, pyridinyl, oxazolyl, thiazolyl, pyrazolyl, triazolyl, pyrimidinyl, and morpholinyl.

"Halo" is -F, -Cl, -Br or -I.

An "animal" is a mammal, *e*.*g*., a human, mouse, rat, guinea pig, dog, cat, horse, cow, pig, or non-human primate, such as a monkey, chimpanzee, baboon or rhesus. In one embodiment, an animal is a human.

Representative "pharmaceutically acceptable salts" include, *e*.*g*., water-soluble and water-insoluble salts, such as the acetate, amsonate (4,4-diaminostilbene-2, 2-disulfonate), benzenesulfonate, benzonate, besylate, bicarbonate, bisulfate, bitartrate, borate, bromide, butyrate, calcium edetate, camphorsulfonate, camsylate, carbonate, chloride, citrate, clavulariate, dihydrochloride, edetate, edisylate, estolate, esylate, fumarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosaliculate, suramate, tannate, tartrate, teoclate, tosylate, triethiodide, and valerate salts. A hydrate is another example of a pharmaceutically acceptable salt.

An "effective amount" when used in connection with an Isoquinoline Derivative is an amount effective for: (a) treating or preventing a Condition; or (b) inhibiting PARS in an *in vivo* or an *in vitro* cell.

An "effective amount" when used in connection with another anticancer agent is an amount that is effective for treating or preventing cancer alone or in combination with an Isoquinoline Derivative. "In combination with" includes administration within the same composition and within separate compositions. In the latter instance, the anticancer agent is administered during a time when the Isoquinoline Derivative exerts its prophylactic or therapeutic effect, or *vice versa*.

The following abbreviations are used herein and have the indicated definitions: AcOH is acetic acid, AIBN is azibisisobutyronitrile, CCl₄ is carbon tetrachloride, CEP is Cecal Ligation and Puncture, DMEM is Dulbecco's Modified Eagle Medium, DMF is *N,N*-dimethylformamide, DMSO is dimethylsulfoxide, EtOAc is ethyl acetate, EtOH is ethanol, HEPES is 4-(2-hydroxyethyl)-1-piperazineethanesulfonic acid, HPLC is high pressure liquid chromatography, LPS is lipopolysaccharide, Me is methyl, MeCN is acetonitrile, MeOH is methanol, MS is mass spectrometry, Ms is mesyl (methanesulfonyl), NBS is N-bromosuccinimide, NEt₃ is triethylamine, NMR is nuclear magnetic resonance, PBS is phosphate-buffered saline (pH 7.4), PARS is poly(ADP-ribose)synthetase, Py is pyridine, SDS is dodecyl sulfate (sodium salt), STZ is streptozotocin, TCA is tricholoroacetic acid, Tf is triflyl (trifluoromethanesulfonyl), TFA is trifluoroacetic acid, THF is tetrahydrofuran; TLC is thin layer chromatography, TMZ is temozolomide, TNF is tumor necrosis factor, TRIS is Tris(hydroxymethyl)aminomethane and Ts is tosyl (p-toluenesulfonyl).

### 5.2 Methods For Using The Isoquinoline Derivatives

The invention also includes methods for inhibiting PARS in a cell. PARS, which is also known as poly(ADP-ribose)synthetase, PARP ((poly(ADP-ribose) polymerase, PARP-1, EC 2.4.99) and ADP-ribosyltransferase (ADPRT, EC 2.4.2.30), is a nuclear enzyme that catalyzes a transfer of the ADP ribose moiety of NAD+ to an acceptor protein.

In one embodiment, the method comprises contacting a cell with an Isoquinoline Derivative in an amount effective to inhibit PARS in the cell. In general, any cell having, or capable of having, PARS activity or capable of expressing PARS can be used. The cell can be provided in any form. For example, the cell can be provided *in vitro, ex vivo*, or *in vivo*. PARS activity can be measured using any method known in the art, *e.g*., methods as described in Banasik et al., J. Biol. Chem. 267:1569-75 (1991). Illustrative examples of cells capable of expressing PARS include, but are not limited to, muscle, bone, gum, nerve, brain, liver, kidney, pancreas, lung, heart, bladder, stomach, colon, rectal, small intestine, skin, esophageal, eye, larynx, uterine, ovarian, prostate, tendon, bone marrow, blood, lymph, testicular, vaginal and neoplastic cells.

In accordance with the invention, the Isoquinoline Derivatives are administered to an animal in need of treatment or prevention of a Condition.

### 5.2.1. Treatment or Prevention of an Inflammatory Disease

The Isoquinoline Derivatives can be used to treat an inflammatory disease. Inflammatory diseases can arise where there is an inflammation of the body tissue. These include local inflammatory responses and systemic inflammation. Examples of inflammatory diseases treatable or preventable using the Isoquinoline Derivatives include, but are not limited to, organ transplant rejection; chronic inflammatory diseases of the joints, including arthritis, rheumatoid arthritis, osteoarthritis and bone diseases associated with increased bone resorption; inflammatory bowel diseases such as ileitis, ulcerative colitis, Barrett's syndrome, and Crohn's disease; inflammatory lung diseases such as asthma, adult respiratory distress syndrome, and chronic obstructive airway disease; inflammatory diseases of the eye including corneal dystrophy, trachoma, onchocerciasis, uveitis, sympathetic ophthalmitis and endophthalmitis; chronic inflammatory diseases of the gum, including gingivitis and periodontitis; tuberculosis; leprosy; inflammatory diseases of the kidney including uremic complications, glomerulonephritis and nephrosis; inflammatory diseases of the skin including sclerodermatitis, psoriasis and eczema; inflammatory diseases of the central nervous system, including chronic demyelinating diseases of the nervous system, multiple sclerosis, AIDS-related neurodegeneration and Alzheimers disease, infectious meningitis, encephalomyelitis, Parkinson's disease, Huntington's disease, amyotrophic lateral sclerosis and viral or autoimmune encephalitis; diabetic complications, including, but not limited to, immune-complex vasculitis, systemic lupus erythematosus (SLE); inflammatory diseases of the heart such as cardiomyopathy, ischemic heart disease, hypercholesterolemia, and atherosclerosis; as well as various other diseases that can have significant inflammatory components, including preeclampsia, chronic liver failure, and brain and spinal cord trauma. The inflammatory disease can also be a systemic inflammation of the body, exemplified by gram-positive or gram negative shock, hemorrhagic or anaphylactic shock, or shock induced by cancer chemotherapy in response to pro-inflammatory cytokines, *e.g*., shock associated with pro-inflammatory cytokines. Such shock can be induced, *e.g*., by a chemotherapeutic agent that is administered as a treatment for cancer.

In one embodiment, the inflammatory disease is the inflammatory disease is an inflammatory disease of a joint, a chronic inflammatory disease of the gum, an inflammatory bowel disease, an inflammatory lung disease, an inflammatory disease of the central nervous system, an inflammatory disease of the eye, gram-positive shock, gram negative shock, hemorrhagic shock, anaphylactic shock, traumatic shock or chemotherapeutic shock.

### 5.2.2. Treatment or Prevention of a Reperfusion Injury

The Isoquinoline Derivatives can be used to treat a reperfusion injury. Reperfusion refers to the process whereby blood flow in the blood vessels is resumed following ischemia, such as occurs following constriction or obstruction of the vessel. Reperfusion injury can result following a naturally occurring episode, such as a myocardial infarction, stroke, or during a surgical procedure where blood flow in vessels is intentionally or unintentionally blocked. Examples of reperfusion injuries treatable or preventable using the Isoquinoline Derivatives include, but are not limited to, intestinal reperfusion injury, myocardial reperfusion injury, and reperfusion injury resulting from cardiopulmonary bypass surgery, aortic aneurysm repair surgery, carotid endarterectomy surgery, or hemorrhagic shock.

In one embodiment, the reperfusion injury results from cardiopulmonary bypass surgery, aortic aneurysm repair surgery, carotid endarterectomy surgery or hemorrhagic shock.

### 5.2.3. Treatment or Prevention of a Reoxygenation Injury Resulting from Organ Transplantation

In another embodiment, the reperfusion injury is a reoxygenation injury resulting from organ transplantation. Examples of reoxygenation injuries treatable or preventable using the Isoquinoline Derivatives include, but are not limited to, transplantation of the following organs: heart, lung, liver, kidney, pancreas, intestine, and cornea.

In one embodiment, a reoxygenation injury resulting from organ transplantation occurs during the organ transplantation.

### 5.2.4. Treatment or Prevention of an Ischemic Condition

The Isoquinoline Derivatives can be used to treat an ischemic condition. Examples of ischemic conditions treatable or preventable using the Isoquinoline Derivatives include, but are not limited to, stable angina, unstable angina, myocardial ischemia, hepatic ischemia, mesenteric artery ischemia, intestinal ischemia, critical limb ischemia, chronic critical limb ischemia, cerebral ischemia, acute cardiac ischemia, and an ischemic disease of the central nervous system, such as stroke or cerebral ischemia.

In one embodiment, the ischemic condition is myocardial ischemia, stable angina, unstable angina, stroke, ischemic heart disease or cerebral ischemia.

### 5.2.5. Treatment or Prevention of Renal Failure

The Isoquinoline Derivatives can be used to treat or prevent renal failure.

In one embodiment, the renal failure is chronic renal failure.

In another embodiment, the renal failure is acute renal failure.

### 5.2.6. Treatment or Prevention of a Vascular Disease

The Isoquinoline Derivatives can be used to treat or prevent a vascular disease other than a cardiovascular disease. Examples of such vascular diseases treatable or preventable using the Isoquinoline Derivatives include, but are not limited to, peripheral arterial occlusion, thromboangitis obliterans, Reynaud's disease and phenomenon, acrocyanosis, erythromelalgia, venous thrombosis, varicose veins, arteriovenous fistula, lymphedema, and lipedema.

### 5.2.7. Treatment or Prevention of a Cardiovascular Disease

The Isoquinoline Derivatives can be used to treat or prevent a cardiovascular disease. Examples of cardiovascular diseases treatable or preventable using the Isoquinoline Derivatives include, but are not limited to, chronic heart failure, atherosclerosis, congestive heart failure, circulatory shock, cardiomyopathy, cardiac transplant, myocardial infarction, and a cardiac arrhythmia, such as atrial fibrillation, supraventricular tachycardia, atrial flutter, and paroxysmal atrial tachycardia.

In one embodiment, the cardiovascular disease is chronic heart failure.

In another embodiment, the cardiovascular disease is a cardiac arrhythmia.

In still another embodiment, the cardiac arrhythmia is atrial fibrillation, supraventricular tachycardia, atrial flutter or paroxysmal atrial tachycardia.

### 5.2.8. Treatment or Prevention of Diabetes or a Diabetic Complication

The Isoquinoline Derivatives can be used to treat or prevent diabetes mellitus or its complications. Examples of diabetes treatable or preventable using the Isoquinoline Derivatives include, but are not limited to, Type I diabetes (Insulin Dependent Diabetes Mellitus), Type II diabetes (Non-Insulin Dependent Diabetes Mellitus), gestational diabetes, autoimmune diabetes, insulinopathies, diabetes due to pancreatic disease, diabetes associated with other endocrine diseases (such as Cushing's Syndrome, acromegaly, pheochromocytoma, glucagonoma, primary aldosteronism or somatostatinoma), Type A insulin resistance syndrome, Type B insulin resistance syndrome, lipatrophic diabetes, and diabetes induced by β-cell toxins. The Isoquinoline Derivatives can be used to treat or prevent a diabetic complication. Examples of diabetes mellitus or its complications that are treatable or preventable using the Isoquinoline Derivatives include, but are not limited to, diabetic cataract, glaucoma, retinopathy, nephropathy, (such as microaluminuria and progressive diabetic nephropathy), polyneuropathy, gangrene of the feet, atherosclerotic coronary arterial disease, peripheral arterial disease, nonketotic hyperglycemic-hyperosmolar coma, mononeuropathies, autonomic neuropathy, foot ulcers, joint problems, and a skin or mucous membrane complication (such as an infection, a shin spot, a candidal infection or necrobiosis lipoidica diabeticorumobesity), hyperlipidemia, hypertension, syndrome of insulin resistance, coronary artery disease, retinopathy, diabetic neuropathy, polyneuropathy, mononeuropathies, autonomic neuropathy, a foot ulcer, a joint problem, a fungal infection, a bacterial infection, and cardiomyopathy.

### 5.2.9. Treatment or Prevention of Parkinson's Disease

The Isoquinoline Derivatives can be used to treat or prevent Parkinson's disease.

### 5.2.10. Treatment or Prevention of Cancer

The Isoquinoline Derivatives can be used to treat or prevent cancer. Examples of cancers treatable or preventable using the Isoquinoline Derivatives include, but are not limited to, the cancers disclosed below in Table 1 and metastases thereof.

**TABLE 1**

| | |
|---|---|
| Solid tumors, including but not limited to: | |
| | fibrosarcoma |
| | myxosarcoma |
| | liposarcoma |
| | chondrosarcoma |
| | osteogenic sarcoma |
| | chordoma |
| | angiosarcoma |
| | endotheliosarcoma |
| | lymphangiosarcoma |
| | lymphangioendotheliosarcoma |
| | synovioma |
| | mesothelioma |
| | Ewing's tumor |
| | leiomyosarcoma |
| | rhabdomyosarcoma |
| | colon cancer |
| | colorectal cancer |
| | kidney cancer |
| | pancreatic cancer |
| | bone cancer |
| | breast cancer |
| | ovarian cancer |
| | prostate cancer |
| | esophageal cancer |
| | stomach cancer |
| | oral cancer |
| | nasal cancer |
| | throat cancer |
| | squamous cell carcinoma |
| | basal cell carcinoma |
| | adenocarcinoma |
| | sweat gland carcinoma |
| | sebaceous gland carcinoma |
| | papillary carcinoma |
| | papillary adenocarcinomas |
| | cystadenocarcinoma |
| | medullary carcinoma |
| | bronchogenic carcinoma |
| | renal cell carcinoma |
| | hepatoma |
| | bile duct carcinoma |
| | choriocarcinoma |
| | seminoma |
| | embryonal carcinoma |
| | Wilms' tumor |
| | cervical cancer |
| | uterine cancer |
| | testicular cancer |
| | small cell lung carcinoma |
| | bladder carcinoma |
| | lung cancer |
| | epithelial carcinoma |
| | skin cancer |
| | melanoma |
| | neuroblastoma |
| | retinoblastoma |
| blood-borne cancers, including but not limited to: | |
| | acute lymphoblastic leukemia ("ALL") |
| | acute lymphoblastic B-cell leukemia |
| | acute lymphoblastic T-cell leukemia |
| | acute myeloblastic leukemia ("AML") |
| | acute promyelocytic leukemia ("APL") |
| | acute monoblastic leukemia |
| | acute erythroleukemic leukemia |
| | acute megakaryoblastic leukemia |
| | acute myelomonocytic leukemia |
| | acute nonlymphocyctic leukemia |
| | acute undifferentiated leukemia |
| | chronic myelocytic leukemia ("CML") |
| | chronic lymphocytic leukemia ("CLL") |
| | hairy cell leukemia |
| | multiple myeloma |
| acute and chronic leukemias: | |
| | lymphoblastic |
| | myelogenous |
| | lymphocytic |
| | myelocytic leukemias |
| Lymphomas: | |
| | Hodgkin's disease |
| | non-Hodgkin's Lymphoma |
| | Multiple myeloma |
| | Waldenström's macroglobulinemia |
| | Heavy chain disease |
| | Polycythemia vera |
| CNS and brain cancers: | |
| | glioma |
| | pilocytic astrocytoma |
| | astrocytoma |
| | anaplastic astrocytoma |
| | glioblastoma multiforme |
| | medulloblastoma |
| | craniopharyngioma |
| | ependymoma |
| | pinealoma |
| | hemangioblastoma |
| | acoustic neuroma |
| | oligodendroglioma |
| | meningioma |
| | vestibular schwannoma |
| | adenoma |
| | metastatic brain tumor |
| | meningioma |
| | spinal tumor |
| | medulloblastoma |

In one embodiment the cancer is lung cancer, breast cancer, colorectal cancer, prostate cancer, a leukemia, a lymphoma, a non-Hodgkin's lymphoma, a skin cancer, a brain cancer, a cancer of the central nervous system, ovarian cancer, uterine cancer, stomach cancer, pancreatic cancer, esophageal cancer, kidney cancer, liver cancer, or a head and neck cancer.

In another embodiment the cancer is metastatic cancer.

In still another embodiment, the animal has previously undergone or is presently undergoing treatment for cancer. Such previous treatments include, but are not limited to, prior chemotherapy, radiation therapy, surgery or immunotherapy, such as cancer vaccines.

The Isoquinoline Derivatives are also useful for the treatment or prevention of a cancer caused by a virus. Such viruses include human papilloma virus, which can lead to cervical cancer (see, *e.g*., Hernandez-Avila et al., Archives of Medical Research (1997) 28:265-271); Epstein-Barr virus (EBV), which can lead to lymphoma (see, *e*.*g*., Herrmann et al., J Pathol (2003) 199(2):140-5); hepatitis B or C virus, which can lead to liver carcinoma (see, *e*.*g*., El-Serag, J Clin Gastroenterol (2002) 35(5 Suppl 2):S72-8); human T cell leukemia virus (HTLV)-I, which can lead to T-cell leukemia (see *e*.*g*., Mortreux et al., Leukemia (2003) 17(1):26-38); human herpesvirus-8 infection, which can lead to Kaposi's sarcoma (see, *e.g*., Kadow et al., Curr Opin Investig Drugs (2002) 3(11):1574-9); and Human Immune deficiency Virus (HIV) infection, which can lead to cancer as a consequence of immunodeficiency (see, *e.g*., Dal Maso et al., Lancet Oncol (2003) 4(2):110-9).

The Isoquinoline Derivatives can also be administered to prevent the progression of a cancer, including but not limited to the cancers listed in Table 1. Such prophylactic use includes that in which non-neoplastic cell growth consisting of hyperplasia, metaplasia, or most particularly, dysplasia has occurred.

Alternatively or in addition to the presence of abnormal cell growth characterized as hyperplasia, metaplasia, or dysplasia, the presence of one or more characteristics of a transformed phenotype, or of a malignant phenotype, displayed *in vivo* or displayed *in vitro* by a cell sample from an animal, can indicate the desirability of prophylactic/therapeutic administration of the Isoquinoline Derviatives. Such characteristics of a transformed phenotype include morphology changes, looser substratum attachment, loss of contact inhibition, loss of anchorage dependence, protease release, increased sugar transport, decreased serum requirement, expression of fetal antigens, disappearance of the 250,000 dalton cell surface protein, etc. (see also *id*., at pp. 84-90 for characteristics associated with a transformed or malignant phenotype).

In a specific embodiment, leukoplakia, a benign-appearing hyperplastic or dysplastic lesion of the epithelium, or Bowen's disease, a carcinoma *in situ*, are treatable or preventable according to the present methods.

In another embodiment, fibrocystic disease (cystic hyperplasia, mammary dysplasia, particularly adenosis (benign epithelial hyperplasia)) are treatable or preventable according to the present methods.

In other embodiments, an animal that exhibits one or more of the following predisposing factors for malignancy can be administered an amount of a Isoquinoline Derivative which is effective to treat or prevent cancer: a chromosomal translocation associated with a malignancy (*e*.*g*., the Philadelphia chromosome for chronic myelogenous leukemia, t(14;18) for follicular lymphoma); familial polyposis or Gardner's syndrome; benign monoclonal gammopathy; a first degree kinship with persons having a cancer or precancerous disease showing a Mendelian (genetic) inheritance pattern (*e*.*g*., familial polyposis of the colon, Gardner's syndrome, hereditary exostosis, polyendocrine adenomatosis, medullary thyroid carcinoma with amyloid production and pheochromocytoma, Peutz-Jeghers syndrome, neurofibromatosis of Von Recklinghausen, retinoblastoma, carotid body tumor, cutaneous melanocarcinoma, intraocular melanocarcinoma, xeroderma pigmentosum, ataxia telangiectasia, Chediak-Higashi syndrome, albinism, Fanconi's aplastic anemia, and Bloom's syndrome; and exposure to carcinogens (*e*.*g*., smoking, second-hand smoke exposure, and inhalation of or contacting with certain chemicals).

### 5.2.10.1 Combination Chemotherapy For the Treatment of Cancer

In one embodiment, the present methods for treating cancer or preventing cancer further comprise administering another anticancer agent.

In one embodiment, the present invention provides methods for treating or preventing cancer in a subject, the method comprising the administration of an effective amount of: (i) an Isoquinoline Derivative and (ii) another anticancer agent.

In one embodiment, (i) an Isoquinoline Derivative and (ii) another anticancer agent are administered in doses commonly employed when such agents are used as monotherapy for the treatment of cancer.

In another embodiment, (i) an Isoquinoline Derivative and (ii) another anticancer agent act synergistically and are administered in doses that are less than the doses commonly employed when such agents are used as monotherapy for the treatment of cancer.

The dosage of the (i) an Isoquinoline Derivative, and (ii) another anticancer agent administered as well as the dosing schedule can depend on various parameters, including, but not limited to, the cancer being treated, the patient's general health, and the administering physician's discretion.

An Isoquinoline Derivative can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concurrently with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of the other anticancer agent to a subject in need thereof. In various embodiments, i) an Isoquinoline Derivative, and (ii) another anticancer agent are administered 1 minute apart, 10 minutes apart, 30 minutes apart, less than 1 hour apart, 1 hour to 2 hours apart, 2 hours to 3 hours apart, 3 hours to 4 hours apart, 4 hours to 5 hours apart, 5 hours to 6 hours apart, 6 hours to 7 hours apart, 7 hours to 8 hours apart, 8 hours to 9 hours apart, 9 hours to 10 hours apart, 10 hours to 11 hours apart, 11 hours to 12 hours apart, no more than 24 hours apart, or no more than 48 hours apart. In one embodiment, i) an Isoquinoline Derivative, and (ii) another anticancer agent are administered with 3 hours. In another embodiment, i) an Isoquinoline Derivative, and (ii) another anticancer agent are administered 1 minute to 24 hours apart.

In one embodiment, an effective amount of an Isoquinoline Derivative and an effective amount of another anticancer agent are present in the same composition. In one embodiment, this composition is useful for oral administration. In another embodiment, this composition is useful for intravenous administration.

Cancers that can be treated or prevented by administering an Isoquinoline Derivative and another anticancer agent include, but are not limited to, the list of cancers set forth in Table 1.

In one embodiment, the cancer is brain cancer.

In specific embodiments, the brain cancer is pilocytic astrocytoma, astrocytoma, anaplastic astrocytoma, glioblastoma multiforme or a metastatic brain cancer.

In a specific embodiment, the cancer is melanoma.

In one embodiment, the cancer is metastatic melanoma.

The Isoquinoline Derivative and the other anticancer agent can act additively or synergistically. A synergistic combination of an Isoquinoline Derivative and another anticancer agent might allow the use of lower dosages of one or both of these agents and/or less frequent dosages of one or both of the Isoquinoline Derivatives and other anticancer agents and/or to administer the agents less frequently can reduce any toxicity associated with the administration of the agents to a subject without reducing the efficacy of the agents in the treatment of cancer. In addition, a synergistic effect might result in the improved efficacy of these agents in the treatment of cancer and/or the reduction of any adverse or unwanted side effects associated with the use of either agent alone.

In one embodiment, an Isoquinoline Derivative and another anticancer agent act synergistically when administered in doses typically employed when such agents are sued as monotherapy for the treatment of cancer. In another embodiment, an Isoquinoline Derivative and another anticancer agent act synergistically when administered in doses that are less than doses typically employed when such agents are used as monotherapy for the treatment of cancer.

In one embodiment, the administration of an effective amount of an Isoquinoline Derivative and an effective amount of another anticancer agent inhibits the resistance of a cancer to the other anticancer agent. In one embodiment, the cancer is a tumor.

Suitable other anticancer agents useful in the methods and compositions of the present invention include, but are not limited to temozolomide, a topoisomerase I inhibitor, procarbazine, dacarbazine, gemcitabine, capecitabine, methotrexate, taxol, taxotere, mercaptopurine, thioguanine, hydroxyurea, cytarabine, cyclophosphamide, ifosfamide, nitrosoureas, cisplatin, carboplatin, mitomycin, dacarbazine, procarbizine, etoposide, teniposide, campathecins, bleomycin, doxorubicin, idarubicin, daunorubicin, dactinomycin, plicamycin, mitoxantrone, L-asparaginase, doxorubicin, epirubicin, 5-fluorouracil, taxanes such as docetaxel and paclitaxel, leucovorin, levamisole, irinotecan, estramustine, etoposide, nitrogen mustards, BCNU, nitrosoureas such as carmustine and lomustine, vinca alkaloids such as vinblastine, vincristine and vinorelbine, platinum complexes such as cisplatin, carboplatin and oxaliplatin, imatinib mesylate, hexamethylmelamine, topotecan, tyrosine kinase inhibitors, tyrphostins herbimycin A, genistein, erbstatin, and lavendustin A.

In one embodiment, the other anticancer agents useful in the methods and compositions of the present invention include, but are not limited to, a drug listed in Table 2 or a pharmaceutically acceptable salt thereof.

**TABLE 2**

| Alkylating agents | |
|---|---|
| Nitrogen mustards: | Cyclophosphamide |
| | Ifosfamide |
| | Trofosfamide |
| | Chlorambucil |
| Nitrosoureas: | Carmustine (BCNU) |
| | Lomustine (CCNU) |
| Alkylsulphonates: | Busulfan |
| | Treosulfan |
| Triazenes: | Dacarbazine |
| | Procarbazine |
| | Temozolomide |
| Platinum containing complexes: | Cisplatin |
| | Carboplatin |
| | Aroplatin |
| | Oxaliplatin |

| Plant Alkaloids | |
|---|---|
| Vinca alkaloids: | Vincristine |
| | Vinblastine |
| | Vindesine |
| | Vinorelbine |
| Taxoids: | Paclitaxel |
| | Docetaxel |

| DNA Topoisomerase Inhibitors | |
|---|---|
| Epipodophyllins: | Etoposide |
| | Teniposide |
| | Topotecan |
| | Irinotecan |
| | 9-aminocamptothecin |
| | Camptothecin |
| | Crisnatol |
| Mitomycins: | Mitomycin C |
| | Anti-metabolites |

| Anti-folates: | |
|---|---|
| DHFR inhibitors: | Methotrexate |
| | Trimetrexate |
| IMP dehydrogenase Inhibitors: | Mycophenolic acid |
| | Tiazofurin |
| | Ribavirin |
| | EICAR |
| Ribonuclotide reductase | Hydroxyurea |
| Inhibitors: | |
| | Deferoxamine |
| Pyrimidine analogs: | |
| Uracil analogs: | 5-Fluorouracil |
| | Fluoxuridine |
| | Doxifluridine |
| | Ralitrexed |
| Cytosine analogs: | Cytarabine (ara C) |
| | Cytosine arabinoside |
| | Fludarabine |
| | Gemcitabine |
| | Capecitabine |
| Purine analogs: | Mercaptopurine |
| | Thioguanine |
| | O-6-benzylguanine |
| DNA Antimetabolites: | 3-HP |
| | 2'-deoxy-5-fluorouridine |
| | 5-HP |
| | alpha-TGDR |
| | aphidicolin glycinate |
| | ara-C |
| | 5-aza-2'-deoxycytidine |
| | beta-TGDR |
| | cyclocytidine |
| | guanazole |
| | inosine glycodialdehyde |
| | macebecin II |
| | Pyrazoloimidazole |
| Hormonal therapies: | |
| Receptor antagonists: | |
| Anti-estrogen: | Tamoxifen |
| | Raloxifene |
| | Megestrol |
| LHRH agonists: | Goscrclin |
| | Leuprolide acetate |
| Anti-androgens: | Flutamide |
| | Bicalutamide |
| Retinoids/Deltoids | |
| | Cis-retinoic acid |
| Vitamin A derivative: | All-trans retinoic acid (ATRA-IV) |
| Vitamin D3 analogs: | EB 1089 |
| | CB 1093 |
| | KH 1060 |
| Photodynamic therapies: | Vertoporfin (BPD-MA) |
| | Phthalocyanine |
| | Photosensitizer Pc4 |
| | Demethoxy-hypocrellin A (2BA-2-DMHA) |
| Cytokines: | Interferon-α |
| | Interferon-β |
| | Interferon-γ |
| | Tumor necrosis factor |
| | Interleukin-2 |
| Angiogenesis Inhibitors: | Angiostatin (plasminogen fragment) |
| | antiangiogenic antithrombin III |
| | Angiozyme |
| | ABT-627 |
| | Bay 12-9566 |
| | Benefin |
| | Bevacizumab |
| | BMS-275291 |
| | cartilage-derived inhibitor (CDI) |
| | CAI |
| | CD59 complement fragment |
| | CEP-7055 |
| | Col 3 |
| | Combretastatin A-4 |
| | Endostatin (collagen XVIII fragment) |
| | Fibronectin fragment |
| | Gro-beta |
| | Halofuginone |
| | Heparinases |
| | Heparin hexasaccharide fragment |
| | HMV833 |
| | Human chorionic gonadotropin |
| | (hCG) |
| | IM-862 |
| | Interferon alpha/beta/gamma |
| | Interferon inducible protein (IP-10) |
| | Interleukin-12 |
| | Kringle 5 (plasminogen fragment) |
| | Marimastat |
| | Metalloproteinase inhibitors |
| | (TIMPs) |
| | 2-Methoxyestradiol |
| | MMI 270 (CGS 27023A) |
| | MoAb IMC-1C11 |
| | Neovastat |
| | NM-3 |
| | Panzem |
| | PI-88 |
| | Placental ribonuclease inhibitor |
| | Plasminogen activator inhibitor |
| | Platelet factor-4 (PF4) |
| | Prinomastat |
| | Prolactin 16kD fragment |
| | Proliferin-related protein (PRP) |
| | PTK 787/ZK 222594 |
| | Retinoids |
| | Solimastat |
| | Squalamine |
| | SS 3304 |
| | SU 5416 |
| | SU6668 |
| | SU11248 |
| | Tetrahydrocortisol-S |
| | Tetrathiomolybdate |
| | Thalidomide |
| | Thrombospondin-1 (TSP-1) |
| | TNP-470 |
| | Transforming growth factor-beta (TGF-b) |
| | Vasculostatin |
| | Vasostatin (calreticulin fragment) |
| | ZD6126 |
| | ZD 6474 |
| | farnesyl transferase inhibitors |
| | (FTI) |
| | Bisphosphonates |
| Antimitotic agents: | Allocolchicine |
| | Halichondrin B |
| | Colchicine |
| | colchicine derivative |
| | dolstatin 10 |
| | Maytansine |
| | Rhizoxin |
| | Thiocolchicine |
| | trityl cysteine |
| Others: | |
| Isoprenylation inhibitors: | |
| Dopaminergic neurotoxins: | 1-methyl-4-phenylpyridinium ion |
| Cell cycle inhibitors: | Staurosporine |
| Actinomycins: | Actinomycin D |
| | Dactinomycin |
| Bleomycins: | Bleomycin A2 |
| | Bleomycin B2 |
| | Peplomycin |
| Anthracyclines: | Daunorubicin |
| | Doxorubicin |
| | Idarubicin |
| | Epirubicin |
| | Pirarubicin |
| | Zorubicin |
| | Mitoxantrone |
| MDR inhibitors: | Verapamil |
| Ca²⁺ATPase inhibitors: | Thapsigargin |

Other anticancer agents that can be used in the compositions and methods of the present invention include, but are not limited to: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine hydrochloride; hydroxyurea; idarubicin hydrochloride; ifosfamide; ilmofosine; interleukin 2 (including recombinant interleukin 2, or rIL2), interferon alfa-2α; interferon alfa-2β; interferon alfa-n1 ; interferon alfa-n3; interferon beta-Iα; interferon gamma-Iβ; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride.

Further anticancer drugs that can be used in the methods and compositions of the invention include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta Lactam Derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-acytidine; dihydrotaxol; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum complexes; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agents; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum complexes; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

In another embodiment, the other anticancer agent is interferon-α.

In another embodiment, the other anticancer agent is interleukin-2.

In one embodiment, the other anticancer agent is an alkylating agent, such as a nitrogen mustard, a nitrosourea, an alkylsulfonate, a triazene, or a platinum-containing agent.

In another embodiment, the other anticancer agent is a triazene alkylating agent.

In a specific embodiment, the other anticancer agent is temozolomide.

Temozolomide can be administered to a subject at dosages ranging from about 60 mg/m² (of a subject's body surface area) to about 250 mg/m² and from about 100 mg/m² to about 200 mg/m². In specific embodiments, the dosages of temozolomide are about 10 mg/m², about 1 mg/m², about 5 mg/m², about 10 mg/m², about 20 mg/m², about 30 mg/m², about 40 mg/m², about 50 mg/m², about 60 mg/m², about 70 mg/m², about 80 mg/m², about 90 mg/m², about 100 mg/m², about 110 mg/m², about 120 mg/m², about 130 mg/m², about 140 mg/m², about 150 mg/m², about 160 mg/m², about 170 mg/m², about 180 mg/m², about 190 mg/m², about 200 mg/m², about 210 mg/m², about 220 mg/m², about 230 mg/m², about 240 mg/m², or about 250 mg/m².

In a particular embodiment, temozolomide is administered orally.

In one embodiment, temozolomide is administered orally to a subject at a dose ranging from about 150 mg/m² to about 200 mg/m².

In another embodiment, temozolomide is administered orally to a subject once per day for five consecutive days at a dose ranging from about 150 mg/m² to about 200 mg/m².

In a specific embodiment, temozolomide is administered orally to a subject once per day for five consecutive days at a dose ranging from about 150 mg/m² to about 200 mg/m² on days 1-5, then again orally once per day for five consecutive days on days 28-32 at a dose ranging from about 150 mg/m² to about 200 mg/m², then again orally once per day for five consecutive days on days 55-59 at a dose ranging from about 150 mg/m² to about 200 mg/m².

In a specific embodiment, the other anticancer agent is procarbazine.

Procarbazine can be administered to a subject at dosages ranging from about 50 mg/m² (of a subject's body surface area) to about 100 mg/m² and from about 60 mg/m² to about 100 mg/m². In specific embodiments, the dosages of procarbazine are about 10 mg/m², about 1 mg/m², about 5 mg/m², about 10 mg/m², about 20 mg/m², about 30 mg/m², about 40 mg/m², about 50 mg/m², about 60 mg/m², about 70 mg/m², about 80 mg/m², about 90 mg/m², about 100 mg/m², about 110 mg/m², about 120 mg/m², about 130 mg/m², about 140 mg/m², about 150 mg/m², about 160 mg/m², about 170 mg/m², about 180 mg/m², about 190 mg/m², about 200 mg/m², about 210 mg/m², about 220 mg/m², about 230 mg/m², about 240 mg/m², about 250 mg/m², about 260 mg/m², about 270 mg/m², about 280 mg/m², about 290 mg/m², about 300 mg/m², about 310 mg/m², about 320 mg/m², about 330 mg/m², about 340 mg/m², about 350 mg/m², about 360 mg/m², about 370 mg/m², about 380 mg/m², about 390 mg/m², about 400 mg/m², about 410 mg/m², about 420 mg/m², about 430 mg/m², about 440 mg/m², about 450 mg/m², about 460 mg/m², about 470 mg/m², about 480 mg/m², about 490 mg/m², or about 500 mg/m²_{.}

In a particular embodiment, procarbazine is administered intravenously.

In one embodiment, procarbazine is administered intravenously to a subject at a dose ranging from about 50 mg/m² to about 100 mg/m².

In another embodiment, procarbazine is administered intravenously to a subject once per day for five consecutive days at a dose ranging from about 50 mg/m² to about 100 mg/m².

In a specific embodiment, procarbazine is administered intravenously to a subject once per day for five consecutive days at a dose ranging from about 50 mg/m² to about 100 mg/m² on days 1-5, then again intravenously once per day for five consecutive days on days 28-32 at a dose ranging from about 50 mg/m² to about 100 mg/m², then again intravenously once per day for five consecutive days on days 55-59 at a dose ranging from about 50 mg/m² to about 100 mg/m².

In another embodiment, procarbazine is administered intravenously to a subject once at a dose ranging from about 50 mg/m² to about 100 mg/m².

In a specific embodiment, the other anticancer agent is dacarbazine.

Dacarbazine can be administered to a subject at dosages ranging from about 60 mg/m² (of a subject's body surface area) to about 250 mg/m² and from about 150 mg/m² to about 250 mg/m². In specific embodiments, the dosages of dacarbazine are about 10 mg/m², about 1 mg/m², about 5 mg/m², about 10 mg/m², about 20 mg/m², about 30 mg/m², about 40 mg/m², about 50 mg/m², about 60 mg/m², about 70 mg/m², about 80 mg/m², about 90 mg/m², about 100 mg/m², about 110 mg/m², about 120 mg/m², about 130 mg/m², about 140 mg/m², about 150 mg/m², about 160 mg/m², about 170 mg/m², about 180 mg/m², about 190 mg/m², about 200 mg/m², about 210 mg/m², about 220 mg/m², about 230 mg/m², about 240 mg/m², about 250 mg/m², about 260 mg/m², about 270 mg/m², about 280 mg/m², about 290 mg/m², about 300 mg/m², about 310 mg/m², about 320 mg/m², about 330 mg/m², about 340 mg/m², about 350 mg/m², about 360 mg/m², about 370 mg/m², about 380 mg/m², about 390 mg/m², about 400 mg/m², about 410 mg/m², about 420 mg/m², about 430 mg/m², about 440 mg/m², about 450 mg/m², about 460 mg/m², about 470 mg/m², about 480 mg/m², about 490 mg/m², or about 500 mg/m².

In a particular embodiment, dacarbazine is administered intravenously.

In one embodiment, dacarbazine is administered intravenously to a subject at a dose ranging from about 150 mg/m² to about 250 mg/m².

In another embodiment, dacarbazine is administered intravenously to a subject once per day for five consecutive days at a dose ranging from about 150 mg/m² to about 250 mg/m².

In a specific embodiment, dacarbazine is administered intravenously to a subject once per day for five consecutive days at a dose ranging from about 150 mg/m² to about 250 mg/m² on days 1-5, then again intravenously once per day for five consecutive days on days 28-32 at a dose ranging from about 150 mg/m² to about 250 mg/m², then again intravenously once per day for five consecutive days on days 55-59 at a dose ranging from about 150 mg/m² to about 250 mg/m².

In one embodiment, dacarbazine is administered intravenously to a subject once at a dose ranging from about 150 mg/m² to about 250 mg/m².

In one embodiment, the other anticancer agent is a Topoisomerase I inhibitor, such as etoposide, teniposide, topotecan, irinotecan, 9-aminocamptothecin, camptothecin, or crisnatol.

In a specific embodiment, the other anticancer agent is irinotecan.

Irinotecan can be administered to a subject at dosages ranging from about 50 mg/m² (of a subject's body surface area) to about 150 mg/m² and from about 75 mg/m² to about 150 mg/m². In specific embodiments, the dosages of irinotecan are about 10 mg/m², about 1 mg/m², about 5 mg/m², about 10 mg/m², about 20 mg/m², about 30 mg/m², about 40 mg/m², about 50 mg/m², about 60 mg/m², about 70 mg/m², about 80 mg/m², about 90 mg/m², about 100 mg/m², about 110 mg/m², about 120 mg/m², about 130 mg/m², about 140 mg/m², about 150 mg/m², about 160 mg/m², about 170 mg/m², about 180 mg/m², about 190 mg/m², about 200 mg/m², about 210 mg/m², about 220 mg/m², about 230 mg/m², about 240 mg/m², about 250 mg/m², about 260 mg/m², about 270 mg/m², about 280 mg/m², about 290 mg/m², about 300 mg/m², about 310 mg/m², about 320 mg/m², about 330 mg/m², about 340 mg/m², about 350 mg/m², about 360 mg/m², about 370 mg/m², about 380 mg/m², about 390 mg/m², about 400 mg/m², about 410 mg/m², about 420 mg/m², about 430 mg/m², about 440 mg/m², about 450 mg/m², about 460 mg/m², about 470 mg/m², about 480 mg/m², about 490 mg/m², or about 500 mg/m².

In a particular embodiment, irinotecan is administered intravenously.

In one embodiment, irinotecan is administered intravenously to a subject at a dose ranging from about 50 mg/m² to about 150 mg/m².

In another embodiment, irinotecan is administered intravenously to a subject once per day for five consecutive days at a dose ranging from about 50 mg/m² to about 150 mg/m².

In a specific embodiment, irinotecan is administered intravenously to a subject once per day for five consecutive days at a dose ranging from about 50 mg/m² to about 150 mg/m² on days 1-5, then again intravenously once per day for five consecutive days on days 28-32 at a dose ranging from about 50 mg/m² to about 150 mg/m², then again intravenously once per day for five consecutive days on days 55-59 at a dose ranging from about 50 mg/m² to about 150 mg/m².

In one embodiment, the invention provides administration of an effective amount of: (i) an Isoquinoline Derivative (ii) one or more other anticancer agents.

In one embodiment, (i) an Isoquinoline Derivative and (ii) one or more other anticancer agents are administered in doses commonly employed when such agents are used as monotherapy for the treatment of cancer.

In another embodiment, (i) an Isoquinoline Derivative and (ii) one or more other anticancer agents act synergistically and are administered in doses that are less than the doses commonly employed when such agents are used as monotherapy for the treatment of cancer.

The dosage of the (i) an Isoquinoline Derivative and (ii) one or more other anticancer agents administered as well as the dosing schedule can depend on various parameters, including, but not limited to, the cancer being treated, the patient's general health, and the administering physician's discretion.

In one embodiment, the other anticancer agent is O-6-benzylguanine.

In another embodiment, the other anticancer agent is O-6-benzylguanine and temozolomide.

In another embodiment, the other anticancer agent is O-6-benzylguanine and procarbazine.

In still another embodiment, the other anticancer agent is O-6-benzylguanine and dacarbazine.

### 5.2.10.2. Multi-Therapy For Cancer

The Isoquinoline Derivatives can be administered to an animal that has undergone or is currently undergoing one or more additional anticancer therapies including, but not limited to, surgery, radiation therapy, or immunotherapy, such as cancer vaccines.

In one embodiment, the invention provides methods for treating or preventing cancer comprising administering to an animal in need thereof (a) an amount of a Isoquinoline Derivative effective to treat or prevent cancer; and (b) another anticancer therapy including, but not limited to, surgery, radiation therapy, or immunotherapy, such as a cancer vaccine.

In one embodiment, the other anticancer therapy is radiation therapy.

In another embodiment, the other anticancer therapy is surgery.

In still another embodiment, the other anticancer therapy is immunotherapy.

In a specific embodiment, the present methods for treating or preventing cancer comprise administering an Isoquinoline Derivative and radiation therapy. The radiation therapy can be administered concurrently with, prior to, or subsequent to the Isoquinoline Derivative, in one embodiment, at least an hour, five hours, 12 hours, a day, a week, a month, in another embodiment, several months (e.g., up to three months), prior or subsequent to administration of the Isoquinoline Derivatives.

Where the other anticancer therapy is radiation therapy, any radiation therapy protocol can be used depending upon the type of cancer to be treated. For example, but not by way of limitation, X-ray radiation can be administered; in particular, high-energy megavoltage (radiation of greater that 1 MeV energy) can be used for deep tumors, and electron beam and orthovoltage X-ray radiation can be used for skin cancers. Gamma-ray emitting radioisotopes, such as radioactive isotopes of radium, cobalt and other elements, can also be administered.

Additionally, in one embodiment the invention provides methods of treatment of cancer using an Isoquinoline Derivative as an alternative to chemotherapy or radiation therapy where the chemotherapy or the radiation therapy results in negative side effects, in the subject being treated. The subject being treated can, optionally, be treated with another anticancer therapy such as surgery, radiation therapy, or immunotherapy.

The Isoquinoline Derivative can also be used *in vitro* or *ex vivo*, such as for the treatment of certain cancers, including, but not limited to leukemias and lymphomas, such treatment involving autologous stem cell transplants. This can involve a process in which the subject's autologous hematopoietic stem cells are harvested and purged of all cancer cells, the subject's remaining bone-marrow cell population is then eradicated via the administration of an Isoquinoline Derivative and/or radiation, and the resultant stem cells are infused back into the subject. Supportive care can be subsequently provided while bone marrow function is restored and the subject recovers.

### 5.3 Therapeutic/Prophylactic Administration

The invention also includes pharmaceutical compositions useful for treating or preventing a Condition. The compositions are suitable for internal use and comprise an effective amount of an Isoquinoline Derivative and a physiologically acceptable carrier or vehicle.

The Isoquinoline Derivatives can be administered in amounts that are effective to treat or prevent a Condition in an animal.

Administration of the Isoquinoline Derivatives can be accomplished via any mode of administration for therapeutic agents. These modes include systemic or local administration such as oral, nasal, parenteral, transdermal, subcutaneous, vaginal, buccal, rectal or topical administration modes. In some instances, administration will result in the release of an Isoquinoline Derivative into the bloodstream.

In one embodiment, the Isoquinoline Derivatives are administered orally.

Depending on the intended mode of administration, the compositions can be in solid, semi-solid or liquid dosage form, such as, for example, injectables, tablets, suppositories, pills, time-release capsules, elixirs, tinctures, emulsions, syrups, powders, liquids, suspensions, or the like, preferably in unit dosages and consistent with conventional pharmaceutical practices. Likewise, they can also be administered in intravenous (both bolus and infusion), intraperitoneal, subcutaneous or intramuscular form, all using forms well known to those skilled in the pharmaceutical arts.

Illustrative pharmaceutical compositions are tablets and gelatin capsules comprising an Isoquinoline Derivative and a physiologically acceptable carrier or vehicle. Illustrative carriers or vehicles include a) a diluent, *e*.*g*., lactose, dextrose, sucrose, mannitol, sorbitol, cellulose, sodium, saccharin, glucose and/or glycine; b) a lubricant, *e*.*g*., silica, talcum, stearic acid, its magnesium or calcium salt, sodium oleate, sodium stearate, magnesium stearate, sodium benzoate, sodium acetate, sodium chloride and/or polyethylene glycol; for tablets also; c) a binder, *e*.*g*., magnesium aluminum silicate, starch paste, gelatin, tragacanth, methylcellulose, sodium carboxymethylcellulose, magnesium carbonate, natural sugars such as glucose or beta-lactose, corn sweeteners, natural and synthetic gums such as acacia, tragacanth or sodium alginate, waxes and/or polyvinylpyrrolidone, if desired; d) a disintegrant, *e*.*g*., starches, agar, methyl cellulose, bentonite, xanthan gum, algiic acid or its sodium salt, or effervescent mixtures; and/or e) absorbent, colorant, flavorant and sweetener.

Liquid, particularly injectable, compositions can, for example, be prepared by dissolution, dispersion, etc. For example, the Isoquinoline Derivative is dissolved in or mixed with a pharmaceutically acceptable solvent such as, for example, water, saline, aqueous dextrose, glycerol, ethanol, and the like, to thereby form an injectable isotonic solution or suspension.

The Isoquinoline Derivatives can be also formulated as a suppository that can be prepared from fatty emulsions or suspensions; using polyalkylene glycols such as propylene glycol, as the carrier.

The Isoquinoline Derivatives can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in United States Patent No. 5,262,564.

Isoquinoline Derivatives can also be delivered by the use of monoclonal antibodies as individual carriers to which the Isoquinoline Derivative molecules are coupled. The Isoquinoline Derivatives can also be coupled with soluble polymers as targetable drug carriers. Such polymers can include polyvinylpyrrolidone, pyran copolymer, polyhydroxypropylmethacrylamide-phenol, polyhydroxyethylaspanamidephenol, or polyethyleneoxidepolylysine substituted with palmitoyl residues. Furthermore, the Isoquinoline Derivatives can be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.

Parental injectable administration can be used for subcutaneous, intramuscular or intravenous injections and infusions. Injectables can be prepared in conventional forms, either as liquid solutions or suspensions or solid forms suitable for dissolving in liquid prior to injection.

One embodiment, for parenteral administration employs the implantation of a slow-release or sustained-released system, according to U.S. Pat. No. 3,710,795, incorporated herein by reference.

The compositions can be sterilized or contain non-toxic amounts of adjuvants, such as preserving, stabilizing, wetting or emulsifying agents, solution promoters, salts for regulating the osmotic pressure pH buffering agents, and other substances, including, but not limited to, sodium acetate or triethanolamine oleate. In addition, they can also contain other therapeutically valuable substances.

Compositions can be prepared according to conventional mixing, granulating or coating methods, respectively, and the present pharmaceutical compositions can contain from about 0.1% to about 99%, preferably from about 1% to about 70% of the Isoquinoline Derivative by weight or volume.

The dosage regimen utilizing the Isoquinoline Derivative can be selected in accordance with a variety of factors including type, species, age, weight, sex and medical condition of the animal; the severity of the condition to be treated; the route of administration; the renal or hepatic function of the animal; and the particular Isoquinoline Derivative employed. A person skilled in the art can readily determine and prescribe the effective amount of the drug useful for treating or preventing a Condition.

Effective dosage amounts of the Isoquinoline Derivatives, when administered to an animal, range from about 0.05 to about 1000 mg of Isoquinoline Derivative per day. Compositions for *in vivo* or *in vitro* use can contain about 0.5, 1.0, 2.5, 5.0, 10.0, 15.0, 25.0, 50.0, 100.0, 250.0, 500.0 or 1000.0 mg of Isoquinoline Derivative. In one embodiment, the compositions are in the form of a tablet that can be scored. Effective plasma levels of the Isoquinoline Derivatives can range from about 0.002 mg to about 50 mg per kg of body weight per day. The amount of an Isoquinoline Derivative that is effective in the treatment or prevention of a Condition can be determined by clinical techniques that are known to those of skill in the art. In addition, in vitro and in vivo assays can optionally be employed to help identify optimal dosage ranges. The precise dose to be employed can also depend on the route of administration, and the seriousness of the condition being treated and can be decided according to the judgment of the practitioner and each patient's circumstances in view of, e.g., published clinical studies. Suitable effective dosage amounts, however, can range from about 10 micrograms to about 5 grams about every 4 h, although they are typically about 500 mg or less per every 4 hours. In one embodiment the effective dosage is about 0.01 mg, 0.5 mg, about 1 mg, about 50 mg, about 100 mg, about 200 mg, about 300 mg, about 400 mg, about 500 mg, about 600 mg, about 700 mg, about 800 mg, about 900 mg, about 1 g, about 1.2 g, about 1.4 g, about 1.6 g, about 1.8 g, about 2.0 g, about 2.2 g, about 2.4 g, about 2.6 g, about 2.8 g, about 3.0 g, about 3.2 g, about 3.4 g, about 3.6 g, about 3.8 g, about 4.0g, about 4.2 g, about 4.4 g, about 4.6 g, about 4.8 g, and about 5.0 g, every 4 hours. Equivalent dosages can be administered over various time periods including, but not limited to, about every 2 hours, about every 6 hours, about every 8 hours, about every 12 hours, about every 24 hours, about every 36 hours, about every 48 hours, about every 72 hours, about every week, about every two weeks, about every three weeks, about every month, and about every two months. The effective dosage amounts described herein refer to total amounts administered; that is, if more than one Isoquinoline Derivative is administered, the effective dosage amounts correspond to the total amount administered.

The dosage regimen utilizing the Isoquinoline Derivative can be selected in accordance with a variety of factors including type, species, age, weight, sex and medical condition of the subject; the severity of the condition to be treated; the route of administration; the renal or hepatic function of the subject; and the particular Isoquinoline Derivative employed. A person skilled in the art can readily determine and prescribe the effective amount of the drug required to prevent, counter or arrest the progress of the Condition.

Isoquinoline Derivatives can be administered in a single daily dose, or the total daily dosage can be administered in divided doses of two, three or four times daily. Furthermore, Isoquinoline Derivatives can be administered in intranasal form via topical use of suitable intranasal vehicles, or via transdermal routes, using those forms of transdermal skin patches well known to those of ordinary skill in that art. To be administered in the form of a transdermal delivery system, the dosage administration can be continuous rather than intermittent throughout the dosage regimen. Other illustrative topical preparations include creams, ointments, lotions, aerosol sprays and gels, wherein the concentration of Isoquinoline Derivative ranges from about 0.1% to about 15%, w/w or w/v.

In one embodiment, the compositions comprise an amount of each of an Isoquinoline Derivative and another anticancer agent which together are effective to treat or prevent cancer. In another embodiment, the amount of Isoquinoline Derivative and another anticancer agent is at least about 0.01% of the combined combination chemotherapy agents by weight of the composition. When intended for oral administration, this amount can be varied from about 0.1% to about 80% by weight of the composition. Some oral compositions can comprise from about 4% to about 50% of an Isoquinoline Derivative and another anticancer agent. Other compositions of the present invention are prepared so that a parenteral dosage unit contains from about 0.01% to about 2% by weight of the composition.

The Isoquinoline Derivatives can be assayed *in vitro* or *in vivo* for the desired therapeutic or prophylactic activity prior to use in humans. Animal model systems can be used to demonstrate safety and efficacy.

The present methods for treating or preventing a Condition in an animal in need thereof can further comprise administering another prophylactic or therapeutic agent to the subject being administered an Isoquinoline Derivative. In one embodiment the other prophylactic or therapeutic agent is administered in an effective amount. The other prophylactic or therapeutic agent includes, but is not limited to, an anti-inflammatory agent, an anti-renal failure agent, an anti-diabetic agent, and anti-cardiovasculare disease agent, an antiemetic agent, a hematopoietic colony stimulating factor, an anxiolytic agent, and an analgesic agent.

In one embodiment, the Isoquinoline Derivative can be administered prior to, concurrently with, or after an anti-inflammatory agent, or on the same day, or within 1 hour, 2 hours, 12 hours, 24 hours, 48 hours or 72 hours of each other.

In another embodiment, the Isoquinoline Derivative can be administered prior to, concurrently with, or after an anti-renal failure agent, or on the same day, or within 1 hour, 2 hours, 12 hours, 24 hours, 48 hours or 72 hours of each other.

In still another embodiment, the Isoquinoline Derivative can be administered prior to, concurrently with, or after an anti-diabetic agent, or on the same day, or within 1 hour, 2 hours, 12 hours, 24 hours, 48 hours or 72 hours of each other.

In yet another embodiment, the Isoquinoline Derivative can be administered prior to, concurrently with, or after an anti-cardiovascular disease agent, or on the same day, or within 1 hour, 2 hours, 12 hours, 24 hours, 48 hours or 72 hours of each other.

In a further embodiment, the Isoquinoline Derivative can be administered prior to, concurrently with, or after an antiemetic agent, or on the same day, or within 1 hour, 2 hours, 12 hours, 24 hours, 48 hours or 72 hours of each other.

In another embodiment, the Isoquinoline Derivative can be administered prior to, concurrently with, or after a hematopoietic colony stimulating factor, or on the same day, or within 1 hour, 2 hours, 12 hours, 24 hours, 48 hours, 72 hours, 1 week, 2 weeks, 3 weeks or 4 weeks of each other.

In still embodiment, the Isoquinoline Derivative can be administered prior to, concurrently with, or after an opioid or non-opioid analgesic agent, or on the same day, or within 1 hour, 2 hours, 12 hours, 24 hours, 48 hours or 72 hours of each other.

In yet another embodiment, the Isoquinoline Derivative can be administered prior to, concurrently with, or after an anxiolytic agent, or on the same day, or within 1 hour, 2 hours, 12 hours, 24 hours, 48 hours or 72 hours of each other.

Effective amounts of the other therapeutic agents are well known to those skilled in the art. However, it is well within the skilled artisan's purview to determine the other therapeutic agent's optimal effective amount range. In one embodiment of the invention, where, another therapeutic agent is administered to a subject, the effective amount of the Isoquinoline Derivative is less than its effective amount would be where the other therapeutic agent is not administered. In this case, without being bound by theory, it is believed that The Isoquinoline Derivative and the other therapeutic agent act synergistically to treat or prevent a Condition.

Anti-inflammatory agents useful in the methods of the present invention include but are not limited to adrenocorticosteroids, such as cortisol, cortisone, fludrocortisone, prednisone, prednisolone, 6a-methylprednisolone, triamcinolone, betamethasone, and dexamethasone; and non-steroidal anti-inflammatory agents (NSAIDs), such as aspirin, acetaminophen, indomethacin, sulindac, tolmetin, diclofenac, ketorolac, ibuprofen, naproxen, flurbiprofen, ketoprofen, fenoprofen, oxaprozin, mefenamic acid, meclofenamic acid, piroxicam, meloxicam, nabumetone, rofecoxib, celecoxib, etodolac, and nimesulide.

Anti-renal failure agents useful in the methods of the present invention include include but are not limited to ACE (angiotensin-converting enzyme) inhibitors, such as captopril, enalaprilat, lisinopril, benazepril, fosinopril, trandolapril, quinapril, and ramipril; diuretics, such as mannitol, glycerin, furosemide, toresemide, tripamide, chlorothiazide, methyclothiazide, indapamide, amiloride, and spironolactone; and fibric acid agents, such as clofibrate, gemfibrozil, fenofibrate, ciprofibrate, and bezafibrate.

Anti-diabetic agents useful in the methods of the present invention include include but are not limited to glucagons; somatostatin; diazoxide; sulfonylureas, such as tolbutamide, acetohexamide, tolazamide, chloropropamide, glybenclamide, glipizide, gliclazide, and glimepiride; insulin secretagogues, such as repaglinide, and nateglinide; biguanides, such as metformin and phenformin; thiazolidinediones, such as pioglitazone, rosiglitazone, and troglitazone; and α-glucosidase inhibitors, such as acarbose and miglitol.

Anti-cardiovascular disease agents useful in the methods of the present invention include include but are not limited to carnitine; thiamine; and muscarinic receptor antagonists, such as atropine, scopolamine, homatropine, tropicamide, pirenzipine, ipratropium, tiotropium, and tolterodine.

Antiemetic agents useful in the methods of the present invention include include, but are not limited to, metoclopromide, domperidone, prochlorperazine, promethazine, chlorpromazine, trimethobenzamide, ondansetron, granisetron, hydroxyzine, acetylleucine monoethanolamine, alizapride, azasetron, benzquinamide, bietanautine, bromopride, buclizine, clebopride, cyclizine, dimenhydrinate, diphenidol, dolasetron, meclizine, methallatal, metopimazine, nabilone, oxyperndyl, pipamazine, scopolamine, sulpiride, tetrahydrocannabinol, thiethylperazine, thioproperazine, tropisetron, and mixtures thereof.

Hematopoietic colony stimulating factors useful in the methods of the present invention include, but are not limited to, filgrastim, sargramostim, molgramostim and epoietin alfa.

Opioid analgesic agents useful in the methods of the present invention include, but are not limited to, morphine, heroin, hydromorphone, hydrocodone, oxymorphone, oxycodone, metopon, apomorphine, normorphine, etorphine, buprenorphine, meperidine, lopermide, anileridine, ethoheptazine, piminidine, betaprodine, diphenoxylate, fentanil, sufentanil, alfentanil, remifentanil, levorphanol, dextromethorphan, phenazocine, pentazocine, cyclazocine, methadone, isomethadone and propoxyphene.

Non-opioid analgesic agents useful in the methods of the present invention include, but are not limited to, aspirin, celecoxib, rofecoxib, diclofinac, diflusinal, etodolac, fenoprofen, flurbiprofen, ibuprofen, ketoprofen, indomethacin, ketorolac, meclofenamate, mefanamic acid, nabumetone, naproxen, piroxicam and sulindac.

Anxiolytic agents useful in the methods of the present invention include, but are not limited to, buspirone, and benzodiazepines such as diazepam, lorazepam, oxazapam, chlorazepate, clonazepam, chlordiazepoxide and alprazolam.

### 5.4 Kits

The invention encompasses kits that can simplify the administration of an Isoquinoline Derivative to a subject.

A typical kit of the invention comprises a unit dosage form of an Isoquinoline Derivative. In one embodiment the unit dosage form is a container, which can be sterile, containing an effective amount of an Isoquinoline Derivative and a physiologically acceptable carrier or vehicle. The kit can further comprise a label or printed instructions instructing the use of the Isoquinoline Derivative to treat or prevent a Condition. The kit can also further comprise a unit dosage form of another prophylactic or therapeutic agent, for example, a container containing an effective amount of the other prophylactic or therapeutic agent. In one embodiment the kit comprises a container containing an effective amount of an Isoquinoline Derivative and an effective amount of another prophylactic or therapeutic agent. Examples of other therapeutic agents include, but are not limited to, those listed above.

Kits of the invention can further comprise a device that is useful for administering the unit dosage forms. Examples of such a device include, but are not limited to, a syringe, a drip bag, a patch, an inhaler, and an enema bag.

### 5.5 Methods For Making The Isoquinoline Derivatives

Examples of synthetic pathways useful for making Isoquinoline Derivatives are set forth in the Examples below and generalized in **Schemes 1-10.**

Methods useful for making Isoquinoline Derivatives of formula **(I)** wherein X is -CH₂- and R₅ is O are illustrated below in Scheme 1. 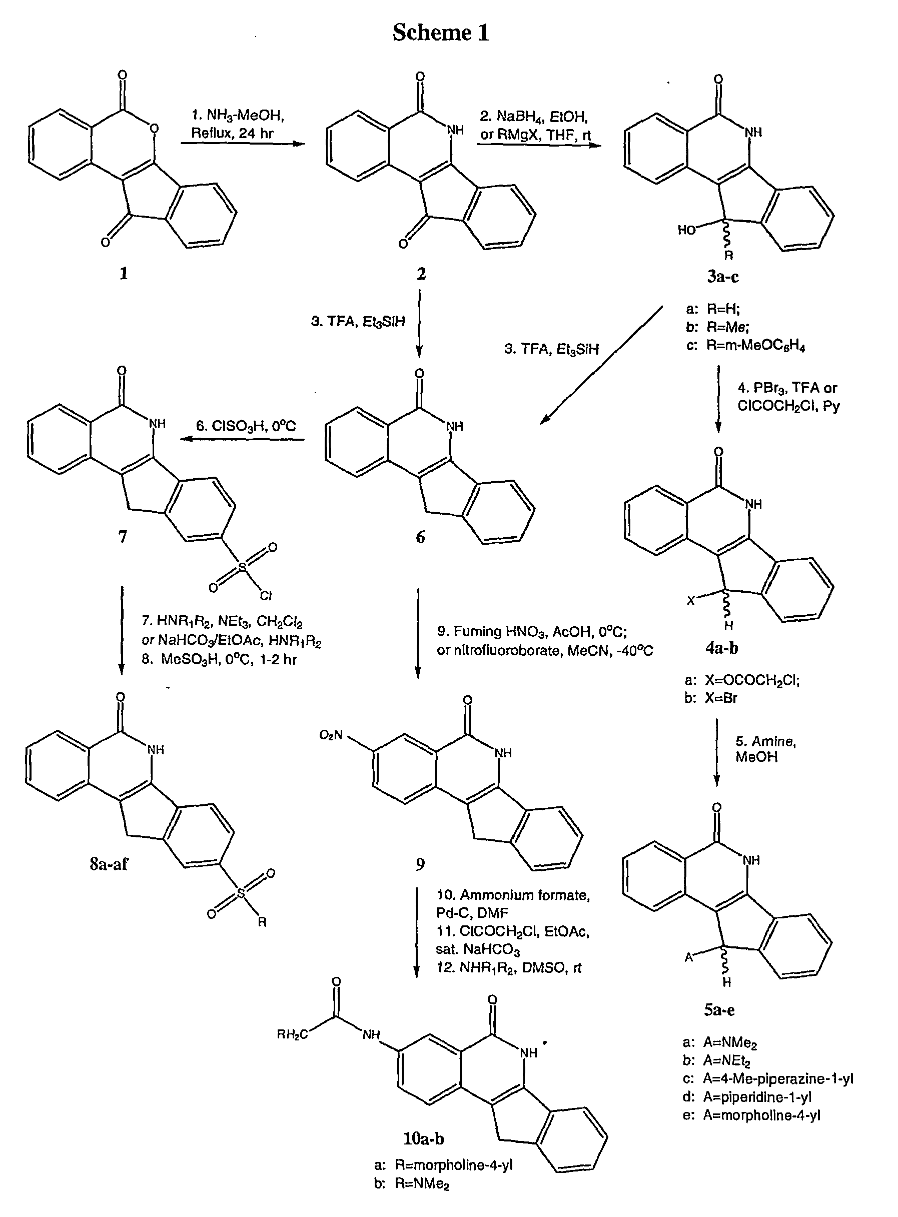 wherein compounds **8a-8af** are as follows:

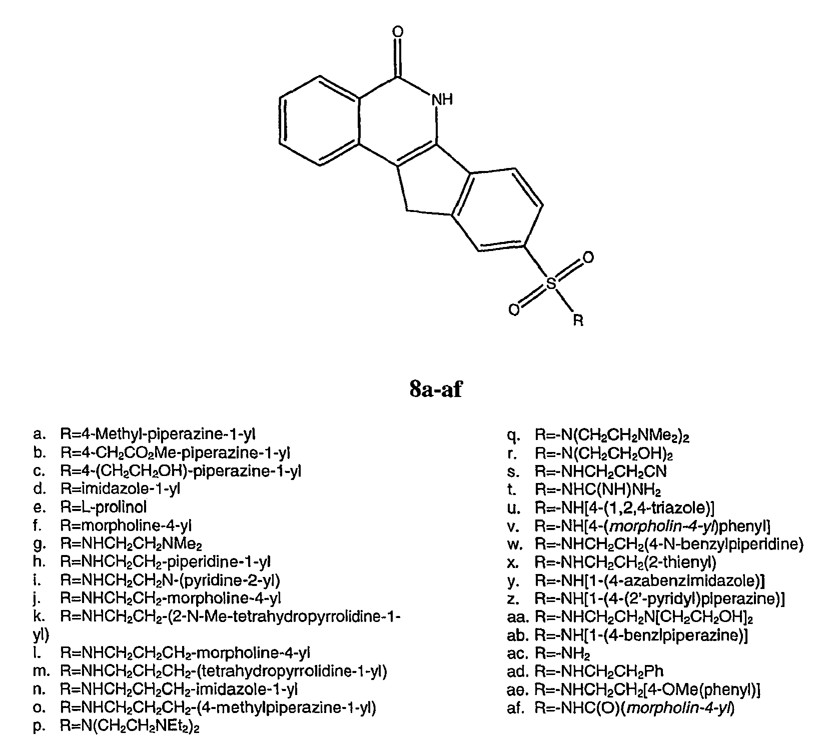

5,6-dihydro-5,11-diketo-11*H*-isoquinoline **(2)** was prepared by reacting compound **1** (Aldrich Chemical, Milwaukee, WI) with ammonia in methanol.

(±) 11-hydroxy-5,6-dihydro-5-oxo-11*H*-indeno[1,2-c]isoquinoline **(3a)** was prepared by reacting **2** with NaBH₄ in ethanol.

(±) 11-hydroxy-11-methyl-5,6-dihydro-5-oxo-11*H*-isoquinoline **(3b)** was prepared by reacting **2** with MeMgI.

(±) 11-hydroxy-11-(m-methoxyphenyl)-5,6-dihydro-5-oxo-11H-indeno[1,2-c]isoquinoline (3c) was prepared from **2** using *m*-MeO-C₆H₄MgI.

(±) 11-*N*,*N*-dimethylamino-5,6-dihydro-5-oxo-11H-indeno[1,2-c]isoquinoline **(5a)** was prepared from **3a** using chloroacetylchloride followed by reacting with dimethylamine. Similarly prepared are: (±) 11-N,N-diethylamino-5,6-dihydro-5-oxo-11H-indeno[1,2-c]isoquinoline **(5b),** (±) 11-*N*-(piperidino-1-yl)-5,6-dihydro-5-oxo-11H-indeno[1,2-c]isoquinoline **(5d),** (±) 11-*N*-(4-methylpiperazino-1-yl)-5,6-dihydro-5-oxol IH-indeno[1,2-c]isoquinoline **(5c),** (±) 11-*N*-(morpholino-4-yl)-5,6-dihydro-5-oxo11H-isoquinolin **(5e).** (+) 11-*N*-(morpholino-4-yl)-5,6-dihydro-5-oxo-11H-indeno[1,2-c]isoquinoline **(5e)** was also prepared from (±) 11-bromo-5,6-dihydro-5-oxo-11H-indeno[1,2-c]isoquinoline **(4b).**

5,6-Dihydro-5-oxo-11*H*-indeno-[1,2-c]isoquinoline **(6)** is prepared by reduction of 5,6-dihydro-5,11-diketo-11H-isoquinoline **(2)** or (±) 11-hydroxy-5,6-dihydro5-oxo-11 H-isoquinoline **(3a)** using CF₃COOH/triethylsilane. 9-Chlorosulphonyl-5,6-dihydro-5-oxo-1 1H-indeno-[1,2-c]isoquinoline **(7)** was prepared by chlorosulfonation of 5,6-dihydro-5-oxo-11H-indeno-[1,2-c]isoquinoline **(6).** 9-[N-(4-methylpiperazine-lyl)sulphonyl]-5,6-dihydro-5-oxo-11H-indeno-[1,2-c]isoquinoline **(8a)** was prepared from 9-chlorosulphonyl-5,6-dihydro-5-oxo-11H-indeno-[1,2-c]isoquinoline (7), and N-methylpiperazine. Similarly prepared are: 9-[*N*-(4-carbomethoxymethylenepiperazino-lyl)sulphonyl]-5,6-dihydro-5-oxo-11H-indeno-[1,2-c]isoquinoline **(8b),** 9-[*N*-4-(2-hydroxyethylpiperazino-1-yl)-sulphonyl]-5,6-dihydro-5-oxo-11H-indeno-[1,2-c]isoquinoline **(8c),** 9-[*N*-(imidazolo-1-yl)sulphonyl]-5,6-dihydro-5-oxo-11H-isoquinoline **(8d),** 9-[*N*-(2-hydroxyprolinyl)sulphonyl]-5,6-dihydro-5-oxo-11H-indeno[1,2-c]isoquinoline **(8e),** 9-[*N*-morpholinesulphonyl]-5,6-dihydro-5-oxo-11H-indeno[1,2-c]isoquinoline **(8f),** 9-[*N*-(2-[*N*,*N*-dimethylamino]ethyl)aminosulphonyl]-5,6-dihydro-5-oxo-11H-indeno[1,2-c]isoquinoline **(8g),** 9-[*N*-(2-[piperidino-1-yl]ethyl)-aminosulphonyl]-5,6-dihydro-5-oxo-11H-indeno[1,2-c]isoquinoline **(8h),** 9-[*N* (2-(pyridino-2-yl)-ethyl)-aminosulphonyl]-5,6-dihydro-5-oxo-11H-indeno[1,2c]isoquinoline **(8i),** 9-[*N*-(2 -[morpholino-4-yl]ethyl) -aminosulphonyl]-5,6-dihydro-5-oxo-11H-indeno[1,2-c]isoquinoline **(8j),** 9-[*N*-(2-[*N*-methyltetrahydropyrroiidino-1-yl]ethyl) aminosulphonyl]-5,6-dihydro-5-oxo-11H-indeno-[1,2-c]isoquinoline (**8k**), 9-[*N*-(3-[morpholino-4-yl] propyl)-aminosulphonyl]-5,6-dihydro-5-oxo-11H-indeno-[1,2c]isoquinoline **(81),** 9-[*N*-(3-[tetrahydropyrrolodino-1-yl]propyl)aminosulphonyl]-5,6-dihydro-5-oxo-11H-indeno-[1,2-c]isoquinoline **(8m),** 9-[*N*-(3-[imidazolo-1-yl]propyl)aminosulphonyl]-5,6-dihydro-5-oxo-11H-indeno-[1,2-c]isoquinoline **(8n),** 9-[*N*-[3-(4-methylpiperazino-1-yl]propyl)-aminosulphonyl]-5,6-dihydro-5-oxo-11H-indeno-[1,2c]isoquinoline (**8o**), 9-[*N*,*N*-di-(2-[*N,N*-diethylamino]ethyl)-aminosulphonyl]-5,6-dihydro-5-oxo-11H-indeno-[1,2-c]isoquinoline **(8p),** 9-[*N,N* di-(2-[*N,N* dimethylamino]ethyl)aminosulphonyl]-5,6-dihydro-5-oxo-11H-indeno-[1,2-c]isoquinoline **(8q),** and 9-[*N*,*N*-di(2-[*N,N*-dihydroxyethylamino] ethyl)-aminosulphonyl]-5,6-dihydro-5-oxo-11H-indeno-[1,2c]isoquinoline **(8r).**

Compounds **8s-8af** can be prepared using the methods described above for making compounds of **8a-8r,** using appropriate amine intermediates.

Scheme 2 illustrates a method useful for making terminal carboxylic acid compounds of formulas **8ag-8ao.** This method comprises reacting sulfonyl chloride 7 with the alkyl ester of an amino acid in the presence of a base, **such as** triethyamine, to provide an intermediate terminal carboxylic acid alkyl ester, which is then hydrolyzed using a base such as sodium hydroxide to provide the corresponding terminal carboxylic acid. 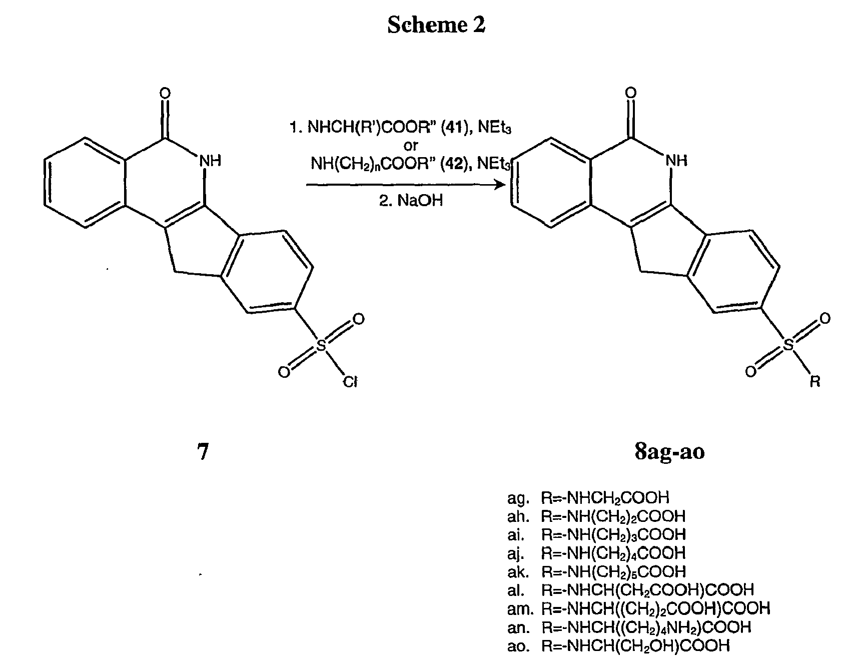 wherein:
R' is -amino-substituted C₁-C₅ alkyl or -hydroxy-substituted C₁-C₅ alkyl
R" is -C₁-C₆ alkyl; and
n is an integer ranging from 1 to 6.

### General Procedure for making 9-sulfonamide carboxylic acid derivatives

### Preparation of 9-sulfonamido carboxylic acid ester

To a 0.5 M solution of an ester of formula **41** or **42** in CH₂Cl₂ is added compound 7 (1.0 eq) and the resulting mixture is stirred for 5 minutes. Triethylamine (about 5 eq) is then added and the resulting reaction is stirred at room temperature and monitored using TLC or HPLC until complete. The reaction mixture is filtered, the solid is washed using MeOH to provide the intermediate 9-sulfonamido carboxylic acid ester which can be used without further purification.

### Ester Hydrolysis

To an approximately 0.5 M solution of a 9-sulfonamide carboxylic acid ester in ethanol is added about 3.0 N aqueous sodium hydroxide (about 5.0 eq) and the resulting reaction is refluxed if necessary and monitored using TLC or HPLC until completion. The reaction mixture is neutralized to about pH 7.0 using about 1.0 N HCl and the neutralized reaction mixture is extracted twice using EtOAc. The combined EtOAc layers are washed sequentially with water and saturated aqueous sodium chloride, then dried over sodium sulfate and concentrated *in vacuo* to afford a crude residue which is purified using flash column chromatography to provide the desired 9-sulfonamide carboxylic acid compound.

Acid hydrolysis with neat TFA can be useful where the sulfonamide has a t-butyl ester group.

In another embodiment, illustrated below in Scheme 3, Isoquinoline Derivatives of general formula **13** can be made by a method comprising contacting a compound of formula **11** and a compound of formula **12** in the presence of a base for a time and at a temperature sufficient to make a compound of formula **13.** 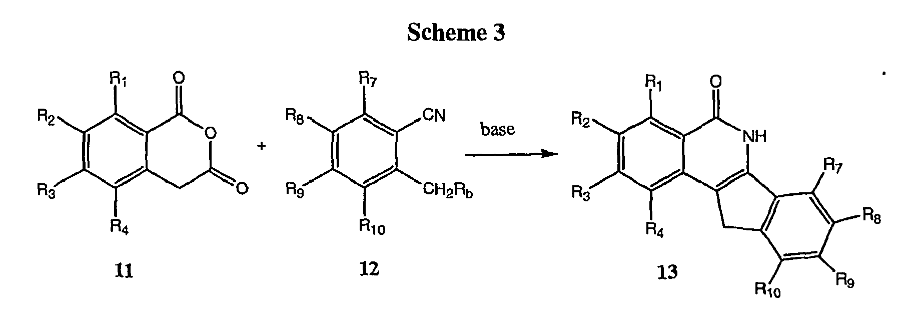 wherein:
R₁-R₄ and R₇-R₁₀ are as defined above for formula (I); and
R_{b} is -Cl, -Br, -I, -OMs, -OTs or -OTf.

In one embodiment, R_{b} is -Br.

In another embodiment, R_{b} and R_{d} are both -Br.

In one embodiment, about 0.1 to about 10 equivalents of a compound of Formula **12** are used per about 1 equivalent of a compound of Formula **11.**

In another embodiment, about 0.5 to about 5 equivalents of a compound of Formula **12** are used per about 1 equivalent of a compound of Formula **11.**

In still another embodiment, about 1 to about 2 equivalents of a compound of Formula **12** are used per about 1 equivalent of a compound of Formula **11.**

In one embodiment, about 1 to about 10 equivalents of base are used per about **1** equivalent of a compound of Formula **11.**

In another embodiment, about 3 to about 7 equivalents of base are used per about 1 equivalent of a compound of Formula **11.**

In a yet another embodiment, about 5 to about 6 equivalents of base are used per about 1 equivalent of a compound of Formula **11.**

Suitable bases for use in the method of Scheme 3 are organic bases such as triethylamine, lithium N-diisopropylamide, diisopropylethylamine, pyridine, lutidine and imidazole; and inorganic bases such as alkali metal carbonates, including sodium carbonate, potassium carbonate and cesium carbonate.

In one embodiment, the base is triethylamine.

In another embodiment, the base is potassium carbonate.

The method of Scheme 3 can be carried out in the presence of a solvent, such as acetonitrile, methylene chloride, chloroform, THF, DMF, DMSO, ethyl acetate, acetone, benzene, diethyl ether, water or mixtures thereof.

In one embodiment, the solvent is acetonitrile.

In another embodiment, the solvent is DMF.

In still another embodiment, where the solvent is not water, the solvent is substantially anhydrous, i.e., comprises less than about 1 % water.

In one embodiment, the method of Scheme 3 is carried out for a time of about 0.5 hours to about 48 hours.

In another embodiment, the method of Scheme 3 is carried out for a time of about 3 hours to about 36 hours.

In still another embodiment, the method of Scheme 3 is carried out for a time of about 8 hours to about 24 hours.

In yet another embodiment, the method of Scheme 3 is carried out for a time of about 15 hours to about 20 hours.

In a further embodiment, the method of Scheme 3 is carried out at a temperature of about 0°C to about 200°C.

In another embodiment, the method of Scheme 3 is carried out at a temperature of about 25°C to about 150°C.

In yet another embodiment, the method of Scheme 3 is carried out at a temperature of about 50°C to about 100°C.

### General Procedure For The Preparation of Compounds of Formula 13

To a solution of a homophthalic anhydride of formula **11** (about 1 to about 2 equivalents) in a suitable solvent, such as acetonitrile, is added a compound of Formula **12** (about 1 to about 2 eq) followed by a suitable base, such as triethylamine (about 1 to about 5 eq). The resulting reaction is reaction is allowed to stir for about 1 hour, at which time a colored precipitate appears. The reaction is then heated at reflux for about 20 hours, cooled to room temperature and filtered. The collected solid is washed using acetonitrile and dried under vacuum to provide a compound of Formula **13.**

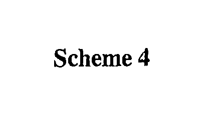

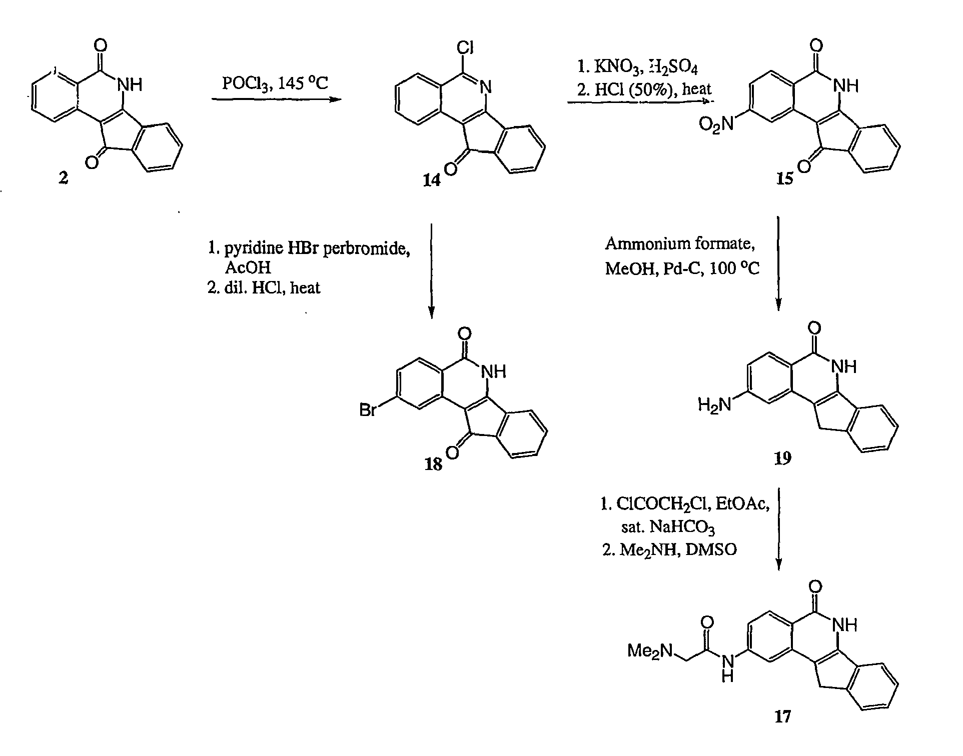

The amide derivative 2-dimethylamino-*N*-(5-oxo-5,11-dihydro-6*H*-indeno[1,2c]isoquinoiin-2-yl)-acetamide **(17)** was prepared from 5-chloro-11H-indeno [1,2c]isoquinoline **(14).** Compound **14** was subjected to nitration to provide nitro compound **15,** which was reduced using ammonium formate to provide amine 16, which was derivatized to acetamide **17,** and followed by amination of the chloroacetamide intermediate. 2-bromo5,6-dihydro-5-oxo-11H-indeno[1,2-c]isoquinoline **(18)** was prepared by bromination of Compound **14.**

Scheme 5 illustrates methods useful for making oxygen-substituted Isoquinoline Derivatives of formula **(I),** where R₅ and X are oxygen. 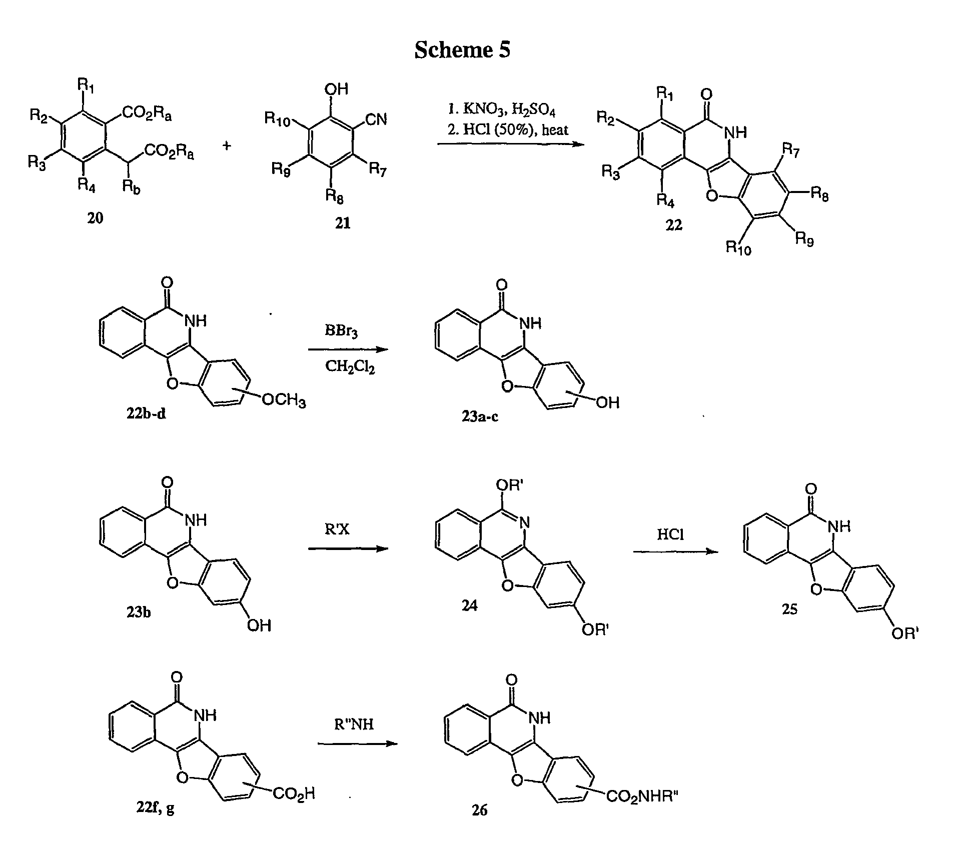 wherein:
R₁-R₅ are as defined above for formula (I);
each occurrence of Rₐ is independently C₁-C₅ alkyl;
R_{b} is -Cl, -Br, -I, -OMs, -OTs or -OTf;
R^{'} is -C₁-C₁₀ alkyl, alkanol or alkylcarboxy; and
R^{"} is -C₁-C₁₀ alkyl, aryl, heterocycle, alkanol or alkylcarboxy.

In one embodiment, Rₐ is methyl.

In another embodiment, R_{b} is -Br

In another embodiment, illustrated above in Scheme 5, Isoquinoline

Compounds of formula **22** can be made by a method comprising contacting a compound of formula **20** and a compound of formula **21** in the presence of a base for a time and at a temperature sufficient to make a compound of formula **22.**

In one embodiment, about 0.1 to about 10 equivalents of a compound of Formula **20** are used per about 1 equivalent of a compound of Formula **21.**

In another embodiment, about 0.5 to about 5 equivalents of a compound of Formula **20** are used per about 1 equivalent of a compound of Formula **21.**

In still another embodiment, about 1 to about 2 equivalents of a compound of Formula **20** are used per about 1 equivalent of a compound of Formula **21.**

In one embodiment, about 1 to about 10 equivalents of base are used per about 1 equivalent of a compound of Formula **21.**

In another embodiment, about 3 to about 7 equivalents of base are used per about 1 equivalent of a compound of Formula **21.**

In a yet another embodiment, about 5 to about 6 equivalents of base are used per about 1 equivalent of a compound of Formula **21.**

Suitable bases for use in the method are organic bases such as triethylamine, diisopropylamine, diisopropylethylamine, pyridine, lutidine and imidazole; and inorganic bases such as alkali metal carbonates such as sodium carbonate, potassium carbonate and cesium carbonate.

In one embodiment, the base is potassium carbonate.

In another embodiment, the base is triethylamine.

The method can be carried out in the presence of a solvent, such as acetonitrile, methylene chloride, chloroform, THF, DMF, DMSO, ethyl acetate, acetone, benzene, diethyl ether, water or mixtures thereof.

In one embodiment, the solvent is DMF.

In another embodiment, the solvent is acetonitrile.

In still another embodiment, the solvent is substantially anhydrous, i.e., comprises less than about 1 % water.

In one embodiment, the method is carried out for a time of about 1 hour to about 96 hours.

In another embodiment, the method is carried out for a time of about 18 hours to about 72 hours.

In yet another embodiment, the method is carried out for a time of about 24 hours to about 48 hours.

In one embodiment, the method is carried out at a temperature of about 25°C to about 200°C.

In another embodiment, the method is carried out at a temperature of about 50°C to about 150°C.

In still another embodiment, the method is carried out at a temperature of about 75°C to about 125°C.

Scheme 6 illustrates methods useful for making nitrogen-substituted Isoquinoline Derivatives of the invention.

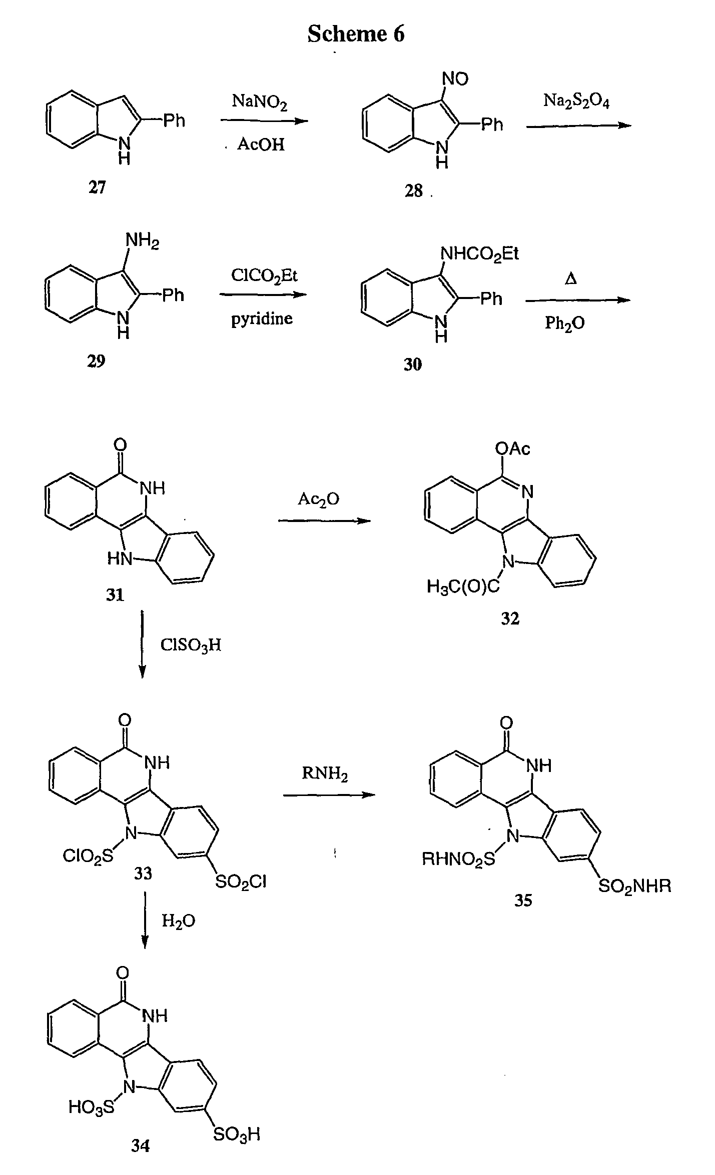

In an alternate embodiment, illustrated below in Scheme 7, nitrogen-substituted Isoquinoline Derivatives of general formula **37** can be made by a method comprising contacting a compound of formula **36** and a compound of formula **11** or formula **20** in the presence of a base for a time and at a temperature sufficient to make a compound of formula **37.** 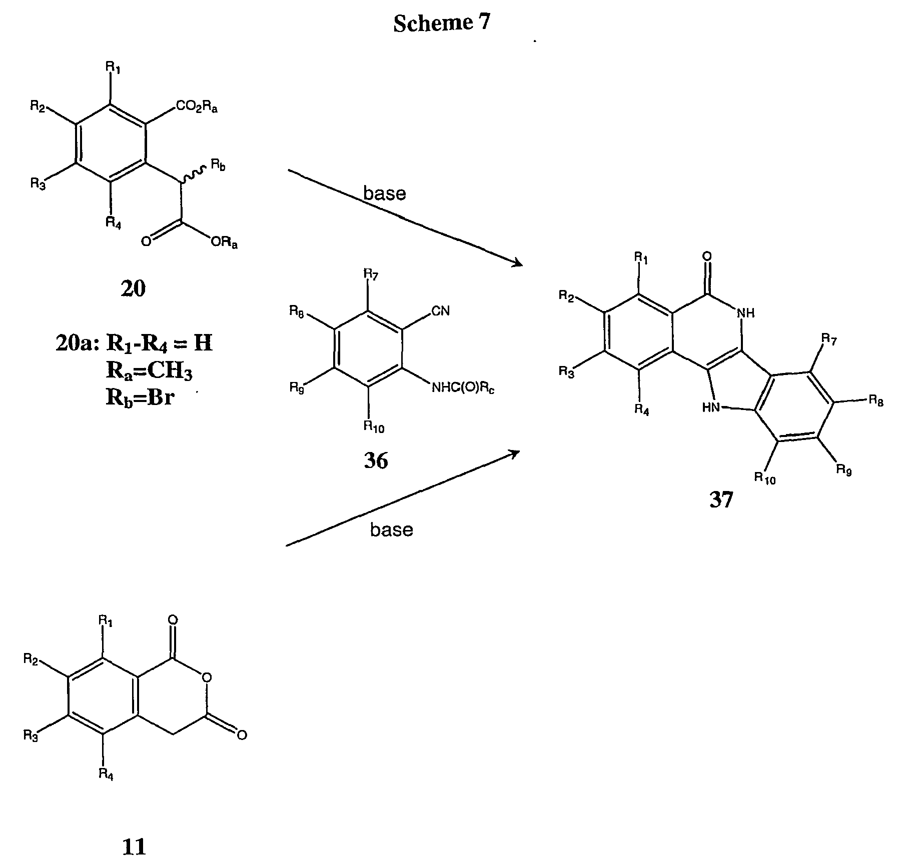 wherein:
R₁-R₄ and R₇-R₁₀ are as defined above for formula **(I);**
each occurrence of Rₐ is independently C₁-C₅ alkyl;
R_{b} is -Cl, -Br, -I, -OMs, -OTs or -OTf, and
R_{c}, is C₁-C₅ alkyl.

In one embodiment, Rₐ is methyl.

In another embodiment, R_{b} is -Br.

In a further embodiment, Rₐ is methyl and R_{b} is -Br.

In still another embodiment, R_{c} is methyl.

In one embodiment, about 0.1 to about 10 equivalents of a compound of Formula **11** are used per about 1 equivalent of a compound of Formula **36.**

In another embodiment, about 0.5 to about 5 equivalents of a compound of Formula **11** are used per about 1 equivalent of a compound of Formula **36.**

In still another embodiment, about I to about 2 equivalents of a compound of Formula **11** are used per about 1 equivalent of a compound of Formula **36.**

In one embodiment, about 0.1 to about 10 equivalents of a compound of Formula **20** are used per about 1 equivalent of a compound of Formula **36.**

In another embodiment, about 0.5 to about 5 equivalents of a compound of Formula **20** are used per about 1 equivalent of a compound of Formula **36.**

In still another embodiment, about 1 to about 2 equivalents of a compound of Formula **20** are used per about 1 equivalent of a compound of Formula **36.**

In one embodiment, about 1 to about 10 equivalents of base are used per about 1 equivalent of a compound of Formula **36.**

In another embodiment, about 3 to about 7 equivalents of base are used per about 1 equivalent of a compound of Formula **11.**

In a yet another embodiment, about 5 to about 6 equivalents of base are used per about 1 equivalent of a compound of Formula **11.**

Suitable bases for use in the method of Scheme 7 are organic bases such as triethylamine, diisopropylamine, diisopropylethylamine, pyridine, lutidine and imidazole; and inorganic bases such as alkali metal carbonates such as sodium carbonate, potassium carbonate and cesium carbonate.

In one embodiment, the base is potassium carbonate.

In another embodiment, the base is triethylamine.

The method of Scheme 7 can be carried out in the presence of a solvent, such as acetonitrile, methylene chloride, chloroform, THF, DMF, DMSO, ethyl acetate, acetone, benzene, diethyl ether, water or mixtures thereof.

In one embodiment, the solvent is DMF.

In another embodiment, the solvent is acetonitrile.

In still another embodiment, the solvent is substantially anhydrous, i.e., comprises less than about 1 % water.

In one embodiment, the method of Scheme 7 is carried out for a time of about 1 hour to about 96 hours.

In another embodiment, the method of Scheme 7 is carried out for a time of about 18 hours to about 72 hours.

In yet another embodiment, the method of Scheme 7 is carried out for a time of about 24 hours to about 48 hours.

In one embodiment, the method of Scheme 7 is carried out at a temperature of about 25°C to about 200°C.

In another embodiment, the method of Scheme 7 is carried out at a temperature of about 50°C to about 150°C.

In still another embodiment, the method of Scheme 7 is carried out at a temperature of about 75°C to about 125°C.

### General Procedure For The Preparation of Compounds of Formula 37

### From a homophthalate:

To a solution of a homophthalate of Formula **20** (about 1 eq) and an *N-*acylanthranilonitrile of Formula **36** (about 1 to about 2 eq) in a solvent such as DMF, under inert atmosphere, is added a base (about 5 eq), such as potassium carbonate and the reaction is allowed to stir for about 48 hours at about 100°C, then cooled to room temperature. The reaction mixture is then poured into about 1 N sodium hydroxide and the resulting solution is extracted with EtOAc. The EtOAc layer is washed sequentially with about 1 N HCl, saturated aqueous sodium chloride, dried over sodium sulfate, filtered and concentrated *in vacuo*. The resulting residue is dissolved using warming in toluene and the resulting solution is cooled to room temperature and precipitated using hexanes. The solid precipitate is filtered, washed using hexanes and dried in a vacuum oven at 50°C for 72 h to provide a Compound of Formula **36.**

The synthesis of phenyl amide **36,** which is a useful intermediate in Scheme 7, is described below in Scheme 8. In this procedure, the amine group of a cyanoaniline compound of formula **38** is acylated using an acyl chloride or an anhydride in the presence of an acid. 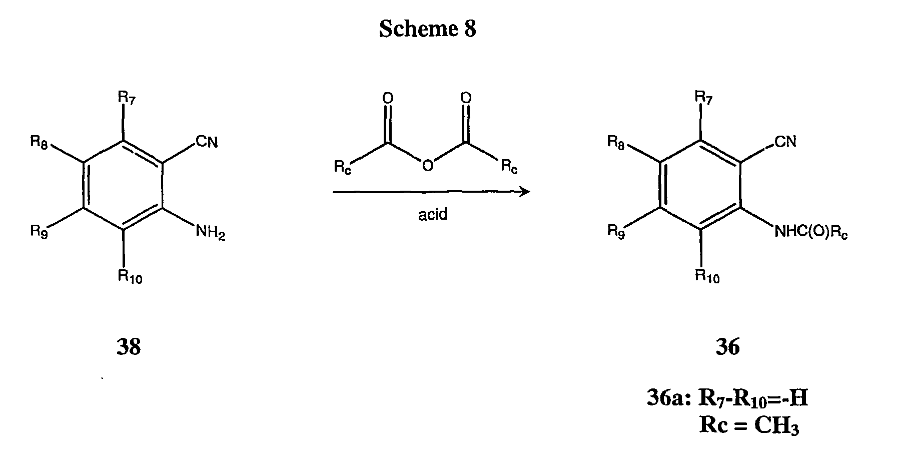 wherein:
R₇-R are as defined above for formula **(I);** and
R_{c} is C₁-C₅ alkyl.

Suitable acids for use in the method of Scheme 8 include, but are not limited to, sulfuric acid and phosphoric acid.

In one embodiment, the acid is sulfuric acid.

In another embodiment, R_{c} is methyl.

The method of Scheme 8 can be carried out in the presence of a solvent, including, but not limited to, acetonitrile, methylene chloride, chloroform, THF, DMF, DMSO, ethyl acetate, acetone, benzene, diethyl ether or mixtures thereof.

### General Procedure For Making a Compound of Formula 36

To a solution of a compound of Formula **38** (about 1 eq) in acetic anhydride (about 6 eq) at 90°C is added 1 drop of sulfuric acid (catalytic) and the resulting reaction is stirred at about 90°C for about 2 h, and is then allowed to sit at room temperature for about 12 h. The reaction mixture is poured onto ice and the resulting solution is stirred for about 2 h, after which time the solution is neutralized to about pH 7.0 using 1 N sodium hydroxide. The resulting precipitate is filtered, washed using water (about 4x) and dried under vacuum for about 72 h to provide a compound of Formula **36.**

In another embodiment, illustrated below in Scheme 9, sulfur substituted Isoquinoline Derivatives of formula **40** can be made by a method comprising contacting a compound of formula **39** and a compound of formula **11a** or formula **20** in the presence of a base for a time and at a temperature sufficient to make a compound of formula **40.** 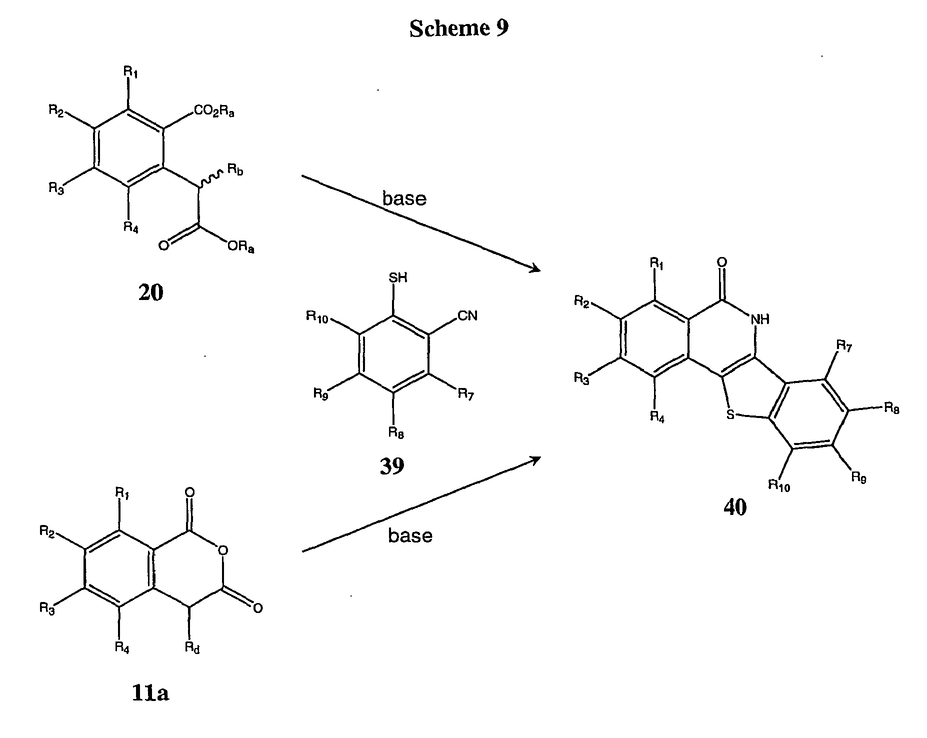
R₁-R₄ and R₇-R₁₀ are as defined above for formula **(I);**
each occurrence of Rₐ is independently C₁-C₅ alkyl;
R_{b} is -Cl, -Br, -I, -OMs, -OTs or -OTf; and
R_{d} is -H or -Br.

In one embodiment, Rₐ is methyl.

In another embodiment, R_{b} is -Br.

In still another embodiment, Rₐ is methyl and R_{b} is -Br.

In yet another embodiment, R_{d} is -H.

In a further embodiment, R_{d} is -Br.

In one embodiment, about 0.1 to about 10 equivalents of a compound of Formula **11a** are used per about 1 equivalent of a compound of Formula **39.**

In another embodiment, about 0.5 to about 5 equivalents of a compound of Formula **11a** are used per about 1 equivalent of a compound of Formula **39.**

In still another embodiment, about 1 to about 2 equivalents of a compound of Formula **11a** are used per about 1 equivalent of a compound of Formula **39.**

In one embodiment, about 0.1 to about 10 equivalents of a compound of Formula **11a** are used per about 1 equivalent of a compound of Formula **39.**

In another embodiment, about 0.5 to about 5 equivalents of a compound of Formula **11a** are used per about 1 equivalent of a compound of Formula **39.**

In yet another embodiment, about 1 to about 2 equivalents of a compound of Formula **11a** are used per about 1 equivalent of a compound of Formula **39.**

In one embodiment, about 0.1 to about 10 equivalents of a compound of Formula 20 are used per about 1 equivalent of a compound of Formula **39.**

In another embodiment, about 0.5 to about 5 equivalents of a compound of Formula **20** are used per about 1 equivalent of a compound of Formula **39.**

In yet another embodiment, about 1 to about 2 equivalents of a compound of Formula **20** are used per about 1 equivalent of a compound of Formula **39.**

In one embodiment, about 1 to about 10 equivalents of base are used per about 1 equivalent of a compound of Formula **39.**

In another embodiment, about 3 to about 7 equivalents of base are used per about 1 equivalent of a compound of Formula **39.**

In a yet another embodiment, about 5 to about 6 equivalents of base are used per about 1 equivalent of a compound of Formula **39.**

Suitable bases for use in the method of Scheme 9 are organic bases such as triethylamine, lithium N-diisopropylamide, diisopropylethylamine, pyridine, lutidine and imidazole; and inorganic bases such as alkali metal carbonates, including sodium carbonate, potassium carbonate and cesium carbonate.

In one embodiment, the base is potassium carbonate.

In another embodiment, the base is triethylamine.

The method of Scheme 9 can be carried out in the presence of a solvent, such as acetonitrile, methylene chloride, chloroform, THF, DMF, DMSO, ethyl acetate, acetone, benzene, diethyl ether, water or mixtures thereof.

In one embodiment, the solvent is DMF.

In another embodiment, the solvent is acetonitrile.

In one embodiment, the method of Scheme 9 is carried out for a time of about 1 hour to about 120 hours.

In another embodiment, the method of Scheme 9 is carried out for a time of about 24 hours to about 96 hours.

In yet another embodiment, the method of Scheme 9 is carried out for a time of about 60 hours to about 80 hours.

In one embodiment, the method of Scheme 9 is carried out at a temperature of about 0°C to about 200°C.

In another embodiment, the method of Scheme 9 is carried out at a temperature of about 25°C to about 150°C.

In still another embodiment, the method of Scheme 9 is carried out at a temperature of about 50°C to about 100°C.

### General Procedure for the Preparation Compounds of Formula 40

### From a homophthalic anhydride:

A solution of a mercaptobenzonitrile of Formula **39** (about 1.0 eq) and a homophthalic anhydride of Formula **11a** (about 2.0 eq) in a suitable solvent such as acetonitrile under inert atmosphere is warmed with stirring until all reactants are in solution. A suitable base such as triethylamine (about 1 to about 5 eq) is added and the reaction is allowed to stir at about 90°C for about 72 hours, then cooled to room temperature. The reaction mixture is filtered, and the collected solid is washed using methanol, then dried in a vacuum oven at about 50°C to provide a compound of Formula **40.**

### From a homophthalate:

A solution of a mercaptobenzonitrile of Formula **39** (about 1.0 eq) and a homophthalate of Formula **20** (about 2.0 eq) in a suitable solvent such as acetonitrile under inert atmosphere is warmed with stirring until all reactants are in solution. A suitable base such as triethylamine (about 1 to about 5 eq) is added and the reaction is allowed to stir at about 90°C for about 72 hours, then cooled to room temperature. The reaction mixture is filtered, and the collected solid is washed using methanol, then dried in a vacuum oven at about 50°C to provide a compound of Formula **40.**

Methods for making Isoquinoline Derivatives of Formula (IV) are illustrated below in Scheme 10. 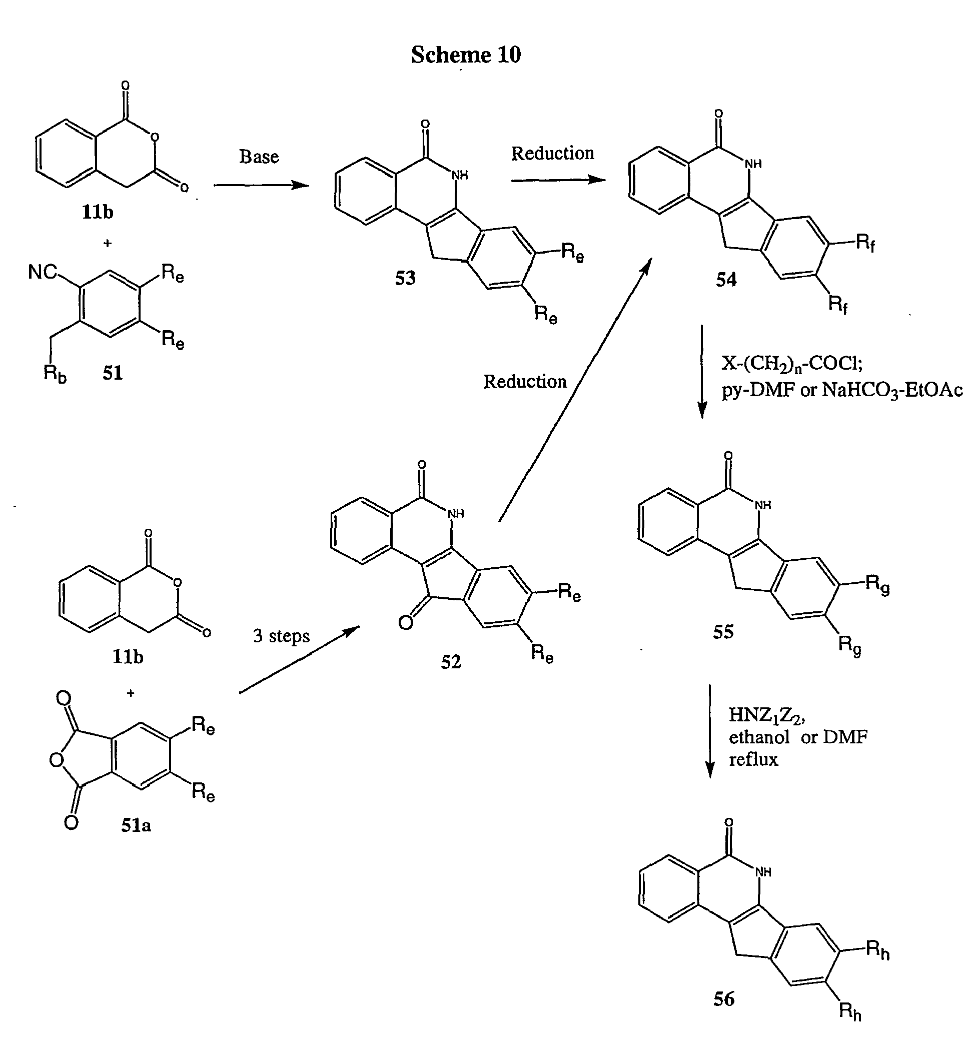 wherein: n, Z₁, and Z₂ as defined above for Formula (IV);
X is a leaving group such as bromide or chloride;
R_{b} is -Cl, -Br, -I, -OMs, -OTs, or -OTf;
one Rₑ is -H and the other Rₑ is -NO₂;
one R_{f} is -H and the other R_{f} is -NH₂;
one R_{g} is -H and the other Rg is -NHC(O)-(CH₂)ₙ-X; and
one Rₕ is -H and the other Rₕ is -NHC(O)-(CH₂)ₙ-NZ₁Z₂.

In one embodiment, R_{b} is -Br.

### General Procedure For The Preparation of Compounds of Formula 56

To a solution of homophthalic anhydride **(11b)** (about 1 equivalent) in a suitable solvent, such as acetonitrile, is added a compound of Formula **51** (about 1 to about 2 equivalents), follwed by a suitable base, such as triethylamine (about 1 to about 5 equivalents). The resultant reaction mixture is allowed to stir for about 1 hour, at which time a precipitate appears. The reaction mixture is then heated to reflux for about 20 hours, cooled to room temperature and filtered. The collected solid is washed with acetonitrile and dried under vacuum to provide a compound of Formula **53.**

Compound **52** can be prepared from homophthalic anhydride **(11b)** and benzoic anhydride in two steps. Homophthalic anhydride and benzoic anhydride are reacted in a suitable solvent such as pyridine in the presence of an acid such as HCl; subsequently reacted with acetic anhydride in pyridine and heated to reflux; and then refluxed in the presence of an amine such as NH₃ in MeOH; to provide the compound of Formula 52.

To a solution of the compound of Formula **52** or **53** in a suitable solvent, such as DMF, is added a reducing agent, such as ammonium formate in the presence of palladium on carbon. The reaction mixture is heated to a temperature of about 90 to 100°C, cooled to room temperature and filtered to provide a compound of the Formula **54.**

The compound of the Formula **54** can be reacted with X-(CH₂)ₙ-COCl, under conditions effective to form an amide of the Formula **55.**

The compound of Formula **55** can be reacted with an amine of formula HNZ₁Z₂, in the presence of a solvent such as ethanol or DMF and heating to reflux, to form the compound of Formula **56.**

The invention is further described in the following examples, which do not limit the scope of the invention described in the claims. The following examples illustrate the synthesis of illustrative Isoquinoline Derivatives and demonstrate their usefulness for treating or preventing a Condition.

### 6. EXAMPLES

### Example 1: Preparation of Illustrative Isoquinoline Derivatives

### a) General Methods

Proton NMR spectra were obtained using a Varian 300 MHz spectrophotometer and chemical shift values (δ) are reported in parts per million (ppm). TLC was performed using TLC plates precoated with silica gel 60 F-254, and preparative TLC was performed using precoated Whatman 60A TLC plates. All intermediates and products were characterized on the basis of ¹H NMR and/or MS data.

### b) Preparation of 5,6-dihydro-5,11-diketo-11H-indeno[1,2-c]isoquinoline (2):

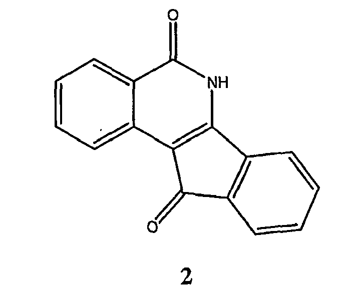

A stirred suspension of **1** (55 g, 0.22 mol) (Scheme 1) in NH₃/MeOH (7.0 N, 700 mL) was refluxed for 24 h. The reaction mixture was then allowed to cool to room temperature and was filtered and washed with MeOH to provide 46 g of the orange colored above-titled product in 84 % yield. ¹H NMR (DMSO-d₆): δ 7.48-7.61 (m, 4H), 7.80-7.88 (m, 1H), 7.86 (d, *J* = 8.7 Hz, 1H), 8.22 (d, *J* = 8.4 Hz, 1H), 8.44 (d, *J* = 7.5 Hz, 1H), 13.05 (s, 1H); ¹³C NMR (DMSO-D₆): δ 106.33, 121.63, 122.94, 123.27, 124.80, 128.45, 132.17, 133.60, 134.03, 134.68, 134.68, 134.81, 137.09, 156.41, 163.76,190.57; MS (ES⁻): m/z 246.2 (M-1); Anal. Calcd for C₁₆H₉NO₂: C, 77.72; H, 3.67; N, 5.67; Found: C, 77.54; H, 3.69, N, 5.69.

### c) Preparation of (±) 11-hydroxy-5,6-dihydro-5-oxo-11H-indeno[1,2-c]isoquinoline (3a):

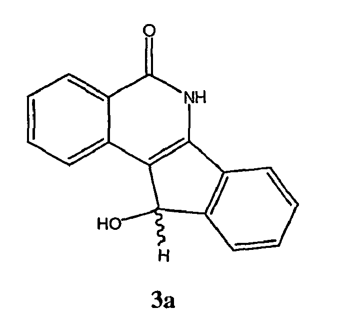

To a stirred suspension of 2 (2.5 g, 0.01 mol) in EtOH (25 mL) was added NaBH₄ (3.75 g, 0.1 mol) at room temperature in small portions over 30 min. The reaction mixture was stirred for an additional 2 h and then cooled to 0°C. It was then triturated with 10 % HCl (10 % soln.). The resulting solid precipitated was filtered and washed with water and MeOH to provide **3a** (2.326 g, 92 %). ¹H NMR (DMSO-d₆): δ 5.58 (d, *J* = 8.1 Hz, 1H), 5.78 (d, *J* =8.7 Hz, 1H), 7.33 -7.89 (m, 6H), 7.95 (d, *J* = 7.8 Hz, 1H, 8.22 (d, *J* = 7.8 Hz, 1H), 12.29 (s, 1H); ¹³C NMR (DMSO-d₆): S 77.44, 118.81, 120.15, 124.28, 125.04, 125.67, 126.34, 128.46, 128.64, 128.95, 133.27, 135.62, 136.12, 139.93, 148.55, 163.69.; MS (ES⁺): m/z 250.1 (M+1); Anal. Calcd for C₆H₁₁NO₂: C, 77.10; H, 4.45; N, 5.62. Found: C, 77.01; H, 4.57, N, 5.59.

Similarly, by reacting 2 with MeMgI and m-MeO-C₆H₄MgBr, respectively, compounds (±) 11-hydroxy-11-methyl-5,6-dihydro-5-oxo-11H-indeno[1,2-c]isoquinoline (3b) and (±) 11-hydroxy-11-(3-methoxyphenyl)-5,6-dihydro-5-oxo-11H-indeno[1,2c] isoquinoline (3c) were prepared.

### d) Preparation of 11-substituted 5,6-dihydro-5-oxo-11H-indeno[1,2-c]isoquinolines (5a-e):

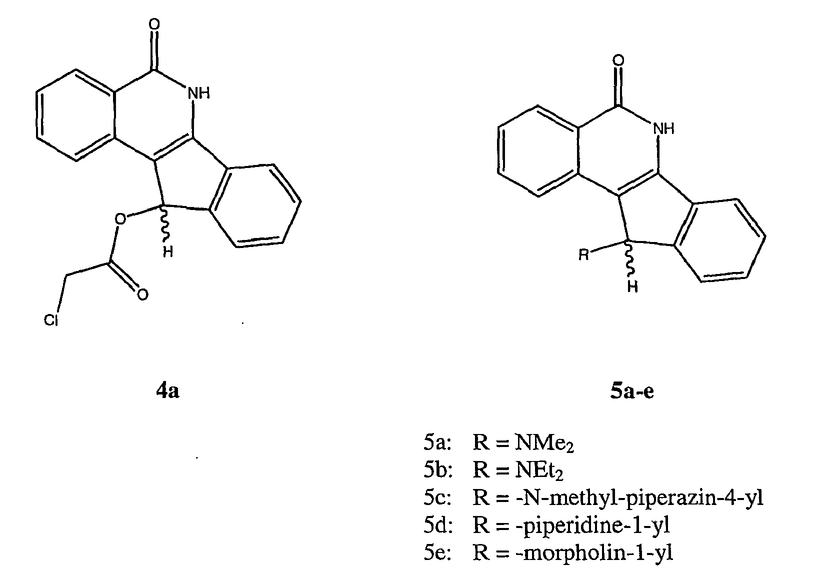

To a stirred suspension of **3a** (0.5 g, 2 mmol) in pyridine (10 mL) was added chloroacetyl chloride (0.81 g, 0.006 mol) at 0°C. The reaction mixture was allowed to warm to room temperature and allowed to stir for 24 h. The reaction mixture was then poured on ice and extracted with EtOAc. The organic layer was separated, dried and concentrated to provide crude compound **4a,** which was treated further with dimethylamine and stirred at room temperature for 24 h. The reaction mixture was poured on ice, and treated with 10 % HCl. The resultant mixture was then basified using saturated aqueous NaHCO₃ and the resulting solid was filtered to provide the desired product **5a.** ¹H NMR (DMSO-D₆): δ 2.31 (s, 6H), 5.00 (s, 1H), 7.28-7.45 (m, 3H), 7.68-7.73 (m, 2H), 7.95 (d,*J* = 6.9 Hz, 1H), 8.10 (d, *J* = 7.8 Hz, 1H), 8.21 (d, *J* = 8.1 Hz, 1H), 12.26 (s, 1H); ¹³C NMR (DMSO-D₆): δ 68.09, 116.28, 120.52, 124.58, 125,74, 126.27, 126.34, 127.68, 128.64, 133.02, 136.27, 144.45, 163.80; MS (ES⁺): m/z 277.2 (M+1).

The following compounds were also prepared by reacting **4a** as above with diethylamine, piperidine, N-methylpiperazine and morpholine, respectively: (±) 11 - diethylamino-5,6-dihydro-5-oxo-11*H*-indeno[1,2-c]isoquinoline **(5b),** (±) 11-(N-methylpiperazin)-5,6-dihydro-5-oxo-11*H*-indeno[1,2-c]isoquinoline **(5c),** (±) 11-(piperidin)-5,6-dihydro-5-oxo-11*H*-indeno[1,2-c]isoquinoline **(5d),** and (±) 11-morpholino-5,6-dihydro-5-oxo-11*H*-mdeno[1,2-c]isoquinoline **(5e).**

### e) Preparation of (±) 11-morphotino-5,6-dihydro-5-oxo-11H-indeno[1,2-c]isoquinoline (5e):

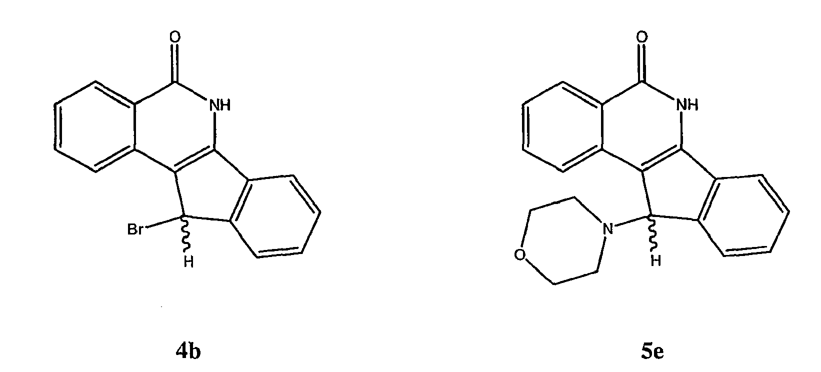

To a stirred suspension of **3a** (0.6 g, 2.4 mmol) in trifluoroacetic acid (5 mL) was added phosphorus tribromide (1.0 M soln. in CH₂Cl₂, 3 mL) at room temperature, and the reaction mixture was stirred for 8 h. The reaction mixture was poured on ice and the resulting solid was filtered to provide bromo compound **4b** (0.61 g, 76 %). ¹H NMR (DMSO-d₆): δ 7.35-7.50 (m, 3H), 7.61 (d, *J* = 6.6 Hz, 1H), 7.73 -7.82 (m, 2H), 7.94 (d, *J* = 6.6 Hz, 1H), 8.23 (d, *J* = 7.8 Hz, 1H, 12.41 (s, 1H); ¹³NMR (DMSO-d₆): δ 52.06, 79.35, 114.43, 120.56, 123.58, 125.27, 125.50, 126.68, 128.55, 128.86, 129.66, 133.73, 135.91, 136.61, 141.39, 143.95, 163.74.

Compound **4b** (0.5 g) was suspended in MeOH (10 mL) and treated with excess morpholine (about 5.0 eq) at room temperature and stirred at 60 °C for 3 h. The reaction mixture was poured on ice, and diluted with ethyl acetate (40 mL). The organic layer was separated and extracted in dil. HCl (10 % soln.), the aqueous layer was then basified with saturated aqueous NaHCO₃ and the resulting solid precipitated was filtered and dried to provide compound **5e** (0.46 g, 90 %). ¹H NMR (DMSO-d₆): δ 2.56 (m, 4H), 3.49 (m, 4H), 5.04 (s, 1H), 7.31-7.45 (m, 3H), 7.65 -7.76 (m, 2H), 7.96 (d, *J* = 7.2 Hz, 1H), 8.20-8.24 (m, 2H), 12.29 (s, H); ¹³C NMR (DMSO-D₆): δ 49.36, 67.62, 68.11, 115.20, 120.60, 124.47, 125.84, 126.34, 126.41, 127.76, 128.30, 128.72, 133.09, 136.30, 13δ.96,140.35, 144.44, 163.67.

### f) Preparation of 5,6-dihydro-5-oxo-11H-indeno[1,2-c]isoquinoline (6):

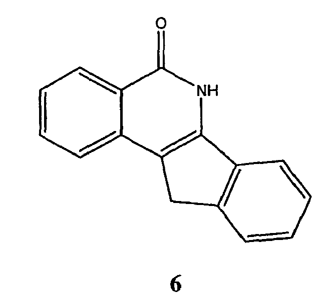

Method I: To a stirred solution of the alcohol **3a** (0.35 g, 1.4 mmol) in trifluoroacetic acid (10 mL) was added at room temperature triethylsilane (0.812 g, 7 mmol) and the reaction mixture was stirred for 24 h. Trifluoroacetic acid was evaporated *in vacuo* and EtOAc was added to the resulting crude product. The resulting solid was filtered and washed with H₂O and EtOAc to provide the above-titled compound **6** (0.285 g, 87 %). ¹H NMR (DMSO-D₆): δ 3.89 (s, 2H), 7.30 -7.47 (m, 3H), 7.59 (d, *J* = 6.9 Hz, 1H), 7.72 -7.74 (m, 2H), 7.98 (d, *J* = 7.8 Hz, 1H), 8.23 (d, *J* = 8.4 Hz, 1H), 12.31 (s, 1H); ¹³C NMR(DMSO-d₆): δ 33.51, 116.50, 120.19, 124.01, 125.51, 125.55, 126.42, 127.50, 127.68, 128.56, 133.45, 136.39, 137.53, 140.18, 143.80, 163.46; MS (ES): m/z 232.1 (M-1); Anal. Calcd forC₁₆H₁₁NO: C, 82.38; H, 4.75; N, 6.00. Found: C, 81.79; H, 4.45, N, 5.99.

Method II: To a stirred suspension of **2** (40 g, 0.16 mol) in trifluoroacetic acid (2.5 L) was added triethylsilane (94 g, 0.8 mol) in small portions at room temperature and the reaction mixture was stirred for 96 h, during which time the reaction progress was monitored using TLC (eluent - 5 % MeOH/CH₂Cl₂). The reaction mixture was slowly poured on ice, filtered, washed with copious amounts of H₂O and MeOH and dried *in vacuo* to provide the above-titled compound **6** (33.1 g, 88 %), whose spectral data were identical to those of a sample of compound **6** that was obtained using Method I.

### g) Preparation of 9-chlorosulfonyl-5,6-dihydro-5-oxo-11H-indeno[1,2-c]isoquinoline (7):

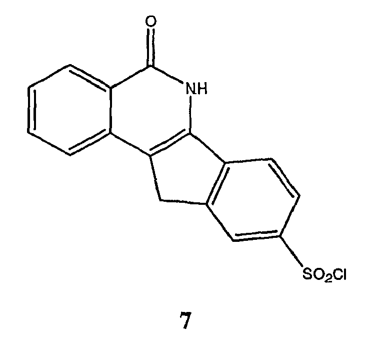

Compound **6** (40 g, 0.17 mol) was added in small portions to chlorosulfonic acid (112 mL, 1.71 mol) at 0°C and the reaction mixture was allowed to warm to room temperature and allowed to stir for 2 h. The reaction mixture was slowly poured on ice and the resulting yellow solid was filtered, washed thoroughly with water and EtOAc and dried *in vacuo* to provide the above-titled product **7** (52 g, 92 %). ¹H NMR (DMSO-d₆): δ 3.91 (s, 2H), 7.43 -7.48 (m, 1H), 7.60 (d, *J* = 7.2 Hz, 1H), 7.74 -7.76 (m, 2H), 7.79 (s, 1H), 7.90 (d, *J* = 7.5 Hz, 1H), 8.23 (d, *J* = 7.8 Hz, 1H), Anal. Calcd. for C₁₆H₁₂ClNO₄S: C, 54.94; H, 3.46; N, 4.00. Found: C, 55.28; H, 3.43, N, 3.68, Karl-Fisher, 2.95.

### h) Preparation of 9-sulphonamido derivatives of 5,6-dihydro-5-oxo-11H-indeno[1,2-c]isoquinolines (8a-af):

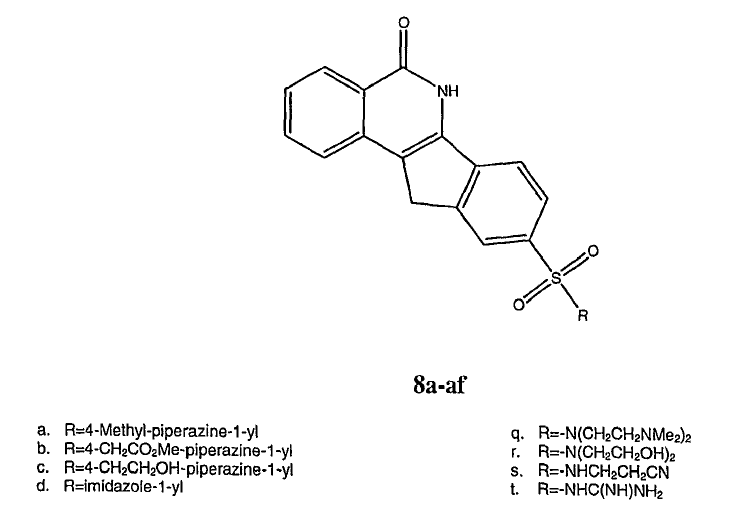

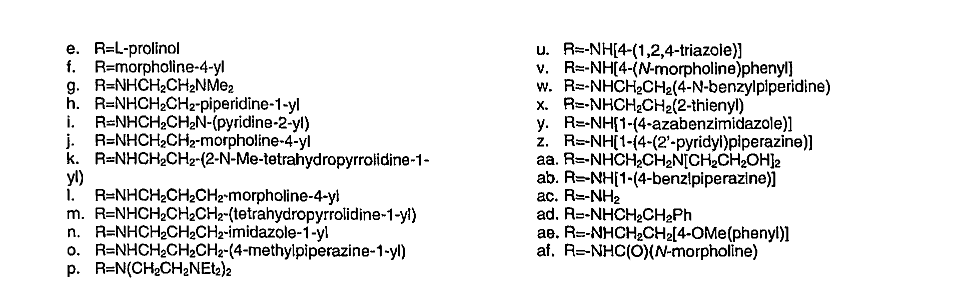

Method I: To a stirred suspension of 3-(4-morpholino)-1-propylamine (17.28 g, 0.12 mol) in EtOAc was added sat. aq. NaHCO₃ (300 mL), and the mixture was allowed to stir for 15 min. Compound **7** (4.0 g, 0.012 mol) was then introduced in small portions at room temperature. The reaction mixture was stirred for 24 h; filtered and washed with H₂O, EtOAc and MeOH; refluxed in MeOH for 30 min; filtered while still warm; and washed with MeOH to provide compound **81** as a free base (2.33 g, 44 %). ¹H NMR(DMSO-d₆): δ 1.47-1.52 (m, 2H), 2.16-2.21 (m, 4H), 2.47-2.48 (m, 2H), 3.44-3.48 (m, 2H), 3.23 (m, 4H), 4.02 (s, 2H), 7.49 -7.58 (m,1H), 7.78-7.82 (m, 3H), 7.97 (s, 1H), 8.14 (d, *J* = 7.8 Hz, 1H), 8.26 (d, *J* = 7.8 Hz, 1H), 9.59 (s, 1H), 12.42 (s, 1H).

The free bases of **8d, 8g, 8h, 8j, 8m-8r** were also prepared by Method I, but substituting 3-(4-morpholino)-1-propylamine with imidazole, 2-dimethylamino-ethylamine, 2-(*N*-piperidinyl)-ethylamine, 2-(*N*-morpholinyl)-ethylamine, 3-(N-tetrahydropyrrolidinyl)-propylamine, 3-(*N*-imidazolyl)-propylamine, 3(*N*-(4-methylpiperazinyl)-propylamine, di-(2-(diethylamino)-ethyl)amine, di-(2(dimethylamino)-ethyl)amine and di-(2-hydroxyethyl)amine, respectively.

Method II: To a stirred suspension of 3-(4-morpholino)-1-propylamine (4.250 g) in CH₂Cl₂ (100 mL) was added **7** (1.950 g, 5.89 mmol) and the resulting mixture was stirred for 5 minutes. Subsequently, triethylamine (3 mL) was added and the reaction mixture was stirred for 24 h at room temperature. After this time the precipitate was collected and washed with MeOH (2 x100 mL) and the crude solid product transferred to a round bottom flask. This material was diluted with MeOH (200 mL), heated to reflux for 30 min. and filtered while still warm. The resulting filtercake was washed with MeOH (200 mL) to provide the desired product as the free base of **81** (1.460 g, 56 %).

The free bases of compounds **8d, 8g, 8h, 8j, 8m, 8n, 8o, 8p, 8q, and 8r** were prepared using Method II, but substituting 3-(4-morpholino)-1-propylamine with about an equivalent amount of imidazole, 2-dimethylamino-ethylamine, 2-(*N*-piperidinyl)-ethylamine, 2-(*N*-morpholinyl)-ethylamine, 3-(*N*-tetrahydropyrrolidinyl)-propylamine, 3-(*N*-imidazolyl)propylamine, 3-(*N*-(4-methylpiperazinyl) propylamine, di-(2-(diethylamino)-ethyl)amine, di(2-(dimethylamino)-ethyl)amine and di-(2-hydroxyethyl)amine, respectively.

### k) Preparation of the mesylate salt of 81:

Free base **81** (1.0g) was added to methanesulfonic acid (10 mL) at 0°C and the resulting mixture was allowed to warm to room temperature and to stir for 2 h. The reaction mixture was then poured into cold MeOH (100 mL, between -10°C and 0°C) and the precipitated solid was filtered, washed with MeOH (100 mL) and dried *in vacuo*. The dried solid was then dissolved in water (100 mL), filtered and lyophilized to provide the methanesulfonate monohydrate salt **81.** (1.020 g, 84 %). ¹H NMR (DMSO-d₆): δ 1.75-1.85 (m, 2H), 2.35 (s, 3H), 2.78-2.84 (m, 2H), 2.96-3.12 (m, 4H), 3.36 (d, *J* = 12.3 Hz, 2H), 3.61 (t, *J* = 11.4 Hz, 2H), 3.94 (d, *J* = 12.9 Hz, 2H), 4.03 (s, 2H), 7.49 -7.55 (m, 1H), 7.76-7.84 (m, 3H), 7.99 (d, *J* = 0.9 Hz, 1H), 8.15 (d, *J* = 8.4 Hz, 1H), 8.25 (d, *J* = 8.4 Hz, 1H), 9.59 (s, 1H), 12.42 (s, 1H); ¹³C NMR (DMSO-d₆): δ 24.27, 33.86, 51.89, 54.51, 64.02, 119.70, 120.39, 123.53, 126.09, 126.45, 128.63, 133.66, 135.80, 138.71, 141.21, 144.57, 163.29; Anal. Calcd for C₂₄H₃₁N₃O₄S₂: C, 52.06; H, 5.46; N, 7.59, Karl-Fisher, 3.36. Found: C, 51.85; H, 5.35, N, 7.30, Karl-Fisher, 4.32.

Similarly, HCl, H₂SO₄, CH₃COOH, and succinic acid salts of **81** were prepared by substituting methanesulfonic acid with about an equivalent amount of HCl, H₂SO₄ and CH₃COOH, respectively.

### I) Preparation of 5,6-dihydro-5-oxo-11H-indeno[1,2-c]isoquinoline (6)

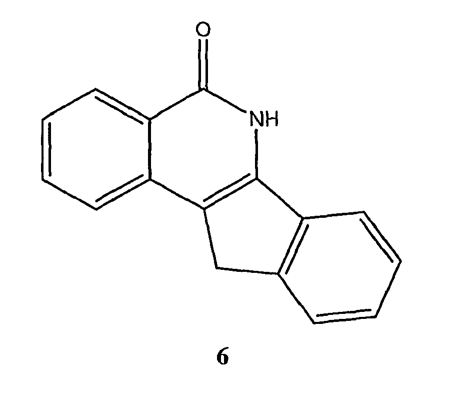

To a solution of homophthalic anhydride (324 mg, 2.0 mmol) in acetonitrile (15 mL) was added 2-cyanobenzyl bromide (431 mg, 2.0 mmol, 1.0 eq) and triethylamine (5 mL). The reaction was allowed to stir under inert atmosphere at room temperature for 30 minutes, after which time a yellow precipitate appeared. The reaction mixture was then heated at reflux for 18 h and the resulting white precipitate was filtered, washed using acetonitrile (3 x 8 mL) and dried under vacuum to provide Compound 6 as a white crystalline solid. Yield = 150 mg (32 %).

### m) Preparation of α-Bromodimethylhomophthalate (20a)

Dimethylhomophthalate (83.1 g) was dissolved in dichloromethane (2 L) and N-bromosuccinimide (121 g, 1.7 eq) was added. The resulting suspension was irradiated for 18 h with a 500 Watt quartz-halogen lamp, which brought the reaction mixture to reflux. The reaction mixture was then washed sequentially with saturated aqueous sodium bicarbonate (4 L), saturated aqueous sodium bisulfite (2 L), and saturated aqueous sodium chloride (2 L). The organic phase was dried using sodium sulfate with a small amount of silica added to remove polar impurities. The organic phase was filtered and concentrated *in vacuo* to provide Compound **20a** as a dark orange oil. Yield = 120.3 g (100 %).

### n) Preparation of 8-Methoxy-6H-11-oxa-6-aza-benzo[a]fluoren-5-one (22a)

α-Bromodimethylhomophthalate **(20a)** (1.16 g) and 2-hydroxy-5 - methoxybenzonitrile (0.6 g, 4 mmol, 1 eq) were dissolved by warming in acetonitrile (6 mL). Triethylamine (5.6 mL; 10 eq) was then added and the reaction was heated at reflux for 48 h under inert atmosphere, then cooled to room temperature. The reaction mixture was diluted with saturated sodium bicarbonate (40 mL) and the resulting suspension was allowed to stir for 2 h, and was then filtered. The filtercake was washed sequentially with 1 N HCl (2 x 50 mL), acetonitrile (2 x 50 mL) and dichloromethane (50 mL), then dried in a vacuum oven at 50°C for three days to provide Compound **22a** as an white solid. Yield = 0.81 g (76 %).

### o) Preparation of 8-Hydroxy-6H-11-oxa-6-aza-benzo[a]fluoren-5-one (23a)

8-Methoxy-*6H*-11-oxa-6-aza-benzo[*a*]fluoren-5-one (22a) (5.0 g) was cooled using an ice bath, and boron tribromide (1 M in methylene chloride, 95 mL, 95 mmol, 5 eq) added in a steady stream under nitrogen. The reaction was heated at reflux under inert atmosphere for two hours, then cooled to room temperature and poured into water (150 mL). The resulting suspension was allowed to stir for 1 h, filtered, and the solids were washed with water (2 x 200 mL). The solids were then diluted with 5 N sodium hydroxide (600 mL) using heating. The resulting solution was cooled to 0°C using an ice bath and the solution was acidified to pH 1 using conc. HCl. The resulting precipitate was vacuum filtered, and the solids washed sequentially with water (3 x 300 mL) and diethyl ether (300 mL) then dried overnight using a vacuum oven at 50°C to provide Compound **23a** as a gray solid. Yield 4.74 g (100 %).

### p) Preparation of 3-Nitroso-2-Phenyindole (28)

A solution of 2-phenylindole **(27)** (25 gm, 0.129 mol) in acetic acid (250 mL) was cooled to 18°C and a solution of sodium nitrite (8 g, 0.115 mol) in water (10 mL) was added dropwise while keeping the temperature of the reaction at ca. 20°C. The resulting reaction was stirred for 30 min at room temperature then diluted with ice water (250 mL). The reaction mixture was was filtered and the solid was washed with water then recrystallized using methanol to provide Compound **28.** Yield = 27.5 gm (96.4 %). ES-MS: 223.22 (M⁺+1); NMR (DMSO-d₆): δ 7.0 (m,1H), 7.1 (m, 1H), 7.22 (m, 1H), 7.32 (m, 2H), 7.40 (m, 1H), 7.48 (m, 2H), 7.60 (m,1H).

### q) Preparation of 3-Amino-2-Phenylindole (29)

To a solution of 3-nitroso-2-phenyl indole **(28)** (25 gm, 0.129 mol) in ethanol (450 ml) was added 2N sodium hydroxide (300 mL, 5.0 eq) followed by sodium dithionite (38 g). The reaction was heated at reflux for 5 h, then filtered. The solid was washed with water and dried under vacuum to provide Compound 29 as a yellow solid. Yield = 15 g (72.1 %). ES-MS: 209.25 (M⁺+ 1); NMR (DMSO-d₆): δ 7.0 (m, 1H), 7.1 (m, 1H), 7.22 (m, 1H), 7.32 (m, 2H), 7.40 (m, 1H), 7.48 (m, 2H), 7.60 (m, 1H).

### r) Preparation of 2-Phenylindole-3-ethylcarbamate (30)

To a 0 °C solution of 3-amino-2-phenylindole **(29)** (1.7 g, 8.17 mmol) in dichloromethane (150 ml) was added triethylamine (5 mL, 4.5 eq) followed by ethyl chloroformate (1 mL). The reaction was allowed to stir for 15 h, after which time the reaction mixture was diluted with water and transferred to a separatory funnel. The dichloromethane (50 mL), washed with water (2 x 50 mL), brine (50 mL) and dried over sodium sulfate. The solvent was removed and dried under vacuum to provide Compound **30** as a black solid (1.6 gm, 72.7 %). ES-MS: 281.25 (M⁺+1); NMR (DMSO-d₆): δ 1.30 (t,3H), 4.12 (t, 2H), 7.0 (m, 1H), 7.1 (m, 1H), 7.22 (m,2H), 7.32 (m, 2H), 7.40(m,1H), 7.48 (m, 2H), 7.60 (m, 1H).

### s) Preparation of 6H,11H-Indolo[3,2-c]Isoquinoline-5-one (31).

A solution of 2-Phenylindole-3-aminoethylcarbamate **(30)** (1.4 g, 5 mmol) in diphenyl ether (10 ml) was heated at reflux for 4 h, then cooled to room temperature. The reaction mixture was filtered and the solid was washed sequentially using warm hexane and warm dichloromethane and dried under vacuum to provide Compound **31** as a gray solid. Yield = 1.6 g (72.7 %). ES-MS: 235.25 (M⁺+1).

### t) Preparation of 6H,11H-Indolo[3,2-c]Isoquinoline-5-one-9,11-diacetate (32).

To a 0°C solution of 6H,11H-Indolo[3,2-c]Isoquinoline-5-one **(31)** (117 mg, 0.5 mmol) in dichloromethane (10 mL) was added triethylamine (2 mL, 30 eq) followed by acetic anhydride (1.8 mL, 35 eq). The reaction was stirred at room temperature for 48 h, then poured over ice and extracted with dichloromethane (100 mL). The dichloromethane layer was washed sequentially using water (2 x 20 mL) and brine (25 mL), then dried using sodium sulfate and concentrated *in vacuo*. The resulting solid residue was dried under vacuum to provide Compound **32** as a brown solid. Yield = 180 mg, 83.7 %. ES-MS: 430.57 (M⁺+1).

### u) Preparation of 6H,11H-Indolo[3,2-c]Isoquinoline-5-one-9,11-disulfonylchloride (33).

Compound **31** (117 mg, 0.5 mmol) was added to chlorosulfonic acid (2 mL, 60 eq) and the resulting reaction mixture was allowed to stir at room temperature for 4 hours, after which time the reaction mixture was poured over ice. The resulting precipitate was filtered, washed sequentially with water and ethyl acetate and dried under vacuum to provide Compound **33** as a light-yellow solid. Yield = 180 mg (83.7 %). ES-MS: 430.57 (M⁺+1).

### v) Preparation of 6H,11H-Indolo[3,2-c]Isoquinoline-5-one-9,11-disulfonamide (35a).

To a solution of **33** (215 mg, 0.5 mmol) in methanol (10 mL) at 0°C was added a 20 % solution of ammonia in methanol (10 mL). The reaction mixture was allowed to stir at room temperature for 15 hours and was then filtered. The resulting solid was washed with methanol and the dried under vacuum to provide Compound **35a** as a yellow solid. Yield = 140 mg , 71.4 %). ES-MS: 392.81 (M⁺+1).

### w) Preparation of N-acetylanthranilonitrile (36a)

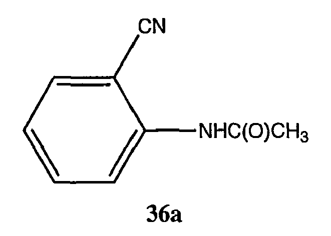

To a solution of anthranilonitrile (4.0 g, 32 mmol) in acetic anhydride (18 mL, 5.5 eq) at 90°C was added 1 drop of sulfuric acid and the resulting reaction was stirred at 90°C for 2 h, then allowed to sit at room temperature for 12 h. The reaction mixture was poured onto ice (ca. 200 mL) and the resulting solution was stirred for 2 h, after which time the solution was neutralized to pH 7.0 using 5 N sodium hydroxide. The resulting precipitate was filtered, washed using water (4 x 50 mL) and dried under vacuum for 72 h to provide Compound **36a** as a white crystalline solid. Yield = 1.07 g (16 %).

### x) Preparation of 6H,11H-indolo[3,2-c]isoquinolin-5-one (31)

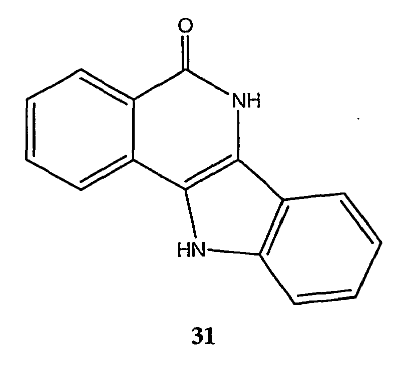

### From α-Brornodimethylhomophthalate

α-Bromodimethylhomophthalate **(20a)** (603 mg, 2.1 mmol) and N-acetylanthranilonitrile **(36a)** (370 mg, 1.1 eq) were dissolved in DMF (5 mL) under inert atmosphere. Potassium carbonate (1.45 g, 5.0 eq) was added and the reaction was stirred for 48 h at 100°C, then cooled to room temperature. The reaction mixture was poured into 1 N sodium hydroxide and the resulting mixture was extracted with EtOAc (50 mL). The EtOAc layer was washed sequentially with 1N HCl (50 mL), saturated aqueous sodium chloride (50 mL), dried over sodium sulfate, filtered and concentrated *in vacuo*. The resulting residue was dissolved by warming in toluene (70 mL) and the solution was cooled to room temperature and upon addition of hexanes (200 mL), a solid precipitate appeared. The solid precipitate was filtered, washed using hexanes (50 mL) and dried in a vacuum oven at 50°C for 72 h to provide Compound **31** as a yellow powder. Yield = 33 mg (6.7 %).

### y) Preparation of 6H,11H-thia-6-aza-benzo[a]fluorene-5-one (40a)

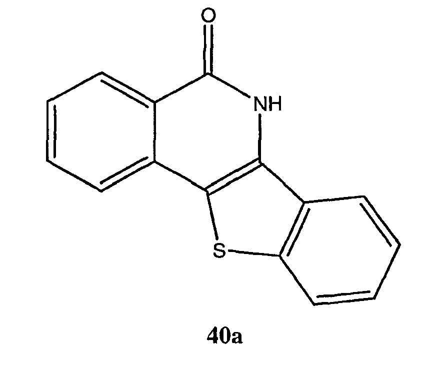

### From homophthalic anhydride:

A solution of 2-mercaptobenzonitrile (39a, i.e. Scheme 9, compound 39, wherein R₇-R₁₀ are hydrogen) (1.35 g, 10 mmol) and homophthalic anhydride **(11b)** (1.6 g, 10.0 mmol, 1.0 eq) in acetonitrile (150 mL) under inert atmosphere was warmed with stirring until all reactants were in solution. Triethylamine (6.9 mL, 50 mmol, 5.0 eq) was added and the reaction was heated at reflux for 72 hours, then cooled to room temperature. After cooling, the reaction mixture was filtered, and the collected solid was washed using methanol (3 x 50 mL), then dried in a vacuum oven at 50°C to provide Compound **40a** as a white solid. Yield = 225 mg (9 %).

### From α-bromodiniethylhomophthalate:

A solution of 2-mercaptobenzonitrile **(39a)** (1.35 g, 10 mmol) and α-bromodimethylhomophthalate **(20a)** (2.87 g, 10.0 mmol, 1.0 eq) in acetonitrile (150 mL) under inert atmosphere was warmed with stirring until all reactants were in solution. Triethylamine (6.9 mL, 50 mmol, 5.0 eq) was added and the reaction was heated at reflux for 72 hours, then cooled to room temperature. After cooling, the reaction mixture was filtered, and the collected solid was washed using methanol (3 x 50 mL), then dried in a vacuum oven at 50°C to provide Compound **40a** as a white solid. Yield = 250 mg (10 %).

### z) Preparation of 5,6-Dihydro-5-oxo-9-nitro-indeno[1,2-c]isoquinoline (53a).

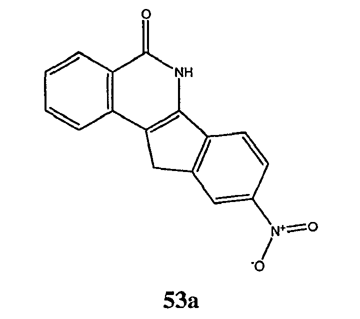

To a refluxing mixture of 2-methyl-4-nitro-benzonitrile (32.4 g, 0.2 mol) and NBS (44.470 g, 0.25 mol) in CCl₄ (300 ml) was added AIBN (0.325 g) and the resultant reaction mixture was refluxed for 4 hours. The reaction mixture was treated with AIBN (0.325 g, 31 mmol) and refluxed further for 4 hours. The reaction mixture was filtered, and the filtered succinimide was washed with CCl₄. The filtrate was concentrated in vacuo to provide a bromo compound (46 g). The bromo compound was dissolved in MeCN (200 ml), and to the reaction mixture was added homophthalic anhydride (30.780 g, 0.19 mol) at room temperature and under inert atmosphere. The reaction mixture was then treated with a solution of triethylamine (84 ml, 0.6 mol) in acetonitrile (100 ml). The reaction mixture was refluxed for 8 hours. The precipitate that formed was removed by filtration and washed with MeCN (100 ml). The washed precipitate was suspended in DMF (300 ml), which was heated at 130 °C, then cooled and filtered. The resultant solid was washed with DMF (100 ml) and dried under vacuum to provide Compound **53a** as a pale yellow solid (18.310 g, 33%). ¹H-NMR (DMSO-d₆): δ, 4.09 (s, 2H), 7.56 (m, 1H), 7.81 - 7.82 (m, 2H), 8.17 (d, J = 8.4 Hz, 1H), 8.26 - 8.34 (m, 2H), 8.44 (s, 1H), 12.47 (s, 1H).

### aa) Preparation of 5,6-Dihydro-5-oxo-9-amino-indeno[1,2-c]isoquinoline (54a).

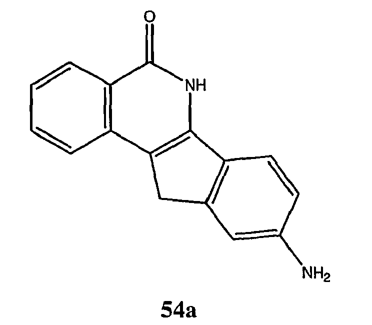

To a suspension of Compound **53a** (5.3 g, 0.019 mol) and ammonium formate (5.985 g, 0.095 mol) in DMF (100 ml) was added Pd-C (5%, 100 mg) at 80 °C. The reaction mixture was stirred at 100 °C for 1 hour. After the reaction mixture became clear, it was filtered through the pad of celite. The celite was washed with DMF. The filtrate was then diluted with ice, and the resultant solid was filtered, washed with water and dried at 80 °C under vacuum to provide Compound **54a** (3.2 g, 68%). ¹H-NMR (DMSO-d₆): δ, 3.89 (s, 2H), 7.18 (d, J = 8.4 Hz, 1H), 7.40 - 7.45 (m, 2H), 7.66 - 7.72 (m, 2H), 7.94 (d, J = 8.1 Hz, 1H), 8.21 (d, J = 8.1 Hz, 1H), 12.28 (s, 1H).

### bb) Preparation of N-[5,6-Dihydro-5-oxo-indeno[1,2-c]isoquinolin-9-yl]-4-bromo-butylamide (55a).

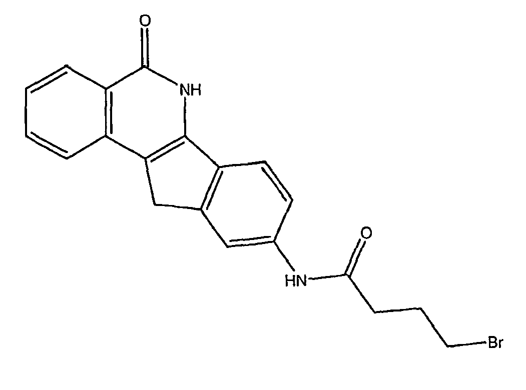

To a suspension of Compound **54a** (1.5 g, 0.006 mol) in saturated NaHCO₃ (150 ml) and ethyl acetate (100 ml) was added 4-bromobutyryl chloride (5 eq). The reaction mixture was stirred at room temperature for 1 hour. The resultant solid was isolated by filtration, washed with water and ethyl acetate, and dried under vacuum to provide Compound **55a** (1.625 g, 68%). ¹H-NMR (DMSO-d₆): δ, 2.09 - 2.13 (m, 2H), 2.47 - 2.52 (m, 2H), 3.58 (t, J = 6.6 Hz, 2H), 3.85 (s, 2H), 7.40 (t, J = 6.3 Hz, 1H), 7.50 (d, J = 8.4 Hz, 1H), 7.66 - 7.71 (m, 2H), 7.86 (d, J = 8.4 Hz, 1H), 7.92 (s, 1H), 8.20 (d, J = 8.1 Hz, 1H), 10.10 (s, 1H), 12.24 (s, 1H).

### cc) Preparation of N-[5,6-Dihydro-5-oxo-indeno[1,2-c]isoquinolin-9-yl]-4-chloro-butylamide (55b).

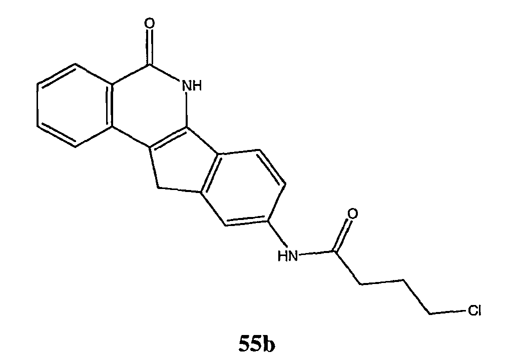

As set forth above for Compound **55a,** Compound **55b** (N-[5,6-dihydro-5-oxo-indeno[1,2-c]isoquinolin-9-yl]-4-chloro-butylamide) was prepared from the amino compound **54a** using chlorobutyryl chloride in the presence of aqueous NaHCO₃ and ethyl acetate. ¹H-NMR (DMSO-d₆): δ, 1.99 - 2.08 (m, 2H), 2.47 - 2.52 (m, 2H), 3.70 (t, J = 6.6 Hz, 2H), 3.86 (s, 2H), 7.38 - 7.44 (m, 1H), 7.50 (d, J = 8.1 Hz, 1H), 7.66 - 7.71 (m, 2H), 7.86 (d, J = 8.1 Hz, 1H), 7.95 (s, 1H), 8.21 (d, J = 8.1 Hz, 1H), 10.09 (s, 1H), 12.24 (s, 1H).

### dd) Preparation of N-[5,6-Dihydro-5-oxo-indeno[1,2-c]isoquinolin-9-yl]-2-chloroacetamide (55c).

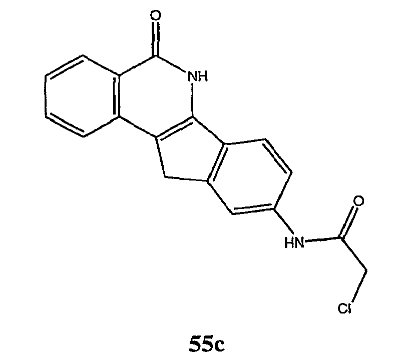

To a suspension of Compound **54a** (1.5 g, 0.0060 mol) in saturated NaHCO₃ (250 ml) and ethyl acetate (250 ml) was added chloroacetyl chloride (5 eq). The reaction mixture was stirred at room temperature for 1 hour. The resultant solid was isolated by filtration; washed sequentially with ethyl acetate, water and methanol; and dried under vacuum to provide Compound **55c** (1.6 g, 82%). ¹H-NMR (DMSO-d₆): δ, 3.89 (s, 2H), 4.27 (s, 2H), 7.40 - 7.45 (dd, J = 6.3 and 8.1 Hz, 1H), 7.52 (d, J = 8.1 Hz, 1H), 7.67 - 7.75 (m, 2H), 7.90 (d, J = 8.4 Hz, 1H), 7.94 (s, 1H), 8.21 (d, J = 8.1 Hz, 1H), 10.43 9s, 1H), 12.28 (s, 1H).

### ee) Preparation of N-[5,6-Dihydro-5-oxo-indeno[1,2-c]isoquinolin-9-yl]-4-morpholino-butylamide (73).

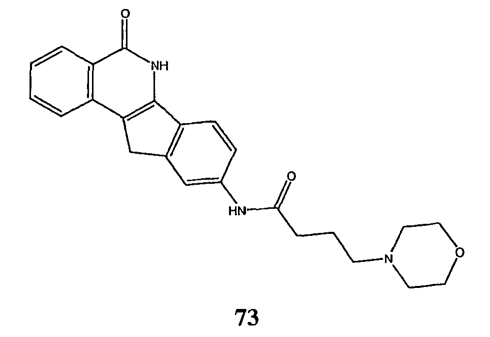

To a suspension of Compound **55a** (1.625 g, 0.004 mol) in DMF (25 ml) was added triethyl amine (5 ml) followed by morpholine (5 ml). The reaction mixture was heated at 140 to 155 °C for 1 hour, cooled to room temperature and allowed to stir overnight. The resultant solid precipitate was filtered; washed sequentially with DMF, water and methanol; and dried under vacuum to provide the free base of Compound **73** (1.380 g, 85%). ¹H-NMR (DMSO-d₆): δ, 1.72 - 1.76 (dd, J = 6.9 and 7.2 Hz, 2H), 2.26 - 2.37 (m, 8H), 3.51 - 3.54 (t, J = 4.2 Hz, 4H), 3.86 (s, 2H), 7.39 - 7.43 (dd, J = 6.3 and 6.6 Hz, 1H), 7.51 (d, J = 6.6 Hz, 1H), 7.66 - 7.74 (m, 2H), 7.86 (d, J = 8.4 Hz, 1H), 7.96 (s, 1H), 8.20 (d, J = 8.1 Hz, 1H), 10 .0 (s, 1H), 12.25 (s, 1H).

### ff) Preparation of the camphor sulfonic acid salt of 73.

To a suspension of the Compound **73** (free base) (0.403 g, 0.001 mol) in MeOH (20 ml) was added camphor sulfonic acid (255 mg, 0.0011 mol). The reaction mixture was allowed to stir at room temperature for 2 hours. The reaction mixture was then concentrated in vacuo, and the resultant residue was dissolved in distilled, deionized water (40 ml); treated with decolorising charcoal (0.5 g); and stirred at 90 to 100 °C for 30 min. The resultant solution was filtered through the pad of celite, and the celite was washed with water. The filtrate was lyopholized to provide the camphor sulfonic acid salt of 73 (0.450 g, 71%). ¹H-NMR (DMSO-d₆): d, 0.72 (s, 3H), 1.02 (s, 3H), 1.20 - 1.30 (m, 2H), 1.76 (d, J = 18Hz, 1H), 1.82 - 1.86 (m, 1H), 1.89 - 1.97 (m, 3H), 1.99 - 2.25 (m, 1H), 2.35 (d, J = 14.7 Hz, 1H), 2.43 - 2.48 (m, 2H), 2.64 - 2.71 (dd, J = 11.7 and 14.7 Hz, 1H), 2.85 (d, J = 14.7 Hz, 1H), 3.05 - 3.13 (m, 4H), 3.46 (d, J = 11.7 Hz, 2H), 3.64 (t, J = 12 Hz, 2H), 3.86 (s, 2H), 3.97 (d, J = 12.3 Hz, 2H), 7.39 - 7.44 (dd, J = 7.8 and 8.1 Hz, 1H), 7.52 (d, J = 8.1 Hz, 1H), 7.67 - 7.75 (m, 2H), 7.87 (d, J = 8.1 Hz, 1H), 7.96 (s, 1H), 8.21 (d, J = 8.1 Hz, 1H), 9.57 (s, 1H), 10.15 (s, 1H), 12.25 (s, 1H).

### gg) Preparation of 2-Dimethylamino-N-(5,6-Dihydro-5-oxo-indeno[1,2-c]isoqumolin-9-yl)-acetamide (43).

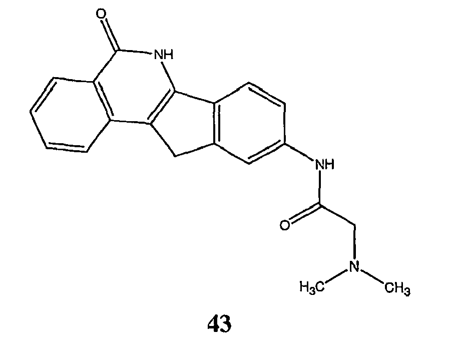

A suspension of Compound **55c** (1.6 g, 0.0049 mol) and dimethyl amine in ethanol (2N, 200 ml) was refluxed for 24 h. Additional solution of dimethyl amine in ethanol (2N, 200ml) was added. The reaction mixture was refluxed further for 24 hours and allowed to cool to room temperature. The resultant solid was filtered, washed with ethanol, and dried under vacuum to provide Compound **43** (1.510 g, 92%). ¹H-NMR (DMSO-d₆): δ, 2.27 (s, 6H), 3.07 (s, 2H), 3.85 (s, 2H), 7.38 - 7.43 (m, 1H), 7.58 (d, J = 8.1 Hz, 1H), 7.66 - 7.73 (m, 2H), 7.87 (d, J = 8.1Hz, 1H), 8.02 (s, 1H), 8.20 (d, J = 8.1 Hz, 1H), 9.82 (s, 1H), 12.21 (s, 1H); MS (ES⁺): *m*/*z* 334.01 (M +1).

### hh) Preparation of camphorsulfonic acid salt of 43.

To a suspension of Compound **43** (free base) (0.1.250 g, 0.0037 mol) in MeOH (200 ml) was added camphor sulfonic acid (0.915 g, 0.0039 mol). The reaction mixture was allowed to at room temperature for 1 hour, and concentrated in vacuo. The resultant residue was dissolved in distilled, deionized water (300 ml); filtered; treated with decolorising charcoal (1 g); and allowed to stir at 100 to 105 °C for 30 minutes. The resultant solution was filtered through a pad of celite, and the celite was washed with water. The filtrate was lyophilized to provide the camphor sulfonic acid salt of Compound **43** (1.660 g, 75%). ¹H-NMR (DMSO-d₆): δ, 0.72 (s, 3H), 1.02 9s, 3H), 1.20 - 1.30 (m, 2H), 1.74 - 1.92 (m, 3H), 2.17 - 2.25 (m, 1H), 2.35 (d, J = 14.7 Hz, 1H), 2.64 (t, J = 9.9 Hz, 1H, 2.80 (d, J = 14.7 Hz, 1H), 3.90 (s, 2H), 4.16 (s, 2H), 7.41 - 7.46 (dd, J = 6.3 and 8.1 hz, 1H), 7.53 (d, J = 8.1 Hz, 1H), 7.68 - 7.73 (m, 2H), 7.92 - 7.94 (m, 2H), 8.22 (d, J= 8.1 Hz, 1H), 9.77 (s, 1H), 10.68 (s, 1H), 12.29 (s, 1H).

### Example 2. Effect of Illustrative Isoquinoline Derivatives on PARS activity in cultured macrophages, using a whole-cell based assay and a purified enzyme assay.

Demonstration of illustrative Isoquinoline Derivatives' ability to inhibit PARS and prevent peroxynitrite induced cytotoxicity was shown using methods described in Virag et al., Br. J. Pharmacol. 1999, 126(3):769-77; and Immunology 1998, 94(3):345-55. Raw mouse macrophages were cultured in DMEM medium with high glucose and supplemented with 10 % fetal bovine serum. Cells were used at 80 % confluence in 12-well plates. Cells were pretreated with various concentrations (100 nM -1 gM) of an Isoquinoline Derivative for 10 min. Peroxynitrite, a prototypical oxidant which induces DNA single strand breakage, was used to induce PARS activation. Peroxynitrite was diluted in phosphate buffered saline (PBS) (pH 11.0) and added to the cells in a bolus of 50 µl. Cells were then incubated for 20 min. Peroxynitrite was decomposed by incubation for 30 min at pH 7.0, used as a control, and failed to influence the parameter studied. After the 20 min incubation, the cells were spun, the medium was aspirated and the cells were resuspended in 0.5 ml assay buffer (56 mM HEPES pH 7.5, 28 mM KCl, 28 mM NaCl, 2 mM MgCl₂, 0.01 % w/v digitonin and 0.125 µM NAD⁺ and 0.5 µCi/ml ³H-NAD⁺). Following an incubation in assay buffer, (10 min at 37°C), PARS activity was measured as follows: 200 µl ice cold 50 % w/v TCA was added and the samples were incubated for 4 hours at 4°C. Samples were then spun (10 min @ 10,000 g) and pellets washed twice with ice cold 5 % w/v TCA and solubilized overnight in 250 µl 2 % w/v SDS/0.1 N NaOH at 37°C. The contents of the tubes were added to 6.5 ml ScintiSafe Plus scintillation liquid (Fisher Scientific) and radioactivity was determined using a liquid scintillation counter (Wallac, Gaithersburg, MD). The results shown in Table 3 demonstrate that the illustrative Isoquinoline Derivatives significantly and dose-dependently inhibit the activation of PARS in the macrophage assay.

**Table 3. Inhibitory effect of various novel substituted isoquinolines on PARS activation in cultured murine macrophages.**

| Compound No. | % PARS inhibition at 1 µM | % PARS inhibition at 300 nM | % PARS inhibition at 100 nM |
|---|---|---|---|
| 2 | 60 | NT | 16 |
| 3a | 67 | NT | 8 |
| 3b | 25 | 0 | NT |
| 3c | 21 | 9 | NT |
| 4b | 88 | NT | 51 |
| 5a | 55 | NT | 10 |
| 5b | 33 | NT | 0 |
| 5c | 24 | NT | 0 |
| 5d | 48 | NT | 0 |
| 5e | 21 | NT | 0 |
| 6 | 65 | NT | 30 |
| 7 | 50 | NT | 0 |
| 8a | NT | 47 | NT |
| 8c | NT | 27 | NT |
| 8d | NT | 82 | 77 |
| 8e | NT | 68 | NT |
| 8g | NT | 55 | 34 |
| 8h | NT | 76 | 56 |
| 8j | NT | 76 | 34 |
| 8k | NT | 38 | 24 |
| 8l | NT | 84 | 34 |
| 8m | NT | 50 | NT |
| 8n | NT | 82 | 74 |
| 8o | NT | 55 | 48 |
| 8p | NT | 45 | 27 |
| 8q | NT | 28 | 20 |
| 8r | NT | 28 | 20 |
| 8s | 54 | NT | 30 |
| 8t | 29 | NT | 17 |
| 8u | NT | NT | 59 |
| 8w | NT | NT | 69 |
| 8x | NT | NT | 54 |
| 8y | NT | NT | 59 |
| 8z | NT | NT | 67 |
| 8aa | NT | NT | 64 |
| 8ab | NT | NT | 49 |
| 8ag | 59 | NT | 35 |
| 8ah | 63 | NT | 67 |
| 8ai | 90 | NT | 69 |
| 8ak | NT | 22* | 8* |
| 8al | 84 | NT | 49 |
| 8am | NT | NT | 65* |
| 8an | 40* | NT | 40* |
| 8ao | 60 | NT | 40 |
| 10a | NT | 59 | 55 |
| 10b | NT | 17 | 17 |
| 22a | 81 | NT | 51 |
| 22b | NT | 20* | 12* |
| 22c | 83 | 66 | 62 |
| 22d | 13* | NT | NT |
| 22e | 53 | 56 | 38 |
| 22f | 27 | 23 | NT |
| 22g | 27 | 23 | NT |
| 23a | 84 | 79 | 34 |
| 23b | 58 | 57 | 53 |
| 23c | 63 | 66 | 63 |
| 25a | 51 | 57 | 53 |
| 25b | 40 | 29 | 25 |
| 25c | 58 | 34 | 23 |
| 25d | 67 | 66 | 53 |
| 25e | 58 | 63 | 40 |
| 26a | 90 | 74 | 51 |
| 26b | 51* | 29* | 21* |
| 31 | 67 | 57 | 18 |
| 34 | NT | 33* | 14* |
| 35a | 75 | 55 | 14 |
| 35b | 42 | 51 | 25 |

| | | | |
|---|---|---|---|
| NT - Not Tested *tested in purified enzyme assay | | | |

The potency of inhibition on purified PARS enzyme was subsequently determined for selected Isoquinoline Derivatives, and the potency was compared with that of 3-aminobenzamide, a prototypical benchmark PARS inhibitor. The assay was performed in 96 well ELISA plates according to instructions provided with a commercially available PARS inhibition assay kit (Trevigen, Gaithersburg, MD). Briefly, wells were coated with 1 mg/mL histone (50 µl/well) at 4°C overnight. Plates were then washed four times with PBS and then blocked by adding 50 µl Strep-Diluent (supplied with the kit). After incubation (lh, room temperature), the plates were washed four times with PBS. Appropriate dilutions of PARS inhibitors were combined with 2x PARS cocktail (1.95 mM NAD⁺, 50 µM biotinylated NAD⁺ in 50 mM TRIS pH 8.0, 25 mM MgCl₂) and high specific activity PARS enzyme (both were supplied with the kit) in a volume of 50 µl. The reaction was allowed to proceed for 30 min at room temperature. After 4 washes in PBS, incorporated biotin was detected by peroxidase-conjugated streptavidin (1:500 dilution) and TACS Sapphire substrate. The assay confirmed the results of the macrophage-based PARS assay. For example, the PARS inhibitor **81** (mesylate salt) exerted 50 % inhibition of PARS activity in this assay at 3 nM, and thus was approximately 50,000 times more potent than the reference compound 3-aminobenzamide.

### Example 3: Effect of Illustrative Isoquinoline Derivatives on PARS activity using cell protection assay

The ability of illustrative Isoquinoline Derivatives to inhibit PARS and prevent peroxynitrite induced cytotoxicity was measured in a cell protection assay using the methods described in Jagtap et al., Bioorg. & Med. Chem. Letters 14 (2004) 81-85. Briefly, raw mouse macrophages were cultured then treated with an illustrative Isoquinoline Derivative at various concentrations ranging from 10 nM to 10 µM for about 15 minutes. Peroxynitrite (750 µM) was then added to the treated macrophages for a 15 minute incubation period to induce PARS activation. The media was removed and replaced with 0.5 mL HEPES (pH 7.5) containing 0.01% digitonin and ³H-NAD (0.5 µCi/mL, final concentration of NAD+ in buffer is 20 nM/L) and the resultant mixture was allowed to stand for 20 minutes. The cells were then scraped from the wells and placed in Eppendorf tubes containing 50% (w/v) of ice-cold TCA (200 µL). The tubes were maintained at 4 °C for four hours, centrifuged at 1800g for 10 minutes, and the supernatant removed. The resultant pellets were washed with 5% (w/v) TCA (200 µL, 2x), then solubilized overnight in 2 % (w/v) SDS/0.1 N NaOH (250 µl) at 37 °C. The contents of the tubes were then added to ScintiSafe Plus scintillation liquid (6.5 ml, Fisher Scientific) and radioactivity was measured using a liquid scintillation counter (Wallac, Gaithersburg, MD). The results shown in Table 4 demonstrate that the illustrative Isoquinoline Derivatives dose-dependently inhibit the activation of PARS.

**Table 4. Inhibitory effect of illustrative Isoquinoline Derivatives on cell protection**

| Compound No. | EC₅₀ (µM) |
|---|---|
| **43** | 0.020 |
| **73** | 0.010 |
| **99** | 0.225 |
| **100** | 0.180 |
| **102** | 0.095 |
| **104** | 0.065 |
| **105** | 0.080 |
| **107** | 0.010 |
| **108** | 0.3 |
| **109** | 0.4 |
| **110** | 0.625 |

### Example 4: Effects of illustrative Isoquinoline Derivatives in various disease models

### a: Effects of illustrative Isoquinoline Derivatives on in vitro cell disease models

In additional *in vitro* studies in isolated thymocytes, cells were exposed to peroxynitrite or hydrogen peroxide (toxic oxidant species) to induce cytotoxicity. In this system the toxicity is, at least in part, related to activation of the nuclear enzyme PARS. In this oxidant-stimulated thymocyte assay (described, in detail, in Virag et al., Immunology 94(3):345-55, 1998), the compounds tested prevented the oxidant-induced suppression of the viability of the cells and did so at the low nanomolar concentration range. An example of this response (Compound **81**, mesylate salt) is shown in Table 4. This assay represents an *in vitro* model of cells dying because of exposure to pro-oxidant species, as it occurs in during the reperfusion of ischemic organs.

**Table 5. Reduction of peroxynitrite induced cytotoxicity by 30 nM - 3 µM of the Isoquinoline Derivative 81 (mesylate salt).**

| | Control | +81 30 nM | +81 100 nM | +81 300 nM | +81 1 µM | +81 3 µM |
|---|---|---|---|---|---|---|
| Cytotoxicity | 98 % | 74 % | 39 % | 2 % | 0 % | 0 % |

### b: Effect of illustrative Isoquinoline Derivatives on in vivo models of inflammatory diseases

In order to substantiate the efficacy of the compounds in inflammatory diseases, the effect of illustrative Isoquinoline Derivatives was demonstrated in a systemic inflammatory model induced by bacterial lipopolysaccharide (LPS), which is reported to be responsible for causing reperfusion injuries and inflammatory diseases such as septic shock and systemic inflammatory response syndrome in animals (*see* Parrillo, N. Engl. J. Med., 328:1471-1478 (1993) and Lamping, J. Clin. Invest. 101:2065-2071 (1998). In a series of experiments, mice were pretreated with intraperitoneal injection of 0.1 and 1 mg/kg of compounds **81** (mesylate salt), **8p** and **8j,** and LPS at 10 mg/kg was injected i.p., and TNF-alpha was measured in the plasma at 90 minutes. As shown in Table 5, all compounds substantially reduced TNF production, indicative of the compounds' anti-inflammatory activity.

**Table 5. Reduction of LPS induced TNF production by 0.1-1 mg/kg intraperitoneal injection of the PARS inhibitor compounds 81 (mesylate salt), 8p and 8j in mice in vivo**

| | 8j (0.1 mg/kg) | 8j (1.0 mg/kg) | 8p (0.1 mg/kg) | 8p (1.0 mg/kg) | 81 (0.1 mg/kg) | 81 (1.0 mg/kg) | Vehicle |
|---|---|---|---|---|---|---|---|
| TNF (ng/ml) | 3831.6 | 5038.8 | 4470.0 | 5090.8 | 3714.6 | 3509.8 | 6994.0 |
| | ± 385.2 | ± 377.1 | ± 184.4 | ± 203.7 | ± 300.9 | ± 311.5 | ± 904.4 |

All compounds markedly suppressed LPS induced TNF production when compared to control.

At high doses, LPS causes multiple organ dysfunction resembling of septic shock, and ultimately death (in part because of the early release of TNF-alpha). Similarly, in a model induced by cecal ligation and puncture (CLP), the live bacteria that derive from the intestinal flora induce systemic inflammation and shock. Agents that inhibit inflammatory mediator production, PARS activation, and cell death in this model prevent mortality induced by LPS or CLP. In experiments with Balb/c mice, injection of 100 mg/kg LPS intraperitoneally caused death in 50 % of the animals over 24 h, whereas treatment of the animals with 3 mg/kg/day of compound **81** (mesylate salt) reduced the endotoxin-induced mortality to 10 % under the same experimental conditions. In response to CLP induced shock, compound **81** (mesylate salt) (3 mg/kg/day) caused a reduction in the mortality from 100 % death to 60 % death over 24 h.

The data demonstrating the reduction of TNF production by illustrative Isoquinoline Derivatives in animals subjected to an inflammation model, coupled with the fact that TNF production is an important trigger of inflammation in various inflammatory diseases (such as, for example, colitis, arthritis and neuroinflammation and shock) indicate that the Isoquinoline Derivatives have therapeutic effects in various systemic and local inflammatory diseases, including the rejection of transplanted organs, which entails both an inflammatory disease component and a reperfusion injury component and, accordingly, are useful for treating or preventing an inflammatory disease or a reperfusion injury.

### c: Effect of illustrative Isoquinoline Derivatives on in vivo models of reperfusion injury

In order to substantiate the efficacy of the Isoquinoline Derivatives in ischemia-reperfusion conditions, the effect of an illustrative Isoquinoline Derivative in a mouse model of ischemic and reperfused gut was tested. The superior mesenteric artery was occluded for 45 min, followed by a reperfusion for 1 h. Following the end of the reperfusion, gut permeability was measured with the FD4 method in evened gut sacks (Liaudet et al., Shock 2000, 14(2):134-41). Ischemia-reperfusion increased the permeability of the gut from 11 ± 4 to 216 ± 27 ml/min/cm², indicative of severe damage of the reperfused gut. Treatment with Compound **81** (mesylate salt) (3 mg/kg i.v., injected 10 min prior to initiation of reperfusion) reduced the increase in the permeability of the gut by approximately 73 %, indicating a marked maintenance of the gut function. The ischemia-reperfusion studies in the gut were associated with a 80 % mortality over 12 hours, whereas only 15 % mortality was noted in the animals treated with **81** (mesylate salt).

In another set of experiments, the effect of Compound **81** (mesylate salt) in a rat model of middle cerebral artery occlusion/reperfusion was assayed as described in Abdelkarim et al., Int J Mol Med. 2001, 7(3):255-60. Occlusion lasted for 2 hours, followed by reperfusion for 24 hours. Infarct size was quantified with tetrazolium staining. Compound **81** (mesylate salt) was administered at 3 mg/kg/day in 3 divided intraperitoneally injected doses, the first dose being administered 10 min prior to the initiation of reperfusion. There was an approximately 80 % reduction in the degree of cortical necrosis and neuronal death in the animals administered with **81** (mesylate salt), when compared to vehicle-treated controls. This protection also translated into functional benefit, such as neurological improvements in the PARS inhibitor treated group.

These data indicate that the Isoquinoline Derivatives have therapeutic effects in various systemic and local conditions of reperfusion injuries, including the rejection of transplanted organs, which entails both an inflammatory disease component and a reperfusion injury component and, accordingly, are useful for treating or preventing an inflammatory disease or a reperfusion injury or reoxygenation injury resulting from organ transplantation.

### d: Effect of illustrative Isoquinoline Derivatives in a diabetes model

PARS inhibitors and PARS deficiency are known to reduce the development of diabetes and the incidence of diabetic complications (Mabley et al., Br J Pharmacol. 2001, 133(6):909-9; and Soriano et al., Nat Med. 2001, 7(1):108-13). In order to substantiate the efficacy of the Isoquinoline Derivatives in a diabetes model, a single high-dose streptozotocin model of diabetes was conducted as previously described. Briefly, 160 mg/kg streptozotocin was injected to mice treated with vehicle or with illustrative Isoquinoline Derivatives intraperitoneally (3 mg/kg) and 3 days later blood sugar levels were determined using a blood glucose meter. The data shown in Table 6 demonstrate that the illustrative Isoquinoline Derivatives attenuate the streptozotocin-induced onset of diabetes as they reduce the hyperglycemia.

**Table 6. Reduction of streptozotocin (STZ) induced hyperglycemia by 3 mg/kg intraperitoneal injection of the PARS inhibitor compounds 81 (mesylate salt), 8p and 8j in mice in vivo**

| | Basal | STZ + Vehicle | STZ + 8j | STZ + 8p | 81 |
|---|---|---|---|---|---|
| Glucose (mg/ml) | 153 ± 21 | 320 ± 13 | 253 ± 24 | 264 ± 24 | 244 ± 21 z |

Accordingly, the Isoquinoline Derivatives are useful for treating or preventing diabetes or a diabetic complication.

### e: Effects of illustrative Isoquinoline Derivatives in overcoming temozolomide resistance in a mismatch repair deficient malignant glioma xenograft

Temozolomide (TMZ) is a methylating agent used in the treatment of malignant gliomas. DNA is methylated by TMZ primarily at the O6 and N7 positions of guanine and N3 of adenine. The cytotoxicity of TMZ is attributed to futile attempts by the mismatch repair system (MMR) to process O6-methylguanine. Tumor cells deficient in MMR are resistant to TMZ. The N-methylpurine adducts are efficiently removed by base excision repair (BER). Cells that are deficient in BER have heightened sensitivity to methylating agents. One of the components of BER is the enzyme PARP.

Inhibition of PARP was examined to determine whether it increases the cytotoxicity of TMZ, particularly in MMR-deficient cells. Groups of ten mice bearing procarbazine resistant xenograft tumors, which are cross-resistant to TMZ, were randomly assigned to one of four separate treatment arms. The four treatment arms were: 1) control arm (5% dextrose in sterile water); 2) compound **81** (mesylate salt) alone; 3) TMZ alone; and 4) combination of compound **81** (mesylate salt) and TMZ. Treatments were administered when the tumor volumes reached the size of 100-500 mm³ on Day 1 only, by intraperitoneal injections as set forth below. The tumors were subsequently measured twice weekly with hand-held vernier calipers and tumor volumes were calculated as follows: (width² x length)/2. Animals tested out of the study when the tumor volume exceeded 1000 mm³ and when tumor volume was greater than five times the tumor volume at the time of initial treatment.

Athymic mice, transplanted with MMR proficient (D-245 MG) or deficient (D-245 MG (PR) xenografts were treated with a combination of TMZ and Compound **81** (mesylate salt). For the tumors deficient in MMR, the most effective dose of compound **81** (mesylate salt) was found to be 150 mg/kg, administered i.p. three times at 4 hr intervals with the first injection in combination with 262.5 mg/kg TMZ (0.75 LD10). This dose of TMZ induced no partial regressions and approximately a 4-day tumor-control growth delay in two experiments. The combination therapy increased the growth delay by 21.6 and 9.7 days (P=0.001 and P=0.006, respectively) with partial regressions observed in 4 of 8 and 3 of 9 mice. The addition of compound **81** (mesylate salt) also increased the tumor growth delay in MMR proficient xenografts. In these experiments, mice were treated with 200 mg/kg of compound **81** (mesylate salt) in combination with 88 mg/kg TMZ. The tumor growth delay for TMZ alone in these mice was 43.1 and 39.2 days, in two sets of experiments. The combination therapy resulted in a modest increase in growth delay to 48.9 and 45.7 days (P=0.001 and P=0.003, respectively). These results indicate that inhibition of PARP by Compound **81** (mesylate salt), an illustrative Isoquinoline Derivative, increases the efficacy of TMZ in treatment of malignant gliomas, particularly in tumors deficient in MMR.

As shown in Figure 1, compound **81** (mesylate salt) markedly enhances the antitumor effects of TMZ *in vivo.*

Accordingly, Compound **81** (mesylate salt), an illustrative Isoquinoline Derivative, is useful for treating or preventing cancer, such as CNS and brain cancers, including gliomas.

The present invention is not to be limited in scope by the specific embodiments disclosed in the examples which are intended as illustrations of a few aspects of the invention and any embodiments that are functionally equivalent are within the scope of this invention. Indeed, various modifications of the invention in addition to those shown and described herein will become apparant to those skilled in the art and are intended to fall within the scope of the appended claims.

A number of references have been cited, the entire disclosures of which have been incorporated herein in their entirety.

Further embodiments are the following:
1. A method for treating cancer, comprising administering to an animal in need thereof, an effective amount of a compound having the formula **(I):** 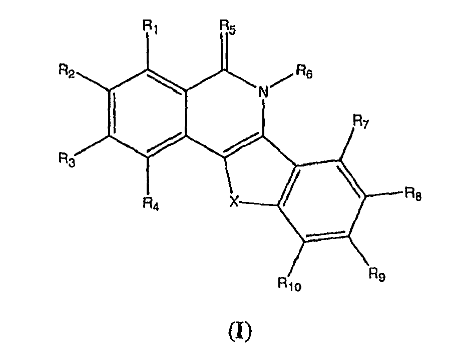 or a pharmaceutically acceptable salt thereof,
   wherein:
   R₅ is O, NH or S;
   R₆ is -H or -C₁-C₅ alkyl_{;}
   X is -C(O)-, -CH₂-, -CH(halo)-, -CH(OH)-(CH₂)ₙ-, -CH(OH), -CH(-aryl)-, -O-, -NH-, -S-, -CH(NR₁₁R₁₂)- or -N(SO₂Y)-, wherein Y is -OH, -NH₂ or -(C₁-C₅ alkyl)-(-3-to 7-membered monocyclic heterocycle);
   R₁₁ and R₁₂ are independently -hydrogen or -C₁-C₁₀ alkyl, or N, R₁₁ and R₁₂ are taken together to form a nitrogen-containing-3- to 7-membered monocyclic heterocycle;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A-B;
   A is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -NZ₁Z₂, -(C₁-C₅ alkylene)-NZ₁,Z₂, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -C(NH)NH₂, each of which, other than -NZ₁Z₂, -C(O)OH, or -C(NH)NH₂, is unsubstituted or substituted with one or more of -O-(C₁-C₅ alkyl), -halo, -hydroxy, -NO₂, -CN, -NZ₁Z₂, -nitrogen-containing-3- to 7-membered monocyclic heterocycle, -C₁-C₁₀ alkyl, -C₂-C₁₀ alkenyl, -C₂-C₁₀ alkynyl, -aryl, -benzyl, -C(O)OH, -C₁-C₅ alkylene-C(O)O-(C₁-C₅ alkyl) or -C₁-C₅ alkylene-OC(O)-(C₁-C₅ alkyl);
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀, alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
2. A method for treating cancer, comprising administering to an animal in need thereof, an effective amount of a compound having the formula **(II):** 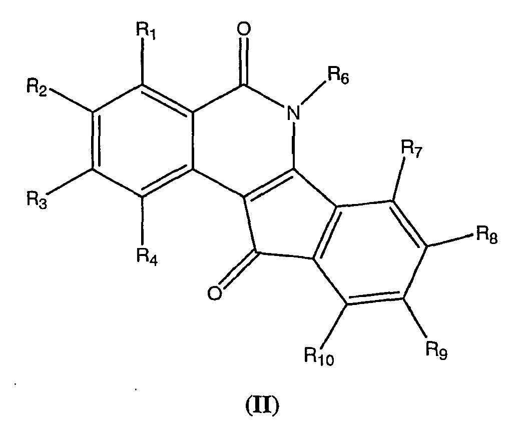 or a pharmaceutically acceptable salt thereof,
   wherein:
   R₆ is -H or C₁-C₅ alkyl;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A'-B';
   A' is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B' is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂;
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   A is -SO₂-, -SO₂NH-, -NHCO-, -NHCONH-, -O-, -CO-, -OC(O)-, -C(O)O-, -CONH-, -CON(C₁-C₅ alkyl)-, -NH-, -CH₂-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂; and
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle.
3. A method for treating cancer, comprising administering to an animal in need thereof, an effective amount of a compound having the formula **(III):** 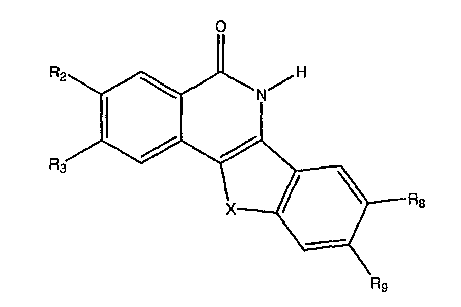 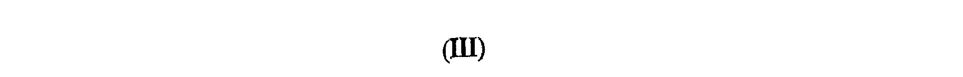 or a pharmaceutically acceptable salt thereof,
   wherein:
   X is -CH₂- or -O-;
   R₂ and R₃ are independently -hydrogen, -halo, -halo-substituted C₁-C₅ alkyl, -hydroxy, -O-(C₁-C₅ alkyl), -C₁-C₅ alkyl, -NO₂, -NH₂, -CONH₂, -C(O)OH, -OC(O)-C₁-C₅ alkyl or -C(O)O-C₁-C₅ alkyl;
   R₈ and R₉ are independently -hydrogen or -A-B;
   A is -SO₂-, -SO₂NH- or -NHCO-;
   B is -C₁-C₅ alkyl, -NZ₁Z₂, -3- to 7-membered monocyclic heterocycle, or -7- to 10-membered bicyclic heterocycle, each of which is unsubstituted or substituted with one or more of -hydroxy-substituted C₁-C₅ alkyl, -amino-substituted C₁-C₅ alkyl, -3- to 7-membered monocyclic heterocycle, or -7- to 10-membered bicyclic heterocycle, each unsubstituted or substituted with -C₁-C₁₀ alkyl or -hydroxy-substituted C₁-C₅ alkyl; and
   Z₁ and Z₂ are independently hydrogen or -C₁-C₈ alkyl, which is unsubstituted or substituted with one or more of -hydroxy or -N(Z₃)(Z₄), where Z₃ and Z₄ are independently, -H or -C₁-C₅ alkyl, which is unsubstituted or substituted with one or more of -hydroxy or -NH₂; or N, Z₃ and Z₄ are taken together to form a nitrogen-containing-3-to 7-membered monocyclic heterocycle; or N, Z₁ and Z₂ are taken together to form a nitrogen-containing-3- to 7-membered monocyclic heterocycle.
4. A method of for treating cancer, comprising administering to an animal in need thereof, an effective amount of a compound having the formula **(IV):** 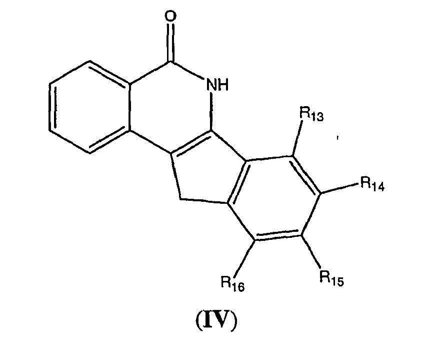 or a pharmaceutically acceptable salt thereof,
   wherein:
   R₁₃ and R₁₆ are hydrogen;
   one of the R₁₄ and R₁₅ groups is -NHC(O)-(CH₂)ₙ-NZ₁Z₂, and the other group is - hydrogen;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
5. The method of item 1, wherein the cancer is colorectal cancer, lung cancer, pancreatic cancer, esophageal cancer, stomach cancer, skin cancer, leukemia, lymphoma, non-Hodgkin's lymphoma, testicular cancer, bladder cancer, kidney cancer, liver cancer, breast cancer, prostate cancer, head and neck cancer, brain cancer, cancer of the central nervous system, uterine cancer, cervical cancer, or ovarian cancer.
6. The method of item 2, wherein the cancer is colorectal cancer, lung cancer, pancreatic cancer, esophageal cancer, stomach cancer, skin cancer, leukemia, lymphoma, non-Hodgkin's lymphoma, testicular cancer, bladder cancer, kidney cancer, liver cancer, breast cancer, prostate cancer, head and neck cancer, brain cancer, cancer of the central nervous system, uterine cancer, cervical cancer, or ovarian cancer.
7. The method of item 3, wherein the cancer is colorectal cancer, lung cancer, pancreatic cancer, esophageal cancer, stomach cancer, skin cancer, leukemia, lymphoma, non-Hodgkin's lymphoma, testicular cancer, bladder cancer, kidney cancer, liver cancer, breast cancer, prostate cancer, head and neck cancer, brain cancer, cancer of the central nervous system, uterine cancer, cervical cancer, or ovarian cancer.
8. The method of item 4, wherein the cancer is colorectal cancer, lung cancer, pancreatic cancer, esophageal cancer, stomach cancer, skin cancer, leukemia, lymphoma, non-Hodgkin's lymphoma, testicular cancer, bladder cancer, kidney cancer, liver cancer, breast cancer, prostate cancer, head and neck cancer, brain cancer, cancer of the central nervous system, uterine cancer, cervical cancer, or ovarian cancer.
9. The method of item 1, wherein the cancer is melanoma, metastatic brain cancer, or glioma.
10. The method of item 2, wherein the cancer is melanoma, metastatic brain cancer, or glioma.
11. The method of item 3, wherein the cancer is melanoma, metastatic brain cancer, or glioma.
12. The method of item 4, wherein the cancer is melanoma, metastatic brain cancer, or glioma.
13. The method of item 9, wherein the glioma is pilocytic astrocytoma, astrocytoma, anaplastic astrocytoma, or glioblastoma multiforme.
14. The method of item 10, wherein the glioma is pilocytic astrocytoma, astrocytoma, anaplastic astrocytoma, or glioblastoma multiforme.
15. The method of item 11, wherein the glioma is pilocytic astrocytoma, astrocytoma, anaplastic astrocytoma, or glioblastoma multiforme.
16. The method of item 12, wherein the glioma is pilocytic astrocytoma, astrocytoma, anaplastic astrocytoma, or glioblastoma multiforme.
17. The method of item 1, further comprising administering an effective amount of temozolomide, procarbazine, dacarbazine, irinotecan, Interleukin-2, or a combination thereof.
18. The method of item 17, wherein the compound is 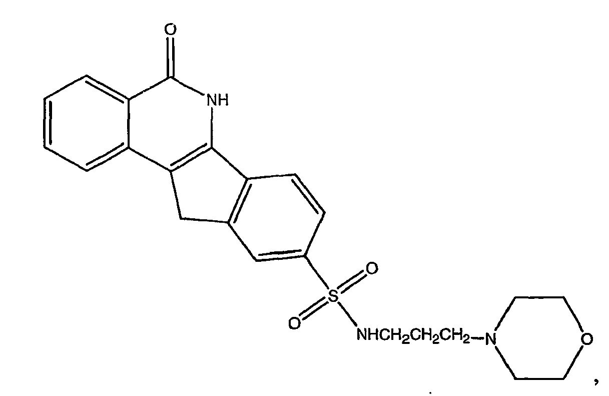 or a pharmaceutically acceptable salt thereof.
19. The method of item 18, wherein the pharmaceutically acceptable salt is mesylate.
20. The method of item 2, further comprising administering an effective amount of temozolomide, procarbazine, dacarbazine, irinotecan, Interleukin-2, or a combination thereof.
21. The method of item 3, further comprising administering an effective amount of temozolomide, procarbazine, dacarbazine, irinotecan, Interleukin-2, or a combination thereof.
22. The method of item 4, further comprising administering an effective amount of temozolomide, procarbazine, dacarbazine, irinotecan, Interleukin-2, or a combination thereof.
23. A composition comprising an effective amount of temozolomide, a pharmacologically acceptable vehicle or carrier, and an effective amount of a compound or pharmaceutically acceptable salt of a compound having the formula **(I):** 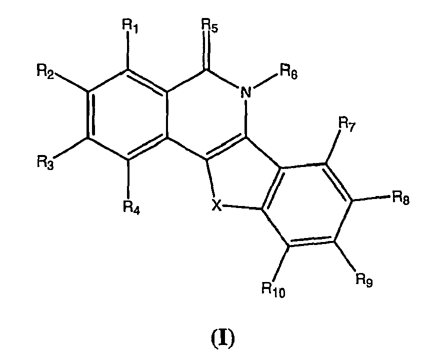 or a pharmaceutically acceptable salt thereof,
   wherein:
   R₅ is O, NH or S;
   R₆ is -H or -C₁-C₅ alkyl;
   X is -C(O)-, -CH₂-, -CH(halo)-, -CH(OH)-(CH₂)ₙ-, -CH(OH), -CH(-aryl)-, -O-, -NH-, -S-, -CH(NR₁₁R₁₂)- or -N(SO₂Y)-, wherein Y is -OH, -NH₂ or -(C₁-C₅ alkyl)-(-3-to 7-membered monocyclic heterocycle);
   R₁₁ and R₁₂ are independently -hydrogen or -C₁-C₁₀ alkyl, or N, R₁₁ and R₁₂ are taken together to form a nitrogen-containing-3- to 7-membered monocyclic heterocycle;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A-B;
   A is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -NZ₁Z₂, -(C₁-C₅ alkylene)-NZ₁,Z₂, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -C(NH)NH₂, each of which, other than -NZ₁Z₂, -C(O)OH, or -C(NH)NH₂, is unsubstituted or substituted with one or more of -O-(C₁-C₅ alkyl), -halo, -hydroxy, -NO₂, -CN, -NZ₁Z₂, -nitrogen-containing-3- to 7-membered monocyclic heterocycle, -C₁-C₁₀ alkyl, -C₂-C₁₀ alkenyl, -C₂-C₁₀ alkynyl, -aryl, -benzyl, -C(O)OH, -C₁-C₅ alkylene-C(O)O-(C₁-C₅ alkyl) or -C₁-C₅ alkylene-OC(O)-(C₁-C₅ alkyl);
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀, alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
24. A composition comprising an effective amount of temozolomide, a pharmacologically acceptable vehicle or carrier, and an effective amount of a compound or pharmaceutically acceptable salt of a compound having the formula **(II):** 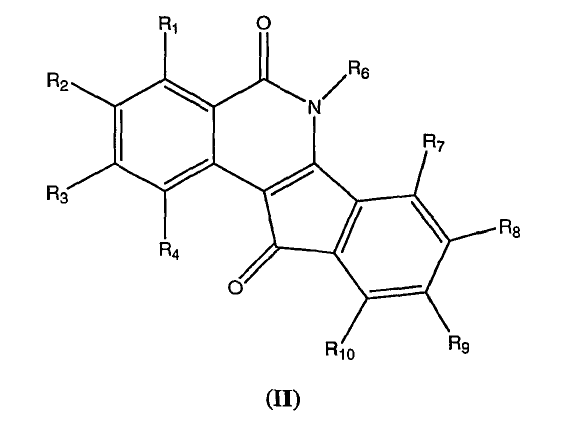 or a pharmaceutically acceptable salt thereof,
   wherein:
   R₆ is -H or C₁-C₅ alkyl;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A'-B';
   A' is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B' is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂;
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   A is -SO₂-, -SO₂NH-, -NHCO-, -NHCONH-, -O-, -CO-, -OC(O)-, -C(O)O-, -CONH-, -CON(C₁-C₅ alkyl)-, -NH-, -CH₂-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂; and
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle.
25. A composition comprising an effective amount of temozolomide, a pharmacologically acceptable vehicle or carrier, and an effective amount of a compound or pharmaceutically acceptable salt of a compound having the formula **(III):** 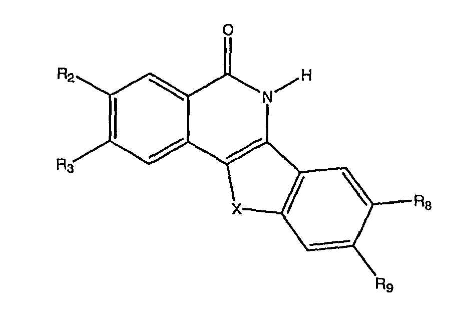 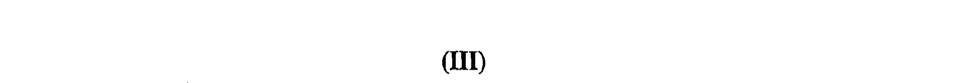 or a pharmaceutically acceptable salt thereof,
   wherein:
   X is -CH₂- or -O-;
   R₂ and R₃ are independently -hydrogen, -halo, -halo-substituted C₁-C₅ alkyl, -hydroxy, -O-(C₁-C₅ alkyl), -C₁-C₅ alkyl, -NO₂, -NH₂, -CONH₂, -C(O)OH, -OC(O)-C₁-C₅ alkyl or -C(O)O-C₁-C₅ alkyl;
   R₈ and R₉ are independently -hydrogen or -A-B;
   A is -SO₂-, -SO₂NH- or -NHCO-;
   B is -C₁-C₅ alkyl, -NZ₁Z₂, -3- to 7-membered monocyclic heterocycle, or -7- to 10-membered bicyclic heterocycle, each of which is unsubstituted or substituted with one or more of -hydroxy-substituted C₁-C₅ alkyl, -amino-substituted C₁-C₅ alkyl, -3- to 7-membered monocyclic heterocycle, or -7- to 10-membered bicyclic heterocycle, each unsubstituted or substituted with -C₁-C₁₀ alkyl or -hydroxy-substituted C₁-C₅ alkyl; and
   Z₁ and Z₂ are independently hydrogen or -C₁-C₈ alkyl, which is unsubstituted or substituted with one or more of -hydroxy or -N(Z₃)(Z₄), where Z₃ and Z₄ are independently, -H or -C₁-C₅ alkyl, which is unsubstituted or substituted with one or more of -hydroxy or -NH₂; or N, Z₃ and Z₄ are taken together to form a nitrogen-containing-3-to 7-membered monocyclic heterocycle; or N, Z₁ and Z₂ are taken together to form a nitrogen-containing-3- to 7-membered monocyclic heterocycle.
26. A composition comprising an effective amount of temozolomide, a pharmacologically acceptable vehicle or carrier, and an effective amount of a compound or pharmaceutically acceptable salt of a compound having the formula **(IV):** 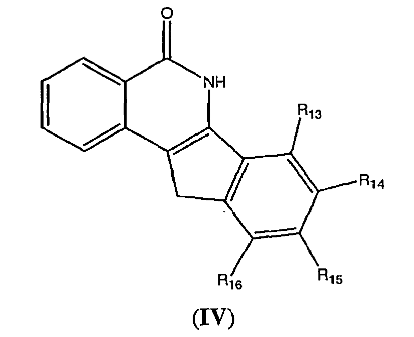 or a pharmaceutically acceptable salt thereof,
   wherein:
   R₁₃ and R₁₆ are hydrogen;
   one of the R₁₄ and R₁₅ groups is -NHC(O)-(CH₂)ₙ-NZ₁Z₂, and the other group is - hydrogen;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
27. A composition comprising an effective amount of procarbazine, a pharmacologically acceptable vehicle or carrier, and an effective amount of a compound or pharmaceutically acceptable salt of a compound having the formula **(I):** 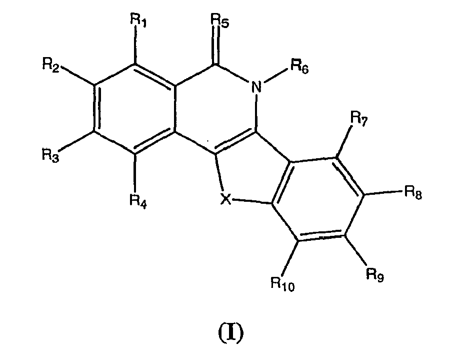 or a pharmaceutically acceptable salt thereof,
   wherein:
   R₅ is O, NH or S;
   R₆ is -H or -C₁-C₅ alkyl;
   X is -C(O)-, -CH₂-, -CH(halo)-, -CH(OH)-(CH₂)ₙ-, -CH(OH), -CH(-aryl)-, -O-, -NH-, -S-, -CH(NR₁₁R₁₂)- or -N(SO₂Y)-, wherein Y is -OH, -NH₂ or -(C₁-C₅ alkyl)-(-3-to 7-membered monocyclic heterocycle);
   R₁₁ and R₁₂ are independently -hydrogen or -C₁-C₁₀ alkyl, or N, R₁₁ and R₁₂ are taken together to form a nitrogen-containing-3- to 7-membered monocyclic heterocycle;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A-B;
   A is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -NZ₁Z₂, -(C₁-C₅ alkylene)-NZ₁,Z₂, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -C(NH)NH₂, each of which, other than -NZ₁Z₂, -C(O)OH, or -C(NH)NH₂, is unsubstituted or substituted with one or more of -O-(C₁-C₅ alkyl), -halo, -hydroxy, -NO₂, -CN, -NZ₁Z₂, -nitrogen-containing-3- to 7-membered monocyclic heterocycle, -C₁-C₁₀ alkyl, -C₂-C₁₀ alkenyl, -C₂-C₁₀ alkynyl, -aryl, -benzyl, -C(O)OH, -C₁-C₅ alkylene-C(O)O-(C₁-C₅ alkyl) or -C₁-C₅ alkylene-OC(O)-(C₁-C₅ alkyl);
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀, alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
28. A composition comprising an effective amount of procarbazine, a pharmacologically acceptable vehicle or carrier, and an effective amount of a compound or pharmaceutically acceptable salt of a compound having the formula **(II):** 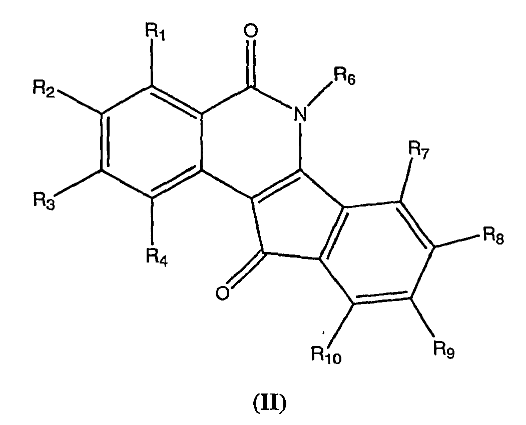 or a pharmaceutically acceptable salt thereof,
   wherein:
   R₆ is -H or C₁-C₅ alkyl;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A'-B';
   A' is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B' is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂;
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   A is -SO₂-, -SO₂NH-, -NHCO-, -NHCONH-, -O-, -CO-, -OC(O)-, -C(O)O-, -CONH-, -CON(C₁-C₅ alkyl)-, -NH-, -CH₂-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂; and
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle.
29. A composition comprising an effective amount of procarbazine, a pharmacologically acceptable vehicle or carrier, and an effective amount of a compound or pharmaceutically acceptable salt of a compound having the formula **(III):** 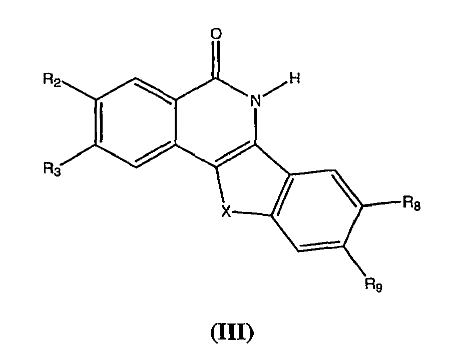 or a pharmaceutically acceptable salt thereof,
   wherein:
   X is -CH₂- or -O-;
   R₂ and R₃ are independently -hydrogen, -halo, -halo-substituted C₁-C₅ alkyl, -hydroxy, -O-(C₁-C₅ alkyl), -C₁-C₅ alkyl, -NO₂, -NH₂, -CONH₂, -C(O)OH, -OC(O)-C₁-C₅ alkyl or -C(O)O-C₁-C₅ alkyl;
   R₈ and R₉ are independently -hydrogen or -A-B;
   A is -SO₂-, -SO₂NH- or -NHCO-;
   B is -C₁-C₅ alkyl, -NZ₁Z₂, -3- to 7-membered monocyclic heterocycle, or -7- to 10-membered bicyclic heterocycle, each of which is unsubstituted or substituted with one or more of -hydroxy-substituted C₁-C₅ alkyl, -amino-substituted C₁-C₅ alkyl, -3- to 7-membered monocyclic heterocycle, or -7- to 10-membered bicyclic heterocycle, each unsubstituted or substituted with -C₁-C₁₀ alkyl or -hydroxy-substituted C₁-C₅ alkyl; and
   Z₁ and Z₂ are independently hydrogen or -C₁-C₈ alkyl, which is unsubstituted or substituted with one or more of -hydroxy or -N(Z₃)(Z₄), where Z₃ and Z₄ are independently, -H or -C₁-C₅ alkyl, which is unsubstituted or substituted with one or more of -hydroxy or -NH₂; or N, Z₃ and Z₄ are taken together to form a nitrogen-containing-3-to 7-membered monocyclic heterocycle; or N, Z₁ and Z₂ are taken together to form a nitrogen-containing-3- to 7-membered monocyclic heterocycle.
30. A composition comprising an effective amount of procarbazine, a pharmacologically acceptable vehicle or carrier, and an effective amount of a compound or pharmaceutically acceptable salt of a compound having the formula **(IV):** 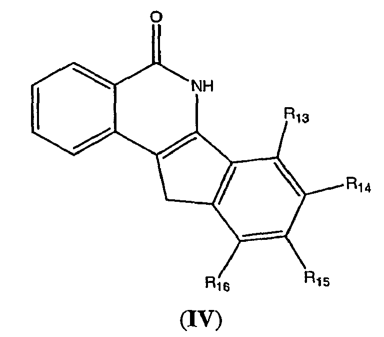 or a pharmaceutically acceptable salt thereof,
   wherein:
   R₁₃ and R₁₆ are hydrogen;
   one of the R₁₄ and R₁₅ groups is -NHC(O)-(CH₂)ₙ-NZ₁Z₂, and the other group is - hydrogen;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
31. A composition comprising an effective amount of dacarbazine, a pharmacologically acceptable vehicle or carrier, and an effective amount of a compound or pharmaceutically acceptable salt of a compound having the formula **(I):** 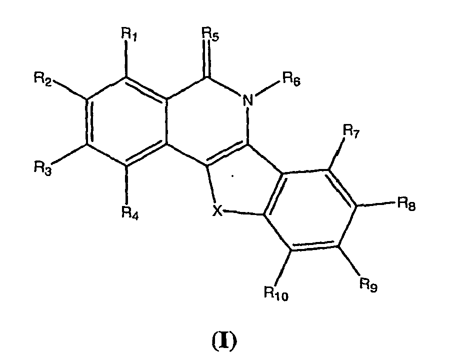 or a pharmaceutically acceptable salt thereof,
   wherein:
   R₅ is O, NH or S;
   R₆ is -H or -C₁-C₅ alkyl;
   X is -C(O)-, -CH₂-, -CH(halo)-, -CH(OH)-(CH₂)ₙ-, -CH(OH), -CH(-aryl)-, -O-, -NH-, -S-, -CH(NR₁₁R₁₂)- or -N(SO₂Y)-, wherein Y is -OH, -NH₂ or -(C₁-C₅ alkyl)-(-3-to 7-membered monocyclic heterocycle);
   R₁₁ and R₁₂ are independently -hydrogen or -C₁-C₁₀ alkyl, or N, R₁₁ and R₁₂ are taken together to form a nitrogen-containing-3- to 7-membered monocyclic heterocycle;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A-B;
   A is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -NZ₁Z₂, -(C₁-C₅ alkylene)-NZ₁,Z₂, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -C(NH)NH₂, each of which, other than -NZ₁Z₂, -C(O)OH, or -C(NH)NH₂, is unsubstituted or substituted with one or more of -O-(C₁-C₅ alkyl), -halo, -hydroxy, -NO₂, -CN, -NZ₁Z₂, -nitrogen-containing-3- to 7-membered monocyclic heterocycle, -C₁-C₁₀ alkyl, -C₂-C₁₀ alkenyl, -C₂-C₁₀ alkynyl, -aryl, -benzyl, -C(O)OH, -C₁-C₅ alkylene-C(O)O-(C₁-C₅ alkyl) or -C₁-C₅ alkylene-OC(O)-(C₁-C₅ alkyl);
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀, alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
32. A composition comprising an effective amount of dacarbazine, a pharmacologically acceptable vehicle or carrier, and an effective amount of a compound or pharmaceutically acceptable salt of a compound having the formula **(II):** 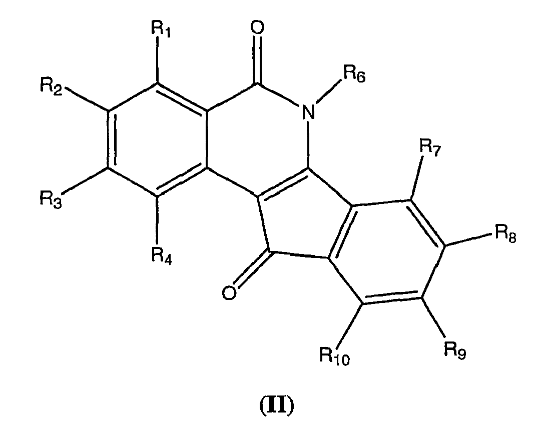 or a pharmaceutically acceptable salt thereof,
   wherein:
   R₆ is -H or C₁-C₅ alkyl;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A'-B';
   A' is -SO₂-, -SO₂NH- -NHCO-, -NHCONH-, -CO-, -C(O)O-, -CONH-, -CON(C₁-C₅ alkyl)-, -NH-, -(CH₂)-, -S- or -C(S)-;
   B' is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂;
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl); -OC(O)(C₁-C₅ alkyl), -NO₂ or -A-B;
   A is -SO₂-, -SO₂NH-, -NHCO-, -NHCONH-, -O-, -CO-, -OC(O)-, -C(O)O-, -CONH-, -CON(C₁-C₅ alkyl)-, -NH-, -CH₂-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂; and
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle.
33. A composition comprising an effective amount of dacarbazine, a pharmacologically acceptable vehicle or carrier, and an effective amount of a compound or pharmaceutically acceptable salt of a compound having the formula **(III):** 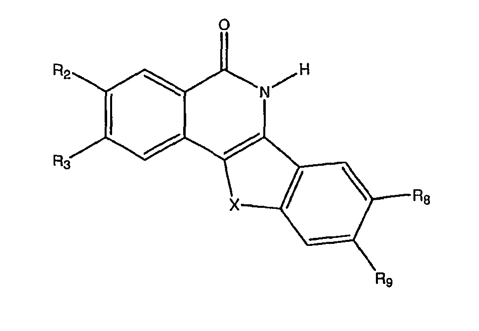 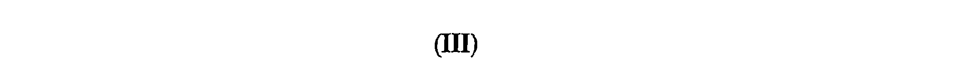 or a pharmaceutically acceptable salt thereof,
   wherein:
   X is -CH₂- or -O-;
   R₂ and R₃ are independently -hydrogen, -halo, -halo-substituted C₁-C₅ alkyl, -hydroxy, -O-(C₁-C₅ alkyl), -C₁-C₅ alkyl, -NO₂, -NH₂, -CONH₂, -C(O)OH, -OC(O)-C₁-C₅ alkyl or -C(O)O-C₁-C₅ alkyl;
   R₈ and R₉ are independently -hydrogen or -A-B;
   A is -SO₂- -SO₂NH- or -NHCO-;
   B is -C₁-C₅ alkyl, -NZ₁Z₂, -3- to 7-membered monocyclic heterocycle, or -7- to 10-membered bicyclic heterocycle, each of which is unsubstituted or substituted with one or more of -hydroxy-substituted C₁-C₅ alkyl, -amino-substituted C₁-C₅ alkyl, -3- to 7-membered monocyclic heterocycle, or -7- to 10-membered bicyclic heterocycle, each unsubstituted or substituted with -C₁-C₁₀ alkyl or -hydroxy-substituted C₁-C₅ alkyl; and
   Z₁ and Z₂ are independently hydrogen or -C₁-C₈ alkyl, which is unsubstituted or substituted with one or more of -hydroxy or -N(Z₃)(Z₄), were Z₃ and Z₄ are independently, -H or -C₁-C₅ alkyl, which is unsubstituted or substituted with one or more of -hydroxy or -NH₂; or N, Z₃ and Z₄ are taken together to form a nitrogen-containing-3-to 7-membered monocyclic heterocycle; or N, Z₁ and Z₂ are taken together to form a nitrogen-containing-3- to 7-membered monocyclic heterocycle.
34. A composition comprising an effective amount of dacarbazine, a pharmacologically acceptable vehicle or carrier, and an effective amount of a compound or pharmaceutically acceptable salt of a compound having the formula **(IV):** 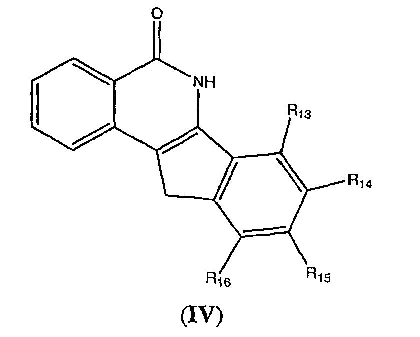 or a pharmaceutically acceptable salt thereof,
   wherein:
   R₁₃ and R₁₆ are hydrogen;
   one of the R₁₄ and R₁₅ groups is -NHC(O)-(CH₂)ₙ-NZ₁Z₂, and the other group is - hydrogen;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
35. A composition comprising an effective amount of irinotecan, a pharmacologically acceptable vehicle or carrier, and an effective amount of a compound or pharmaceutically acceptable salt of a compound having the formula **(I):** 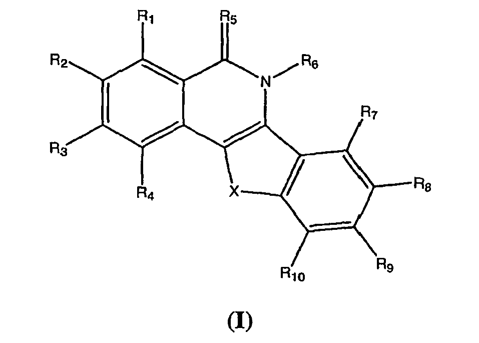 or a pharmaceutically acceptable salt thereof,
   wherein:
   R₅ is O, NH or S;
   R₆ is -H or -C₁-C₅ alkyl_{;}
   X is -C(O)-, -CH₂-, -CH(halo)-, -CH(OH)-(CH₂)ₙ-, -CH(OH), -CH(-aryl)-, -O-, -NH-, -S-, -CH(NR₁₁R₁₂)- or -N(SO₂Y)-, wherein Y is -OH, -NH₂ or -(C₁-C₅ alkyl)-(-3-to 7-membered monocyclic heterocycle);
   R₁₁ and R₁₂ are independently -hydrogen or -C₁-C₁₀ alkyl, or N, R₁₁ and R₁₂ are taken together to form a nitrogen-containing-3- to 7-membered monocyclic heterocycle;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A-B;
   A is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -NZ₁Z₂, -(C₁-C₅ alkylene)-NZ₁,Z₂, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -C(NH)NH₂, each of which, other than -NZ₁Z₂, -C(O)OH, or -C(NH)NH₂, is unsubstituted or substituted with one or more of -O-(C₁-C₅ alkyl), -halo, -hydroxy, -NO₂, -CN, -NZ₁Z₂, -nitrogen-containing-3- to 7-membered monocyclic heterocycle, -C₁-C₁₀ alkyl, -C₂-C₁₀ alkenyl, -C₂-C₁₀ alkynyl, -aryl, -benzyl, -C(O)OH, -C₁-C₅ alkylene-C(O)O-(C₁-C₅ alkyl) or -C₁-C₅ alkylene-OC(O)-(C₁-C₅ alkyl);
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀, alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
36. A composition comprising an effective amount of irinotecan, a pharmacologically acceptable vehicle or carrier, and an effective amount of a compound or pharmaceutically acceptable salt of a compound having the formula **(II):** 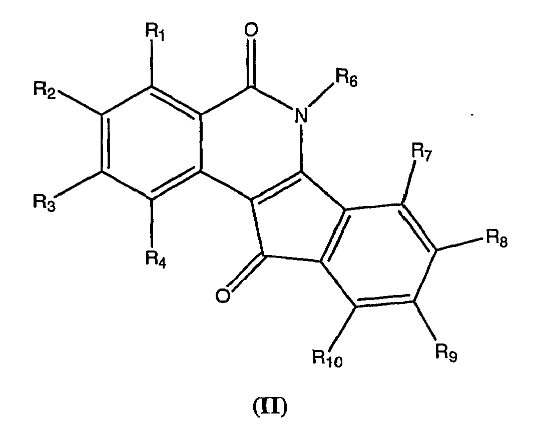 or a pharmaceutically acceptable salt thereof,
   wherein:
   R₆ is -H or C₁-C₅ alkyl;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A'-B';
   A' is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B' is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂;
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   A is -SO₂-, -SO₂NH-, -NHCO-, -NHCONH-, -O-, -CO-, -OC(O)-, -C(O)O-, -CONH-, -CON(C₁-C₅ alkyl)-, -NH-, -CH₂-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂; and
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle.
37. A composition comprising an effective amount of irinotecan, a pharmacologically acceptable vehicle or carrier, and an effective amount of a compound or pharmaceutically acceptable salt of a compound having the formula **(III):** 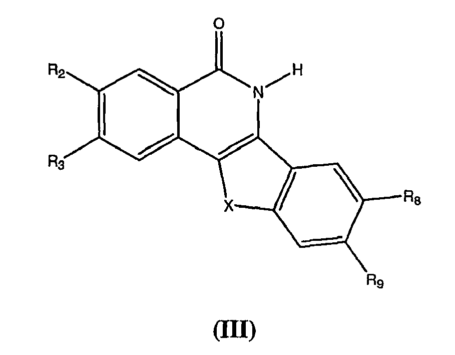 or a pharmaceutically acceptable salt thereof,
   wherein:
   X is -CH₂- or -O-;
   R₂ and R₃ are independently -hydrogen, -halo, -halo-substituted C₁-C₅ alkyl, -hydroxy, -O-(C₁-C₅ alkyl), -C₁-C₅ alkyl, -NO₂, -NH₂, -CONH₂, -C(O)OH, -OC(O)-C₁-C₅ alkyl or -C(O)O-C₁-C₅ alkyl;
   R₈ and R₉ are independently -hydrogen or -A-B;
   A is -SO₂-, -SO₂NH- or -NHCO-;
   B is -C₁-C₅ alkyl, -NZ₁Z₂, -3- to 7-membered monocyclic heterocycle, or -7- to 10-membered bicyclic heterocycle, each of which is unsubstituted or substituted with one or more of -hydroxy-substituted C₁-C₅ alkyl, -amino-substituted C₁-C₅ alkyl, -3- to 7-membered monocyclic heterocycle, or -7- to 10-membered bicyclic heterocycle, each unsubstituted or substituted with -C₁-C₁₀ alkyl or -hydroxy-substituted C₁-C₅ alkyl; and
   Z₁ and Z₂ are independently hydrogen or -C₁-C₈ alkyl, which is unsubstituted or substituted with one or more of -hydroxy or -N(Z₃)(Z₄), where Z₃ and Z₄ are independently, -H or -C₁-C₅ alkyl, which is unsubstituted or substituted with one or more of -hydroxy or -NH₂; or N, Z₃ and Z₄ are taken together to form a nitrogen-containing-3-to 7-membered monocyclic heterocycle; or N, Z₁ and Z₂ are taken together to form a nitrogen-containing-3- to 7-membered monocyclic heterocycle.
38. A composition comprising an effective amount of irinotecan, a pharmacologically acceptable vehicle or carrier, and an effective amount of a compound or pharmaceutically acceptable salt of a compound having the formula **(IV):** 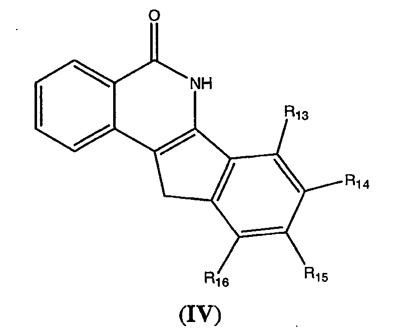 or a pharmaceutically acceptable salt thereof,
   wherein:
   R₁₃ and R₁₆ are hydrogen;
   one of the R₁₄ and R₁₅ groups is -NHC(O)-(CH₂)ₙ-NZ₁Z₂, and the other group is - hydrogen;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
39. A composition comprising an effective amount of Interleukin-2, a pharmacologically acceptable vehicle or carrier, and an effective amount of a compound or pharmaceutically acceptable salt of a compound having the formula **(I):** 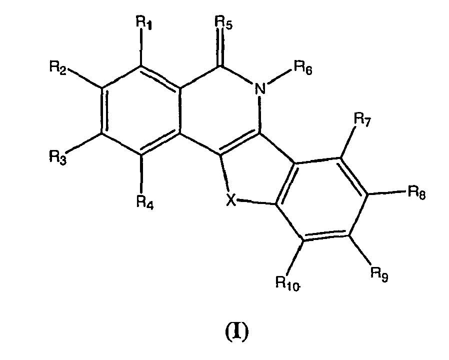 or a pharmaceutically acceptable salt thereof,
   wherein:
   R₅ is O, NH or S;
   R₆ is -H or -C₁-C₅ alkyl;
   X is -C(O)-, -CH₂-, -CH(halo)-, -CH(OH)-(CH₂)ₙ-, -CH(OH), -CH(-aryl)-, -O-, -NH-, -S-, -CH(NR₁₁R₁₂)- or -N(SO₂Y)-, wherein Y is -OH, -NH₂ or -(C₁-C₅ alkyl)-(-3-to 7-membered monocyclic heterocycle);
   R₁₁ and R₁₂ are independently -hydrogen or -C₁-C₁₀ alkyl, or N, R₁₁ and R₁₂ are taken together to form a nitrogen-containing-3- to 7-membered monocyclic heterocycle;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A-B;
   A is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -NZ₁Z₂, -(C₁-C₅ alkylene)-NZ₁,Z₂, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -C(NH)NH₂, each of which, other than -NZ₁Z₂, -C(O)OH, or -C(NH)NH₂, is unsubstituted or substituted with one or more of -O-(C₁-C₅ alkyl), -halo, -hydroxy, -NO₂, -CN, -NZ₁Z₂, -nitrogen-containing-3- to 7-membered monocyclic heterocycle, -C₁-C₁₀ alkyl, -C₂-C₁₀ alkenyl, -C₂-C₁₀ alkynyl, -aryl, -benzyl, -C(O)OH, -C₁-C₅ alkylene-C(O)O-(C₁-C₅ alkyl) or -C₁-C₅ alkylene-OC(O)-(C₁-C₅ alkyl);
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀, alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
40. A composition comprising an effective amount of Interleukin-2, a pharmacologically acceptable vehicle or carrier, and an effective amount of a compound or pharmaceutically acceptable salt of a compound having the formula **(II):** 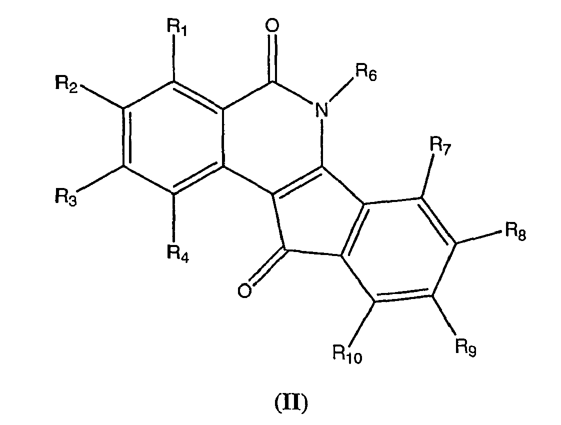 or a pharmaceutically acceptable salt thereof,
   wherein:
   R₆ is -H or C₁-C₅ alkyl;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A'-B';
   A' is -SO₂- -SO₂NH- -NHCO-, -NHCONH-, -CO-, -C(O)O-, -CONH-, -CON(C₁-C₅ alkyl)-, -NH-, -(CH₂)-, -S- or -C(S)-;
   B' is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂;
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   A is -SO₂- -SO₂NH-, -NHCO-, -NHCONH-, -O-, -CO-, -OC(O)-, -C(O)O-, -CONH-, -CON(C₁-C₅ alkyl)-, -NH-, -CH₂-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂; and
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle.
41. A composition comprising an effective amount of Interleukin-2, a pharmacologically acceptable vehicle or carrier, and an effective amount of a compound or pharmaceutically acceptable salt of a compound having the formula **(III):** 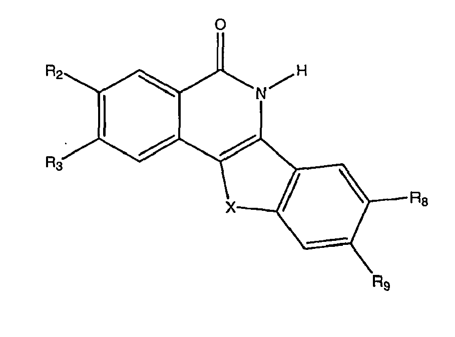 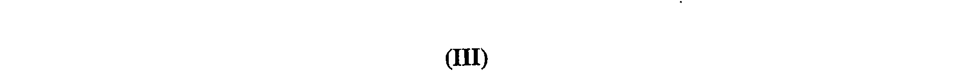 or a pharmaceutically acceptable salt thereof,
   wherein:
   X is -CH₂- or -O-;
   R₂ and R₃ are independently -hydrogen, -halo, -halo-substituted C₁-C₅ alkyl, -hydroxy, -O-(C₁-C₅ alkyl), -C₁-C₅ alkyl, -NO₂ -NH₂, -CONH₂, -C(O)OH, -OC(O)-C₁-C₅ alkyl or -C(O)O-C₁-C₅ alkyl;
   R₈ and R₉ are independently -hydrogen or -A-B;
   A is -SO₂-, -SO₂NH- or -NHCO-;
   B is -C₁-C₅ alkyl, -NZ₁Z₂, -3- to 7-membered monocyclic heterocycle, or -7- to 10-membered bicyclic heterocycle, each of which is unsubstituted or substituted with one or more of -hydroxy-substituted C₁-C₅ alkyl, -amino-substituted C₁-C₅ alkyl, -3- to 7-membered monocyclic heterocycle, or -7- to 10-membered bicyclic heterocycle, each unsubstituted or substituted with -C₁-C₁₀ alkyl or -hydroxy-substituted C₁-C₅ alkyl; and
   Z₁ and Z₂ are independently hydrogen or -C₁-C₈ alkyl, which is unsubstituted or substituted with one or more of -hydroxy or -N(Z₃)(Z₄), where Z₃ and Z₄ are independently, -H or -C₁-C₅ alkyl, which is unsubstituted or substituted with one or more of -hydroxy or -NH₂; or N, Z₃ and Z₄ are taken together to form a nitrogen-containing-3-to 7-membered monocyclic heterocycle; or N, Z₁ and Z₂ are taken together to form a nitrogen-containing-3- to 7-membered monocyclic heterocycle.
42. A composition comprising an effective amount of Interleukin-2, a pharmacologically acceptable vehicle or carrier, and an effective amount of a compound or pharmaceutically acceptable salt of a compound having the formula **(IV):** 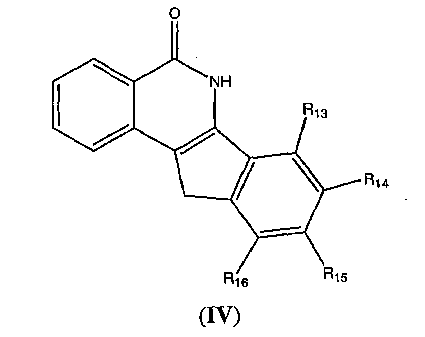 or a pharmaceutically acceptable salt thereof,
   wherein:
   R₁₃ and R₁₆ are hydrogen;
   one of the R₁₄ and R₁₅ groups is -NHC(O)-(CH₂)ₙ-NZ₁Z₂, and the other group is - hydrogen;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
43. A method for treating a vascular disease other than a cardiovascular disease, comprising administering to an animal in need thereof, an effective amount of a compound having the formula **(I):** 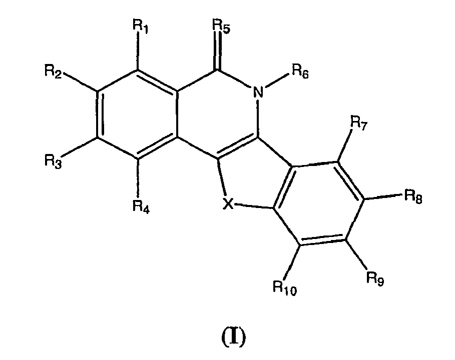 or a pharmaceutically acceptable salt thereof,
   wherein:
   R₅ is O, NH or S;
   R₆ is -H or -C₁-C₅ alkyl;
   X is -C(O)-, -CH₂-, -CH(halo)-, -CH(OH)-(CH₂)n-, -CH(OH), -CH(-aryl)-, -O-, -NH-, -S-, -CH(NR₁₁R₁₂)- or -N(SO₂Y)-, wherein Y is -OH, -NH₂ or -(C₁-C₅ alkyl)-(-3-to 7-membered monocyclic heterocycle);
   R₁₁ and R₁₂ are independently -hydrogen or -C₁-C₁₀ alkyl, or N, R₁₁ and R₁₂ are taken together to form a nitrogen-containing-3- to 7-membered monocyclic heterocycle;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A-B;
   A is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -NZ₁Z₂, -(C₁-C₅ alkylene)-NZ₁,Z₂, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -C(NH)NH₂, each of which, other than -NZ₁Z₂, -C(O)OH, or -C(NH)NH₂, is unsubstituted or substituted with one or more of -O-(C₁-C₅ alkyl), -halo, -hydroxy, -NO₂, -CN, -NZ₁Z₂, -nitrogen-containing-3- to 7-membered monocyclic heterocycle, -C₁-C₁₀ alkyl, -C₂-C₁₀ alkenyl, -C₂-C₁₀ alkynyl, -aryl, -benzyl, -C(O)OH, -C₁-C₅ alkylene-C(O)O-(C₁-C₅ alkyl) or -C₁-C₅ alkylene-OC(O)-(C₁-C₅ alkyl);
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀, alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
44. A method for treating a vascular disease other than a cardiovascular disease, comprising administering to an animal in need thereof, an effective amount of a compound having the formula **(II):** 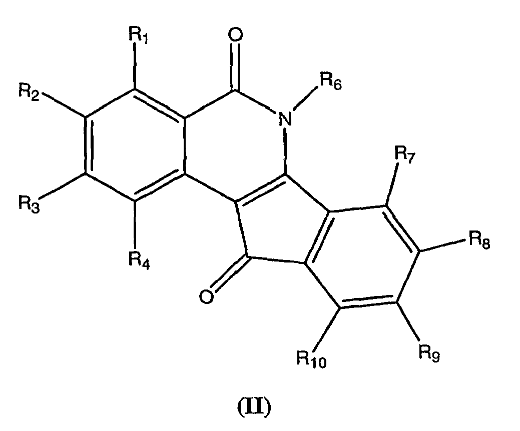 or a pharmaceutically acceptable salt thereof,
   wherein:
   R₆ is -H or C₁-C₅ alkyl;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A'-B';
   A' is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B' is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂;
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀ alkenyl, -c₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   A is -SO₂-, -SO₂NH-, -NHCO-, -NHCONH-, -O-, -CO-, -OC(O)-, -C(O)O-, -CONH-, -CON(C₁-C₅ alkyl)-, -NH-, -CH₂-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂; and
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle.
45. A method for treating a vascular disease other than a cardiovascular disease, comprising administering to an animal in need thereof, an effective amount of a compound having the formula **(III):** 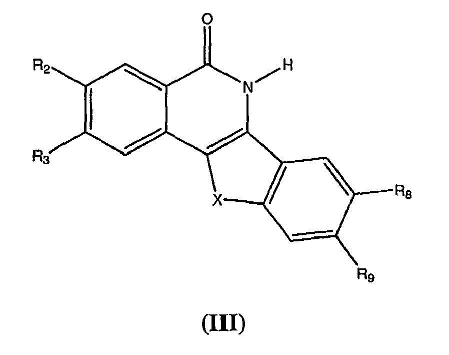 or a pharmaceutically acceptable salt thereof,
   wherein:
   X is -CH₂- or -O-;
   R₂ and R₃ are independently -hydrogen, -halo, -halo-substituted C₁-C₅ alkyl, -hydroxy, -O-(C₁-C₅ alkyl), -C₁-C₅ alkyl, -NO₂, -NH₂, -CONH₂, -C(O)OH, -OC(O)-C₁-C₅ alkyl or -C(O)O-C₁-C₅ alkyl;
   R₈ and R₉ are independently -hydrogen or -A-B;
   A is -SO₂-, -SO₂NH- or -NHCO-;
   B is -C₁-C₅ alkyl, -NZ₁Z₂, -3- to 7-membered monocyclic heterocycle, or -7- to 10-membered bicyclic heterocycle, each of which is unsubstituted or substituted with one or more of -hydroxy-substituted C₁-C₅ alkyl, -amino-substituted C₁-C₅ alkyl, -3- to 7-membered monocyclic heterocycle, or -7- to 10-membered bicyclic heterocycle, each unsubstituted or substituted with -C₁-C₁₀ alkyl or -hydroxy-substituted C₁-C₅ alkyl; and
   Z₁ and Z₂ are independently hydrogen or -C₁-C₈ alkyl, which is unsubstituted or substituted with one or more of -hydroxy or -N(Z₃)(Z₄), where Z₃ and Z₄ are independently, -H or -C₁-C₅ alkyl, which is unsubstituted or substituted with one or more of -hydroxy or -NH₂; or N, Z₃ and Z₄ are taken together to form a nitrogen-containing-3-to 7-membered monocyclic heterocycle; or N, Z₁ and Z₂ are taken together to form a nitrogen-containing-3- to 7-membered monocyclic heterocycle.
46. A method for treating a vascular disease other than cardiovascular disease, comprising administering to an animal in need thereof, an effective amount of a having the formula **(IV):** 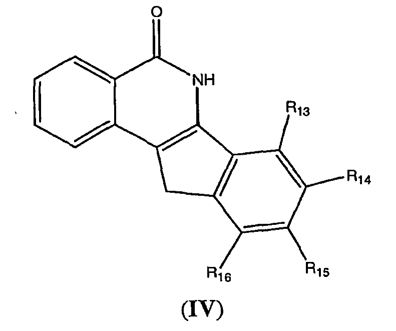 or a pharmaceutically acceptable salt thereof,
   wherein:
   R₁₃ and R₁₆ are hydrogen;
   one of the R₁₄ and R₁₅ groups is -NHC(O)-(CH₂)ₙ-NZ₁Z₂, and the other group is - hydrogen;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
47. The method of item 43, wherein the vascular disease is peripheral arterial occlusion, thromboangitis obliterans, Reynaud's disease and phenomenon, acrocyanosis, erythromelalgia, venous thrombosis, varicose veins, arteriovenous fistula, lymphedema, or lipedema.
48. The method of item 44, wherein the vascular disease is peripheral arterial occlusion, thromboangitis obliterans, Reynaud's disease and phenomenon, acrocyanosis, erythromelalgia, venous thrombosis, varicose veins, arteriovenous fistula, lymphedema, or lipedema.
49. The method of item 45, wherein the vascular disease is peripheral arterial occlusion, thromboangitis obliterans, Reynaud's disease and phenomenon, acrocyanosis, erythromelalgia, venous thrombosis, varicose veins, arteriovenous fistula, lymphedema, or lipedema.
50. The method of item 46, wherein the vascular disease is peripheral arterial occlusion, thromboangitis obliterans, Reynaud's disease and phenomenon, acrocyanosis, erythromelalgia, venous thrombosis, varicose veins, arteriovenous fistula, lymphedema, or lipedema.
51. A method for treating renal failure, comprising administering to an animal in need thereof, an effective amount of a compound having the formula **(I):** 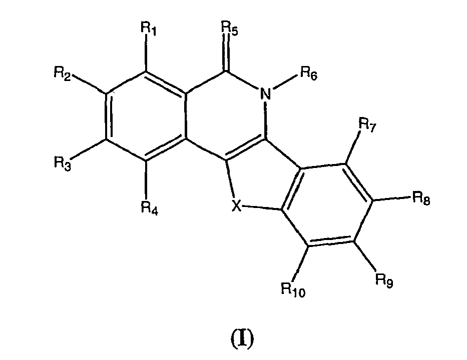 or a pharmaceutically acceptable salt thereof,
   wherein:
   R₅ is O, NH or S;
   R₆ is -H or -C₁-C₅ alkyl_{;}
   X is -C(O)-, -CH₂-, -CH(halo)-, -CH(OH)-(CH₂)ₙ-, -CH(OH), -CH(-aryl)-, -O-, -NH-, -S-, -CH(NR₁₁R₁₂)- or -N(SO₂Y)-, wherein Y is -OH, -NH₂ or -(C₁-C₅ alkyl)-(-3-to 7-membered monocyclic heterocycle);
   R₁₁ and R₁₂ are independently -hydrogen or -C₁-C₁₀ alkyl, or N, R₁₁ and R₁₂ are taken together to form a nitrogen-containing-3- to 7-membered monocyclic heterocycle;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A-B;
   A is -SO₂- -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -NZ₁Z₂, -(C₁-C₅ alkylene)-NZ₁,Z₂, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -C(NH)NH₂, each of which, other than -NZ₁Z₂, -C(O)OH, or -C(NH)NH₂, is unsubstituted or substituted with one or more of -O-(C₁-C₅ alkyl), -halo, -hydroxy, -NO₂, -CN, -NZ₁Z₂, -nitrogen-containing-3- to 7-membered monocyclic heterocycle, -C₁-C₁₀ alkyl, -C₂-C₁₀ alkenyl, -C₂-C₁₀ alkynyl, -aryl, -benzyl, -C(O)OH, -C₁-C₅ alkylene-C(O)O-(C₁-C₅ alkyl) or -C₁-C₅ alkylene-OC(O)-(C₁-C₅ alkyl);
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀, alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
52. A method for treating renal failure, comprising administering to an animal in need thereof, an effective amount of a compound having the formula **(II):** 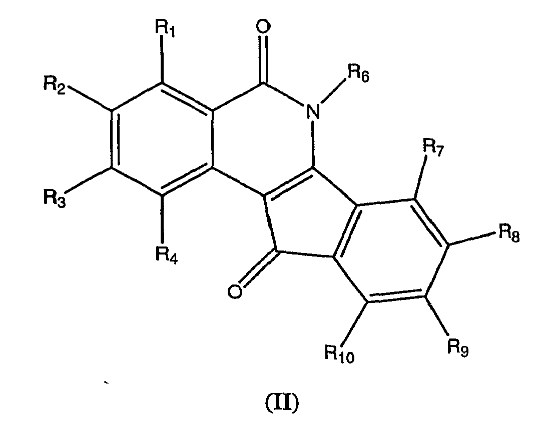 or a pharmaceutically acceptable salt thereof,
   wherein:
   R₆ is -H or C₁-C₅ alkyl;
   R₁ is -hydrogen, -halo, -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -NO₂ or -A'-B';
   A' is -SO₂-, -SO₂NH-, -NHCO-, -NHCONH-, -CO-, -C(O)O-, -CONH-, -CON(C₁-C₅ alkyl)-, -NH-, -(CH₂)-, -S- or -C(S)-;
   B' is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -amino-substituted C₁-C₅ alkyl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂;
   R₂, R₃, R₄, R₇, R₈, R₉ and R₁₀ are independently -hydrogen, -halo, -hydroxy, -O-(C₁-C₅ alkyl), -C₁-C₁₀ alkyl, -halo-substituted C₁-C₅ alkyl, -C₂-C₁₀ alkenyl, -C₃-C₈ monocyclic cycloalkyl, -aryl, -NH₂, -amino-substituted C₁-C₅ alkyl, -C(O)OH, -C(O)O(C₁-C₅ alkyl), -OC(O)(C₁-C₅ alkyl), -NO₂ or -A-B;
   A is -SO₂-, -SO₂NH-, -NHCO-, -NHCONH-, -O-, -CO-, -OC(O)-, -C(O)O-, -CONH-, -CON(C₁-C₅ alkyl)-, -NH-, -CH₂-, -S- or -C(S)-;
   B is -C₁-C₁₀ alkyl, -C₂-C₁₀ alkenyl, -3- to 7-membered monocyclic heterocycle, - 7- to 10-membered bicyclic heterocycle, -C₃-C₈ monocyclic cycloalkyl, -aryl, -(C₁-C₅ alkyl)-(-3- to 7-membered monocyclic heterocycle), -H₂NC(O)-substituted aryl, -C(O)OH, -C(O)O-(C₁-C₅ alkyl), -C(O)O-phenyl or -NZ₁Z₂; and
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle.
53. A method for treating renal failure, comprising administering to an animal in need thereof, an effective amount of a compound or pharmaceutically acceptable salt of a compound having the formula **(III):** 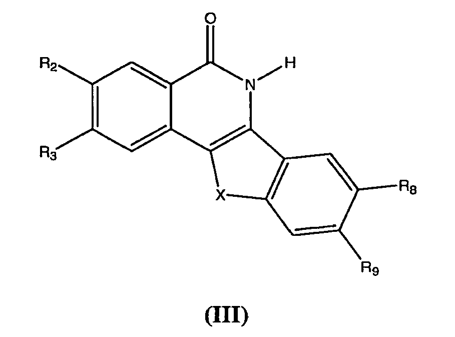 or a pharmaceutically acceptable salt thereof,
   wherein:
   X is -CH₂- or -O-;
   R₂ and R₃ are independently -hydrogen, -halo, -halo-substituted C₁-C₅ alkyl, -hydroxy, -O-(C₁-C₅ alkyl), -C₁-C₅ alkyl, -NO₂, -NH₂, -CONH₂, -C(O)OH, -OC(O)-C₁-C₅ alkyl or -C(O)O-C₁-C₅ alkyl;
   R₈ and R₉ are independently -hydrogen or -A-B;
   A is -SO₂-, -SO₂NH- or -NHCO-;
   B is -C₁-C₅ alkyl, -NZ₁Z₂, -3- to 7-membered monocyclic heterocycle, or -7- to 10-membered bicyclic heterocycle, each of which is unsubstituted or substituted with one or more of -hydroxy-substituted C₁-C₅ alkyl, -amino-substituted C₁-C₅ alkyl, -3- to 7- membered monocyclic heterocycle, or -7- to 10-membered bicyclic heterocycle, each unsubstituted or substituted with -C₁-C₁₀ alkyl or -hydroxy-substituted C₁-C₅ alkyl; and
   Z₁ and Z₂ are independently hydrogen or -C₁-C₈ alkyl, which is unsubstituted or substituted with one or more of -hydroxy or -N(Z₃)(Z₄), where Z₃ and Z₄ are independently, -H or -C₁-C₅ alkyl, which is unsubstituted or substituted with one or more of -hydroxy or -NH₂; or N, Z₃ and Z₄ are taken together to form a nitrogen-containing-3-to 7-membered monocyclic heterocycle; or N, Z₁ and Z₂ are taken together to form a nitrogen-containing-3- to 7-membered monocyclic heterocycle.
54. A method of for treating renal failure, comprising administering to an animal in need thereof, an effective amount of a having the formula **(IV):** 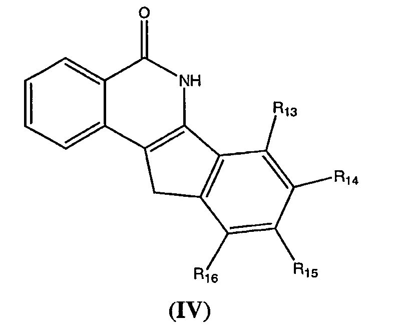 or a pharmaceutically acceptable salts thereof,
   wherein:
   R₁₃ and R₁₆ are hydrogen;
      one of the R₁₄ and R₁₅ groups is -NHC(O)-(CH₂)ₙ-NZ₁Z₂, and the other group is - hydrogen;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
55. The method of item 51, wherein the renal failure is chronic renal failure or acute renal failure.
56. The method of item 52, wherein the renal failure is chronic renal failure or acute renal failure.
57. The method of item 53, wherein the renal failure is chronic renal failure or acute renal failure.
58. The method of item 54, wherein the renal failure is chronic renal failure or acute renal failure.
59. A compound of the Formula **(IV):** 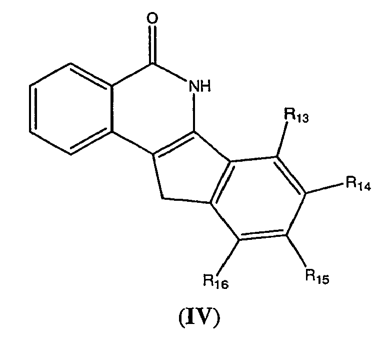 or a pharmaceutically acceptable salt thereof,
   wherein:
   R₁₃ and R₁₆ are hydrogen;
   one of the R₁₄ and R₁₅ groups is -NHC(O)-(CH₂)ₙ-NZ₁Z₂, and the other group is - hydrogen;
   Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing-3- to 7-membered monocyclic heterocycle; or N, Z₁ and Z₂ are taken together to form a nitrogen-containing-3- to 7-membered monocyclic heterocycle; and
   n is an integer ranging from 0-5.
60. The compound or pharmaceutically acceptable salt of the compound of item 59, wherein R₁₄ is -NHC(O)-(CH₂)ₙ-NZ₁Z₂ and R₁₃, R₁₅, and R₁₆ are each hydrogen.
61. The compound or pharmaceutically acceptable salt of the compound of item 59, wherein R₁₅ is -NHC(O)-(CH₂)ₙ-NZ₁Z₂ and R₁₃, R₁₄, and R₁₆ are each hydrogen.
62. The compound or pharmaceutically acceptable salt of the compound of item 59, wherein n is 1.
63. The compound or pharmaceutically acceptable salt of the compound of item 59, wherein n is 2.
64. The compound or pharmaceutically acceptable salt of the compound of item 59, wherein n is 3.
65. The compound or pharmaceutically acceptable salt of the compound of item 59, wherein n is 4.
66. The compound or pharmaceutically acceptable salt of the compound of item 59, wherein n is 5.
67. A composition comprising a physiologically acceptable carrier or vehicle and an effective amount of a compound or a pharmaceutically acceptable salt of the compound of item 59.
68. A method of treating a reperfusion injury, comprising administering to an animal in need thereof an effective amount of a compound or pharmaceutically acceptable salt of the compound of item 59.
69. The method of item 68, wherein the reperfusion injury is stroke or myocardial infarction.
70. A method of treating an inflammatory disease, comprising admininstering to an animal in need thereof an effective amount of a compound or pharmaceutically acceptable salt of the compound of item 59.
71. The method of item 70, wherein the inflammatory disease is an inflammatory disease of a joint, a chronic inflammatory disease of the gum, an inflammatory bowel disease, an inflammatory lung disease, an inflammatory disease of the central nervous system, an inflammatory disease of the eye, gram-positive shock, gram negative shock, hemorrhagic shock, anaphylactic shock, traumatic shock, or chemotherapeutic shock.
72. A method of treating diabetes or a diabetic complication, comprising admininstering to an animal in need thereof an effective amount of a compound or pharmaceutically acceptable salt of the compound of item 59.
73. The method of item 72, wherein the diabetes is type I diabetes or type II diabetes.
74. A method of treating an ischemic condition, comprising admininstering to an animal in need thereof an effective amount of a compound or pharmaceutically acceptable salt of the compound of item 59.
75. The method of item 74, wherein the ischemic condition is myocardial ischemia, stable angina, unstable angina, stroke, ischemic heart disease, or cerebral ischemia.
76. A method of treating a reoxygenation injury resulting from organ transplantation, comprising administering to an animal in need thereof an effective amount of a compound or pharmaceutically acceptable salt of the compound of item 59.
77. A method of treating Parkinson's disease, comprising admininstering to an animal in need thereof an effective amount of a compound or pharmaceutically acceptable salt of the compound of item 59.

## Claims

1. A compound of the Formula **(IV):** 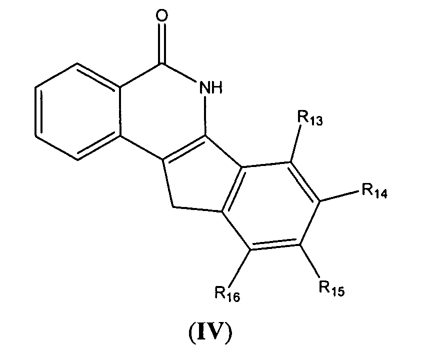 or a pharmaceutically acceptable salt thereof,
wherein:
R₁₃ and R₁₆ are hydrogen;
one of the R₁₄ and R₁₅ groups is -NHC(O)-(CH₂)ₙ-NZ₁Z₂, and the other group is hydrogen;
Z₁ and Z₂ are independently -H or -C₁-C₁₀ alkyl, which is unsubstituted or substituted with one or more of -halo, -OH or -N(Z₃)(Z₄), where Z₃ and Z₄ are independently, -H or -C₁-C₅ alkyl, which is unsubstituted or substituted with one or more of -halo, -hydroxy or -NH₂; or N, Z₃ and Z₄ are taken together to form a nitrogen-containing -3- to 7-membered monocyclic heterocycle; or N, Z₁ and Z₂ are taken together to form a nitrogen-containing -3- to 7-membered monocyclic heterocycle; and
n is an integer ranging from 0-5.

2. The compound or pharmaceutically acceptable salt of the compound of claim 1, wherein R₁₄ is -NHC(O)-(CH₂)ₙ-NZ₁Z₂ and R₁₃, R₁₅, and R₁₆ are each hydrogen.

3. The compound or pharmaceutically acceptable salt of the compound of claim 1, wherein R₁₅ is -NHC(O)-(CH₂)ₙ-NZ₁Z₂ and R₁₃, R₁₄, and R₁₆ are each hydrogen.

4. The compound or pharmaceutically acceptable salt of the compound of claim 1, wherein n is 1, 2, 3, 4, or 5.

5. The compound of claim 1 having the formula

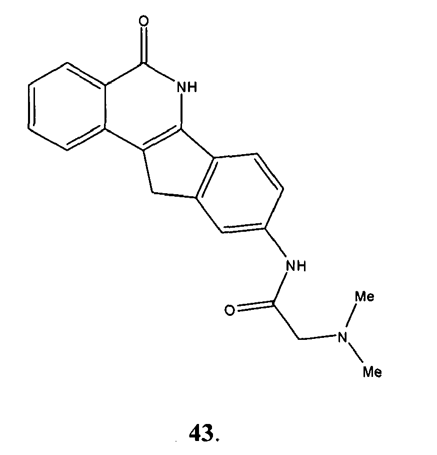

6. A composition comprising a physiologically acceptable carrier or vehicle and an effective amount of a compound or a pharmaceutically acceptable salt of a compound as defined in any of the claims 1 to 5.

7. A composition according to claim 6 further comprising an effective amount of temozolomide, procarbazine, dacarbazine, irinotecan or Interleukin-2.

8. A compound or pharmaceutically acceptable salt of a compound as defined in any one of claims 1 to 5 for treating a subject having
(i) a reperfusion injury,
(ii) an inflammatory disease,
(iii) diabetes or a diabetic complication,
(iv) an ischemic condition,
(v) a reoxygenation injury resulting from organ transplantation,
(vi) a vascular disease other than a cardiovascular disease,
(vii) renal failure,
(viii) Parkinson's disease, or
(ix) cancer.

9. The compound of claim 8, wherein the reperfusion injury is stroke or myocardial infarction.

10. The compound of claim 8, wherein the inflammatory disease is an inflammatory disease of a joint, a chronic inflammatory disease of the gum, an inflammatory bowel disease, an inflammatory lung disease, an inflammatory disease of the central nervous system, an inflammatory disease of the eye, gram-positive shock, gram negative shock, hemorrhagic shock, anaphylactic shock, traumatic shock, or chemotherapeutic shock.

11. The compound of claim 8, wherein the diabetes is type I diabetes or type II diabetes.

12. The compound of claim 8, wherein the ischemic condition is myocardial ischemia, stable angina, unstable angina, stroke, ischemic heart disease, or cerebral ischemia.

13. The method of claim 8, wherein the vascular disease is peripheral arterial occlusion, thromboangitis obliterans, Reynaud's disease and phenomenon, acrocyanosis, erythromelalgia, venous thrombosis, varicose veins, arteriovenous fistula, lymphedema, or lipedema.

14. The method of claim 8, wherein the renal failure is chronic renal failure or acute renal failure.

15. The method of claim 8, wherein the cancer is colorectal cancer, lung cancer, pancreatic cancer, esophageal cancer, stomach cancer, skin cancer, leukemia, lymphoma, non-Hodgkin's lymphoma, testicular cancer, bladder cancer, kidney cancer, liver cancer, breast cancer, prostate cancer, head and neck cancer, brain cancer, cancer of the central nervous system, uterine cancer, cervical cancer, or ovarian cancer.

16. The method of claims 8 or 15, wherein the cancer is melanoma, metastatic brain cancer, or glioma.

17. The method of claim 16, wherein the glioma is pilocytic astrocytoma, astrocytoma, anaplastic astrocytoma, or glioblastoma multiforme.

18. The compound of any one of claims 8 or 15-17, further comprising an effective amount of temozolomide, procarbazine, dacarbazine, irinotecan, Interleukin-2, or a combination thereof.
